(19) Europäisches Patentamt
European Patent Office
Office européen des brevets

(11) Veröffentlichungsnummer: **0 056 144**
**A1**

(12) **EUROPÄISCHE PATENTANMELDUNG**

(21) Anmeldenummer: **81110731.7**

(22) Anmeldetag: **23.12.81**

(51) Int. Cl.³: **C 07 D 295/14**, C 07 D 211/14, C 07 D 209/44, C 07 D 221/22, C 07 D 213/56, C 07 D 401/04, C 07 D 213/55, A 61 K 31/455, A 61 K 31/55, A 61 K 31/40, A 61 K 31/395 // (C07D401/04, 211/14, 213/56)

(30) Priorität: **10.01.81 DE 3100535**

(43) Veröffentlichungstag der Anmeldung: **21.07.82 Patentblatt 82/29**

(84) Benannte Vertragsstaaten: **AT BE CH DE FR IT LI LU NL SE**

(71) Anmelder: **Dr. Karl Thomae GmbH, Postfach 1755, D-7950 Biberach (Riss) (DE)**

(72) Erfinder: **Griss, Gerhart, Dr. Dipl.-Chem., Schopperweg 1, D-7950 Biberach 1 (DE)**
Erfinder: **Sauter, Robert, Dr. Dipl.-Chem., Albert-Schweitzer-Weg 9, D-7958 Laupheim (DE)**
Erfinder: **Grell, Wolfgang, Dr. Dipl.-Chem., Amriswilstrasse 7, D-7950 Biberach 1 (DE)**
Erfinder: **Hurnaus, Rudolf, Dr. Dipl.-Chem., Silcherstrasse 19, D-7950 Biberach 1 (DE)**
Erfinder: **Rupprecht, Eckhardt, Dr. Dipl.-Bio., Riedbachstrasse 15, D-7960 Aulendorf-Tannhausen (DE)**
Erfinder: **Kaubisch, Nikolaus, Dr. Dipl.-Chem., Moltkestrasse 3, D-6550 Bad Kreuznach (DE)**
Erfinder: **Eisele, Bernhard, Dr. Dipl.-Chem., Beethovenstrasse 12, D-7950 Biberach 1 (DE)**
Erfinder: **Kähling, Joachim, Dr., Haydnweg 8, D-7950 Biberach 1 (DE)**

(54) **Neue Carbonsäure-Derivate, ihre Herstellung und ihre Verwendung als Arzneimittel.**

(57) Carbonsäurederivate der allgemeinen Formel

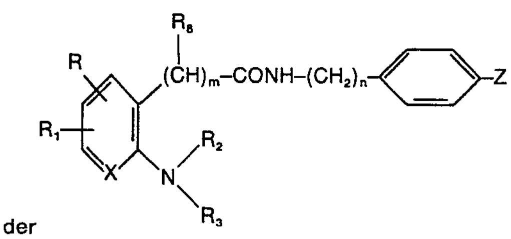

, (I)

in der

m die Zahl 0 oder 1,

n die Zahl 1 oder 2,

R ein Wasserstoffatom oder eine Alkylgruppe mit 1 bis 3 Kohlenstoffatomen,

$R_1$ ein Wasserstoff-, Fluor-, Chlor-, Brom- oder Jodatom, eine Alkyl- oder Alkoxygruppe mit jeweils 1 bis 4 Kohlenstoffatomen, eine Cyano-, Hydroxy- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_2$ und $R_3$ zusammen mit dem dazwischen liegenden Stickstoffatom eine Piperidino-, 3,5-Dimethyl-piperidino-, Octahydro-1H-azonino-, Decahydro-azecino-, 1,3-Dihydro-isoindolo-, Hexahydro-isoindolo- oder Octahydro-isoindologruppe, eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, der durch eine oder mehrere Alkylgruppen mit jeweils 1 bis 3 Kohlenstoffatomen substituiert sein kann, oder eine durch ein Halogenatom, eine Alkoxygruppe mit 1 bis 3 Kohlenstoffatomen oder eine Aminogruppe substituierte 1,3-Dihydro-isoindolgruppe oder auch, wenn m die Zahl 1 darstellt, die Pyrrolidino-, Hexamethylenimino- oder Heptamethyleniminogruppe,

X eine CH-Gruppe oder ein Stickstoffatom,

$R_8$ ein Wasserstoffatom, eine Alkylgruppe mit 1 bis 3 Kohlenstoffatomen oder eine Arylgruppe und

Z eine gegebenenfalls veresterte Carboxygruppe bedeuten, deren optisch aktive Antipoden, sofern sie ein asymmetrisches Kohlenstoffatom enthalten, und deren Salze, insbesondere deren physiologisch verträgliche Säureadditionssalze mit anorganischen oder organischen Säuren oder Basen. Außerdem sind 2-Amino-carbonsäure-Derivate beschrieben, die wertvolle Zwischenprodukte zur Herstellung der Verbindungen der allgemeinen Formel I darstellen.

Die Verbindungen können nach für analoge Verbindungen üblichen Verfahren hergestellt werden.

EP 0 056 144 A1

0056144

DR. KARL THOMAE GMBH
D-7950 Biberach 1

Case 5/821
Dr. Fl./Kp.

# Neue Carbonsäure-Derivate, ihre Herstellung und ihre Verwendung als Arzneimittel

Gegenstand der vorliegenden Erfindung sind neue Carbonsäure-Derivate der allgemeinen Formel

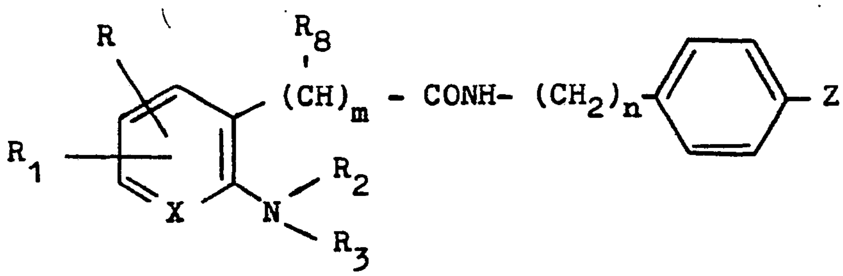

,(I)

deren Additionssalze, insbesondere deren physiologisch verträgliche Salze mit anorganischen oder organischen Säuren und auch Basen, wenn Z eine Carboxygruppe darstellt, und Verfahren zu ihrer Herstellung sowie die neuen Carbonsäure-Derivate enthaltende Arzneimittel.

Ein weiterer Gegenstand der vorliegenden Erfindung sind die neuen 2-Amino-carbonsäure-Derivate der allgemeinen Formel

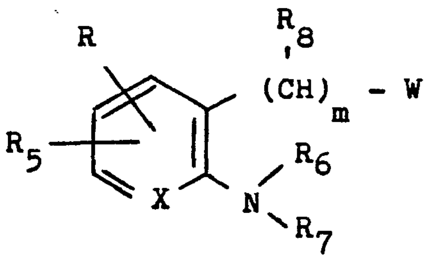

,(Ia)

deren Additionssalze, insbesondere deren Salze mit anorganischen oder organischen Säuren und auch Basen, wenn W die Carboxygruppe darstellt, und Verfahren zu ihrer Herstellung sowie die Verwendung der neuen 2-Amino-carbonsäure-Derivate als Arzneimittel und/oder als Zwischenprodukte zur Herstellung der neuen Verbindungen der allgemeinen Formel I.

Die neuen Verbindungen der allgemeinen Formeln I und Ia und deren optisch aktive Antipoden, sofern sie ein asymmetrisches Kohlenstoffatom enthalten, weisen wertvolle pharmakologische Eigenschaften auf, nämlich eine Wirkung auf den Intermediärstoffwechsel. So besitzen die Verbindungen der allgemeinen Formel I insbesondere eine blutzuckersenkende und/oder lipidsenkende Wirkung und die Verbindungen der allgemeinen Formel Ia eine lipidsenkende Wirkung.

In den obigen allgemeinen Formeln I und Ia bedeutet

m die Zahl 0 oder 1,

n die Zahl 1 oder 2,

R ein Wasserstoffatom oder eine Alkylgruppe mit 1 bis 3 Kohlenstoffatomen,

$R_1$ ein Wasserstoff-, Fluor-, Chlor-, Brom- oder Jodatom, eine Alkyl- oder Alkoxygruppe mit jeweils 1 bis 4 Kohlenstoffatomen, eine Cyano-, Hydroxy- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_2$ und $R_3$ zusammen mit dem dazwischen liegenden Stickstoffatom eine Piperidino-, 3,5-Dimethyl-piperidino-, Octahydro-1H-azonino-, Decahydro-azecino-, 1,3-Dihydro-isoindolo-, Hexahydro-isoindolo- oder Octahydro-isoindologruppe, eine durch eine Alkylgruppe mit 5 bis 10 Kohlenstoffatomen substituierte

Piperidinogruppe, eine Azabicycloalkylgruppe mit 7 bis 12 Kohlenstoffatomen im Bicycloalkanring, der durch eine oder mehrere Alkylgruppen mit jeweils 1 bis 3 Kohlenstoffatomen substituiert sein kann, oder eine durch ein Halogenatom, eine Alkoxygruppe mit 1 bis 3 Kohlenstoffatomen oder eine Aminogruppe substituierte 1,3-Dihydro-isoindologruppe oder auch, wenn m die Zahl 1 darstellt, die Pyrrolidino-, Hexamethylenimino- oder Heptamethyleniminogruppe,

X eine CH-Gruppe oder ein Stickstoffatom,

Z und W jeweils eine gegebenenfalls veresterte Carboxygruppe,

$R_5$ ein Wasserstoff- oder Halogenatom, eine Amino-, Cyano-, Hydroxy-, Carboxy- oder Acetaminogruppe, eine Alkyl- oder Alkoxygruppe mit jeweils 1 bis 4 Kohlenstoffatomen,

$R_6$ und $R_7$ zusammen mit dem dazwischenliegenden Stickstoffatom eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, welche durch eine oder mehrere Alkylgruppen mit 1 bis 3 Kohlenstoffatomen substituiert sein kann, eine Octahydro-1H-azonino-, 1,3-Dihydro-isoindolo-, Hexahydro-isoindolo- oder Octahydroisoindologruppe oder eine durch ein Halogenatom, eine Alkoxygruppe mit 1 bis 3 Kohlenstoffatomen oder eine Aminogruppe substituierte 1,3-Dihydro-isoindologruppe und

$R_8$ ein Wasserstoffatom, eine Alkylgruppe mit 1 bis 3 Kohlenstoffatomen oder eine Arylgruppe.

Für die bei der Definition der Reste R, $R_1$ bis $R_8$ und Z bzw. W eingangs erwähnten Bedeutungen kommen beispielsweise

für R die Bedeutung des Wasserstoffatoms, die der Methyl-, Äthyl-, Propyl- oder Isopropylgruppe,

für $R_1$ die des Wasserstoff-, Fluor-, Chlor-, Brom- oder Jodatoms, die der Methyl-, Äthyl-, Propyl-, Isopropyl-, Butyl-, Isobutyl-, tert.Butyl-, Hydroxy-, Methoxy-, Äthoxy-, Propoxy-, Isopropoxy-, Butoxy-, Isobutoxy-, Cyan-, Trifluormethyl-, Phenyl-, Methylphenyl-, Äthylphenyl-, Isopropylphenyl-, Fluorphenyl-, Chlorphenyl- oder Bromphenylgruppe,

für $R_2$ und $R_3$ zusammen mit dem dazwischen liegenden Stickstoffatom die der Pyrrolidino-, Piperidino-, 3,5-Dimethylpiperidino-, Pentyl-(3)-piperidino-, Nonyl-(5)-piperidino-, Hexamethylenimino-, Octahydro-1H-azonino-, 1,3-Dihydro-isoindolo-, Hexahydro-isoindolo-, Octahydro-isoindolo-, Chlor-1,3-dihydro-isoindolo-, Brom-1,3-dihydro-isoindolo-, Methoxy-1,3-dihydro-isoindolo-, Äthoxy-1,3-dihydro-isoindolo-, Isopropoxy-1,3-dihydro-isoindolo-, Amino-1,3-dihydro-isoindolo- oder 2-Azabicyclo-nonan-2-yl-gruppe,

für Z und W jeweils die der Carboxy-, Methoxycarbonyl-, Äthoxycarbonyl-, Propoxycarbonyl-, Isopropoxycarbonyl-, Butoxycarbonyl-, Isobutoxycarbonyl-, tert.Butoxycarbonyl-, Pentoxycarbonyl-, Hexoxycarbonyl-, Heptoxycarbonyl-, Allyloxycarbonyl-, Phenoxycarbonyl-, Benzyloxycarbonyl-, Phenyläthoxycarbonyl-, Cyclopropoxycarbonyl-, Cyclopentoxycarbonyl-, Cyclohexyloxycarbonyl- oder Cycloheptoxycarbonylgruppe,

für $R_5$ die Bedeutung des Wasserstoff-, Fluor-, Chlor-, Brom- oder Jodatoms, die der Methyl-, Äthyl-, Cyano-, Hydroxy-, Methoxy-, Carboxy-, Amino- oder Acetaminogruppe,

für $R_6$ und $R_7$ zusammen mit dem dazwischenliegenden Stickstoffatom die der Pentyl-(3)-piperidino-, Nonyl-(5)-piperidino-, 2-Azabicyclo-nonan-2-yl-, 1,3-Dihydro-isoindolo-, Chlor-1,3-dihydro-isoindolo-, Brom-1,3-dihydro-isoindolo-, Methoxy-1,3-dihydro-isoindolo-, Äthoxy-1,3-dihydro-isoindolo-, Isopropoxy-1,3-dihydro-isoindolo-, Amino-1,3-dihydro-isoindolo-, Hexahydro-isoindolo- oder Octahydro-isoindologruppe und

für $R_8$ die des Wasserstoffatoms, die der Methyl-, Äthyl-, Propyl-, Isopropyl- oder Phenylgruppe in Betracht.

Bevorzugte Verbindungen der obigen allgemeinen Formeln I und Ia sind diejenigen, in denen

m die Zahl 0 oder 1,

n die Zahl 1 oder 2,

X die CH-Gruppe oder ein Stickstoffatom,

R ein Wasserstoffatom oder die Methylgruppe,

$R_1$ ein Wasserstoff-, Fluor-, Chlor-, Brom- oder Jodatom, eine Cyano-, Trifluormethyl-, Hydroxy-, Methoxy-, Methyl-, Methylphenyl-, Chlorphenyl- oder Bromphenylgruppe,

$R_2$ und $R_3$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch eine Methoxy- oder Aminogruppe, ein Chlor- oder Bromatom substituierte 1,3-Dihydro-isoindologruppe, eine Hexahydro-isoindolo- oder 2-Aza-bicyclo-nonan-2-ylgruppe oder

eine Octahydro-1H-azoninogruppe, wenn
m die Zahl 1 oder
m die Zahl 0,
$R_1$ ein Wasserstoff-, Fluor- oder Jodatom oder eine Methylgruppe und
X eine CH-Gruppe oder
m die Zahl 0,
X ein Stickstoffatom und
$R_1$ kein Wasserstoffatom bedeuten, oder

eine Decahydro-1H-azecinogruppe, wenn
m die Zahl 1 oder
m die Zahl O,
$R_1$ eine Methylgruppe, ein Wasserstoff-, Fluor-, Brom- oder Jodatom und
X die CH-Gruppe oder
m die Zahl O,
X ein Stickstoffatom und
$R_1$ kein Wasserstoffatom bedeuten, oder

eine Piperidinogruppe, wenn
m die Zahl 1 oder
m die Zahl O,
X ein Stickstoffatom und
$R_1$ kein Wasserstoffatom oder
m die Zahl O,
X eine CH-Gruppe und
$R_1$ ein Wasserstoffatom bedeuten, oder

eine Octahydro-isoindologruppe, wenn
m die Zahl 1 oder
m die Zahl O,
$R_1$ ein Wasserstoff-, Fluor-, Brom- oder Jodatom, eine Methyl-, Hydroxy-, Methoxy- oder Cyanogruppe und
X die CH-Gruppe oder
m die Zahl O und
X ein Stickstoffatom bedeuten, oder

eine 3,5-Dimethyl-piperidinogruppe, wenn
m die Zahl 1 oder
m die Zahl O,
$R_1$ ein Wasserstoff-, Chlor- oder Bromatom,
R die Methylgruppe und
X ein Stickstoffatom oder
m die Zahl O,
$R_1$ eine Methyl-, Hydroxy- oder Cyangruppe, ein Fluor- oder Jodatom und

X eine CH-Gruppe bedeuten, oder auch eine Pyrrolidino-, Hexamethylenimino- oder Heptamethyleniminogruppe, wenn

m die Zahl 1 darstellt,

$R_8$ ein Wasserstoffatom, eine Methyl- oder Phenylgruppe,

Z und W je eine Carboxy- oder eine Alkoxycarbonylgruppe mit insgesamt 2 bis 4 Kohlenstoffatomen,

$R_5$ ein Wasserstoff-, Fluor-, Chlor-, Brom- oder Jodatom, eine Alkylgruppe mit 1 bis 4 Kohlenstoffatomen, eine Hydroxy-, Methoxy-, Amino-, Cyano-, Carboxy- oder Acetaminogruppe,

$R_6$ und $R_7$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch ein Chlor- oder Bromatom, eine Methoxy- oder eine Aminogruppe substituierte 1,3-Dihydro-isoindologruppe, eine Hexahydro-isoindolo- oder 2-Aza-bicyclononan-2-yl-gruppe oder auch eine Octahydro-isoindologruppe, wenn m die Zahl 1 oder

X ein Stickstoffatom oder m die Zahl O,

$R_5$ ein Wasserstoff-, Fluor-, Brom- oder Jodatom, eine Alkylgruppe mit 1 bis 4 Kohlenstoffatomen, eine Hydroxy-, Methoxy-, Cyano-, Carboxy- oder Acetaminogruppe und

X eine CH-Gruppe darstellen, bedeuten, und, sofern sie ein asymmetrisches Kohlenstoffatom enthalten, deren optisch aktive Antipoden und deren Salze.

Besonders bevorzugte Verbindungen der obigen allgemeinen Formeln I und Ia sind jedoch diejenigen, in denen

m die Zahl O oder 1,

n die Zahl 2,

X eine CH-Gruppe,

Z eine Carboxy- oder Alkoxycarbonylgruppe mit insgesamt 2 bis 4 Kohlenstoffatomen,

W eine Carboxygruppe,

R ein Wasserstoffatom,

$R_1$ in 5-Stellung ein Fluor-, Chlor-, Brom- oder Jodatom oder eine Methylgruppe oder auch ein Wasserstoffatom, wenn $R_2$ und $R_3$ zusammen mit dem dazwischenliegenden Stickstoffatom die Octahydro-1H-azoninogruppe darstellt,

$R_5$ in 5-Position ein Wasserstoff-, Chlor- oder Bromatom,

$R_2$ und $R_3$ bzw. $R_6$ und $R_7$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch ein Chlor- oder Bromatom substituierte 1,3-Dihydro-isoindologruppe, eine 2-Aza-bicyclo-nonan-3-yl- oder Hexahydro-isoindologruppe oder

$R_2$ und $R_3$ zusammen mit dem dazwischenliegenden Stickstoffatom eine Octahydro-1H-azoninogruppe, wenn

m die Zahl 1 oder

m die Zahl O und

$R_1$ eine Methylgruppe, ein Wasserstoff-, Fluor- oder Jodatom darstellen, oder

$R_6$ und $R_7$ zusammen mit dem dazwischenliegenden Stickstoffatom eine Octahydro-isoindologruppe, wenn

m die Zahl 1 oder

m die Zahl O und

$R_5$ ein Bromatom darstellen, oder

$R_2$ und $R_3$ zusammen mit dem dazwischenliegenden Stickstoffatom eine Pyrrolidino-, Piperidino-, Hexamethyleniminooder Heptamethyleniminogruppe, wenn

m die Zahl 1 darstellt, und

$R_8$ ein Wasserstoffatom, eine Methyl- oder Phenylgruppe bedeuten und, sofern sie ein asymmetrisches Kohlenstoffatom enthalten, deren optisch aktive Antipoden und deren physiologisch verträglichen Additionssalze mit anorganischen oder organischen Säuren oder auch Basen, wenn Z oder W eine Carboxygruppe darstellt.

Erfindungsgemäß erhält man die neuen Verbindungen nach folgenden Verfahren:

1. Herstellung der 2-Amino-carbonsäure-Derivate der allgemeinen Formel Ia:

a) Zur Herstellung von Verbindungen der allgemeinen Formel Ia, in der W die Carboxygruppe darstellt:

Hydrolyse einer Verbindung der allgemeinen Formel

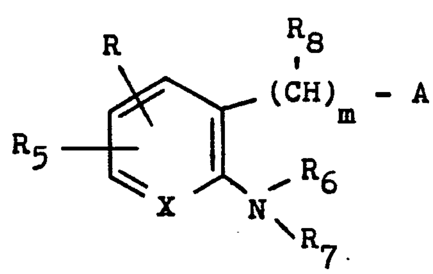

,(II)

in der

R, $R_5$ bis $R_8$, m und X wie eingangs definiert sind und

A eine durch Hydrolyse in eine Carboxygruppe überführbare Gruppe darstellt.

Als derartige hydrolysierbare Gruppen kommen beispielsweise die Nitrilgruppe, funktionelle Derivate der Carboxygruppe wie deren unsubstituierte oder substituierte Amide, Ester, Thioester, Orthoester, Iminoäther, Amidine oder Anhydride, eine Malonester-(1)-yl-gruppe, die Tetrazolylgruppe, eine gegebenenfalls substituierte 1,3-Oxazol-(2)-yl- oder Dihydro-1,3-oxazol-(2)-yl-gruppe in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten

Lösungsmittel wie Wasser, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet in einer Verbindung der allgemeinen Formel II, B die Cyangruppe, so wird die Umsetzung zweckmäßigerweise in Gegenwart von Äthanol/Chlorwasserstoff durchgeführt, hierbei bildet sich im Reaktionsgemisch der entsprechende Imino- und Orthoester bzw. nach Wasserzugabe der entsprechende Ester, welcher nach Zugabe von Wasser hydrolysiert wird.

b) Zur Herstellung von Verbindungen der allgemeinen Formel Ia, in der $R_5$ eine Aminogruppe darstellt:

Reduktion einer Nitroverbindung der allgemeinen Formel

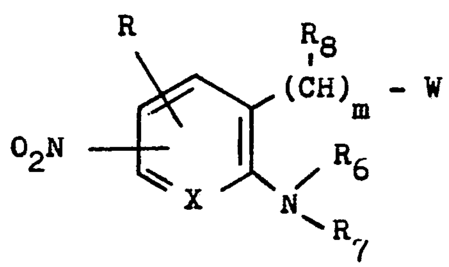
,(III)

in der
R, $R_6$ bis $R_8$, m, W und X wie eingangs definiert sind.

Die Reduktion wird zweckmäßigerweise in einem Lösungsmittel wie Methanol, Äthanol, Wasser, Wasser/Äthanol, Dioxan, Methanol/Dioxan, Essigester, Dimethylformamid oder Dioxan/Dimethylformamid mit katalytisch angeregtem Wasserstoff, z.B. mit Wasserstoff in Gegenwart eines Hydrierungskatalysators wie Palladium/Kohle, Platin oder Raney-Nickel bei einem Wasserstoffdruck von 1 bis 10 bar, mit Hydrazin

in Gegenwart von Raney-Nickel, mit nascierendem Wasserstoff, z.B. mit Zink/Essigsäure, Zinn/Salzsäure oder Eisen/Salzsäure, oder mit einem Metallsalz, z.B. Zinn-(II)-chlorid/Salzsäure oder Eisen-(II)-sulfat/Schwefelsäure, bei Temperaturen zwischen 0 und 50°C, vorzugsweise jedoch bei Raumtemperatur, durchgeführt.

c) Zur Herstellung von Verbindungen der allgemeinen Formel Ia, in der $R_5$ eine Hydroxygruppe, eine Cyangruppe, ein Wasserstoff-, Fluor-, Chlor- oder Bromatom darstellt:

Umsetzung einer Verbindung der allgemeinen Formel

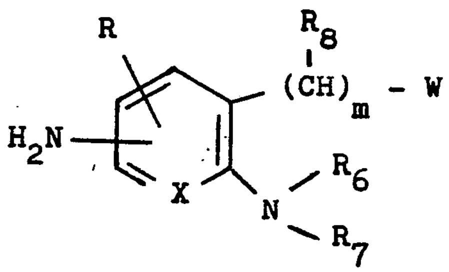

,(IV)

in der
R, $R_6$ bis $R_8$, m, W und X wie eingangs definiert sind, mit einem Nitrit und anschließendes Erwärmen des so erhaltenen Diazoniumsalzes gegebenenfalls in Gegenwart von Kupfer oder eines entsprechenden Kupfer-(I)-Salzes.

Die Umsetzung wird zweckmäßigerweise in der Weise durchgeführt, daß eine Verbindung der allgemeinen Formel IV, in einem geeigneten Lösungsmittel, z.B. in Wasser/Salzsäure, Methanol/Salzsäure oder Dioxan/Salzsäure, mit einem Nitrit, z.B. Natriumnitrit oder einem Ester der salpetrigen Säure, in ein Diazoniumsalz bei niederen Temperaturen, z.B. bei Temperaturen zwischen -10 und 5°C, übergeführt wird.

Das so erhaltene entsprechende Diazoniumsalz wird anschließend, z.B. als Fluorborat, als Hydrosulfat in Schwefelsäure, als Hydrochlorid in Gegenwart von Kupfer oder in Gegenwart eines entsprechenden Kupfer-(I)-Salzes wie Kupfer-(I)-chlorid/Salzsäure, Kupfer-(I)-bromid/Bromwasserstoffsäure oder Trinatrium-kupfer-(I)-tetracyanid bei pH 7, durch Erwärmen, z.B. auf Temperaturen zwischen 15 und 90°C, in die entsprechende Verbindung übergeführt.

d) Zur Herstellung von Verbindungen der allgemeinen Formel Ia, in der $R_5$ ein Wasserstoffatom darstellt:

Enthalogenierung einer Verbindung der allgemeinen Formel

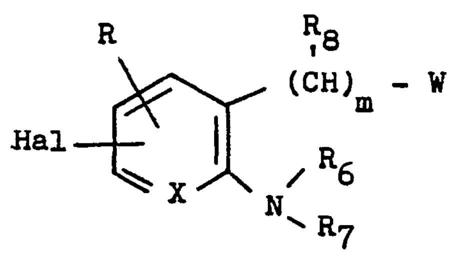

,(V)

in der
$R$, $R_6$ bis $R_8$, m, X und W wie eingangs definiert sind und Hal ein Halogenatom, z.B. ein Chlor-, Brom- oder Jodatom, darstellt.

Die Enthalogenierung wird vorzugsweise in einem Lösungsmittel wie Methanol, Äthanol, Essigsäureäthylester oder Eisessig mittels katalytisch angeregtem Wasserstoff, z.B. mit Wasserstoff in Gegenwart von Platin, Palladium/Kohle oder Raney-Nickel, bei Temperaturen zwischen 0 und 75°C, vorzugsweise jedoch bei Raumtemperatur, und bei einem Wasserstoffdruck von 1-5 bar durchgeführt.

e) Zur Herstellung von Verbindungen der allgemeinen Formel Ia, in der W die Carboxygruppe darstellt:

Umsetzung einer metallorganischen Verbindung der allgemeinen Formel

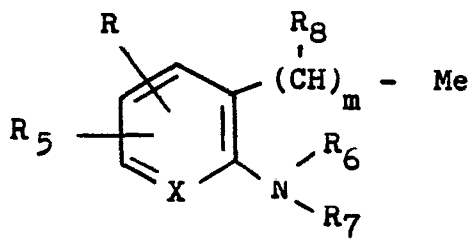
,(VI)

in der
R, $R_5$ bis $R_8$, m und X wie eingangs definiert sind und Me ein Alkaliatom, vorzugsweise ein Lithiumatom, oder einen Erdalkalihalogenidrest, vorzugsweise den Magnesiumchlorid- oder Magnesiumbromidrest, darstellt, mit Kohlendioxid.

Die Umsetzung wird vorzugsweise in der Weise durchgeführt, daß eine Verbindung der allgemeinen Formel VI, gegebenenfalls gelöst in einem inerten Lösungsmittel wie Diäthyläther oder Tetrahydrofuran, in festes Kohlendioxid eingetragen wird.

f) Zur Herstellung von Verbindungen der allgemeinen Formel Ia, in der X ein Stickstoffatom darstellt:

Umsetzung einer Verbindung der allgemeinen Formel

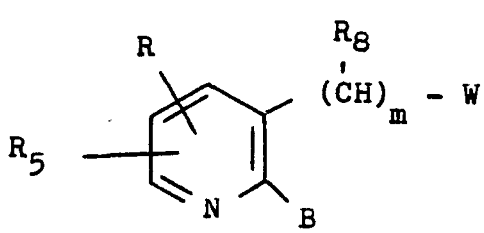
(VII)

in der
R, $R_5$, $R_8$, W und m wie eingangs definiert sind und
B einen austauschbaren Rest wie ein Halogenatom oder eine Alkylsulfonylgruppe, z.B. ein Chlor- oder Bromatom, eine Methyl- oder Äthylsulfonylgruppe, bedeutet, mit einem Amin der allgemeinen Formel

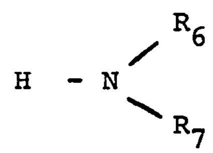
,(VIII)

in der
$R_6$ und $R_7$ wie eingangs definiert sind.

Die Umsetzung wird zweckmäßigerweise in einem Lösungsmittel wie Wasser, Wasser/Methanol, Wasser/Äthanol, Wasser/Isopropanol, Dimethylformamid oder in einem Überschuß des eingesetzten Amins der allgemeinen Formel VIII gegebenenfalls in Gegenwart einer anorganischen oder tertiären organischen Base, gegebenenfalls in Gegenwart eines Reaktionsbeschleunigers wie Kupfer und gegebenenfalls in einem Druckgefäß bei Temperaturen zwischen 20 und 150°C, vorzugsweise jedoch bei der Siedetemperatur des Reaktionsgemisches, z.B. bei Temperaturen zwischen 80 und 100°C, durchgeführt. Die Umsetzung kann jedoch auch ohne Lösungsmittel durchgeführt werden.

g) Zur Herstellung von Verbindungen der allgemeinen Formel Ia, in der $R_5$ eine Alkoxygruppe mit 1 bis 4 Kohlenstoffatomen darstellt:

Alkylierung einer Hydroxyverbindung der allgemeinen Formel

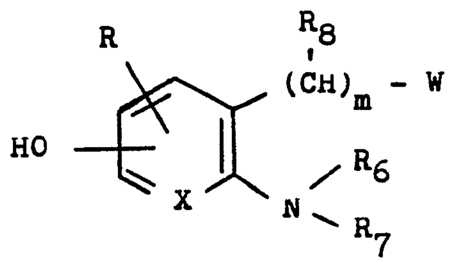

,(IX)

in der

R, $R_6$ bis $R_8$, W, X und m wie eingangs definiert sind, mit einer Verbindung der allgemeinen Formel

$$D - R_5' \quad ,(X)$$

in der

$R_5$' eine Alkylgruppe mit 1 bis 4 Kohlenstoffatomen und D eine nukleophile Austrittsgruppe oder zusammen mit dem benachbarten H-Atom des Restes $R_5$' eine Diazogruppe bedeuten, und erforderlichenfalls anschließende Hydrolyse.

Als Austrittsgruppe kommt beispielsweise ein Chlor-, Brom- oder Jodatom oder eine Sulfonyloxygruppe wie die Methansulfonyloxy-, p-Toluolsulfonyloxy- oder Methoxysulfonyloxygruppe in Betracht.

Die Umsetzung wird zweckmäßigerweise in einem Lösungsmittel wie Äther, Tetrahydrofuran, Äthanol, Aceton, Dimethylformamid oder Dimethylsulfoxid gegebenenfalls in Gegenwart einer Base wie Natriumkarbonat, Kaliumkarbonat, Bariumhydroxid, Natriumäthylat oder Kalium-tert.butylat mit einem Alkylierungsmittel wie Diazomethan, Diazoäthan, Methyljodid, Äthyljodid, Isopropylbromid, Butylbromid, Dimethylsulfat, Diäthylsulfat oder p-Toluolsulfonsäuremethylester bei Temperaturen zwischen 0 und 100°C, vorzugsweise jedoch bei Temperaturen zwischen 15 und 70°C, durchgeführt.

Bedeutet hierbei W eine Carboxygruppe, so wird diese bei der Alkylierung mit einem Diazoalkan oder bei der Alkylierung in Gegenwart einer starken Base gleichzeitig verestert. Der so erhaltene Ester wird anschließend gewünschtenfalls mittels Hydrolyse in Gegenwart einer Säure oder Base in die entsprechende Carbonsäure übergeführt.

Eine erfindungsgemäß erhaltene Verbindung der allgemeinen Formel Ia, in der W eine Alkoxycarbonylgruppe darstellt, kann anschließend mittels Hydrolyse in eine entsprechende Verbindung der allgemeinen Formel Ia, in der W die Carboxygruppe darstellt, übergeführt werden.

Die nachträgliche Hydrolyse wird vorzugsweise in einem mit Wasser mischbaren Lösungsmittel wie Methanol, Äthanol, Dioxan, Wasser/Äthanol oder Wasser/Tetrahydrofuran in Gegenwart einer Säure wie Salzsäure oder Schwefelsäure oder einer Base wie Natrium- oder Kaliumhydroxid bei erhöhten Temperaturen, z.B. bei der Siedetemperatur des Reaktionsgemisches, durchgeführt.

2. Herstellung der Carbonsäure-Derivate der allgemeinen Formel I:

a) Umsetzung einer Carbonsäure der allgemeinen Formel

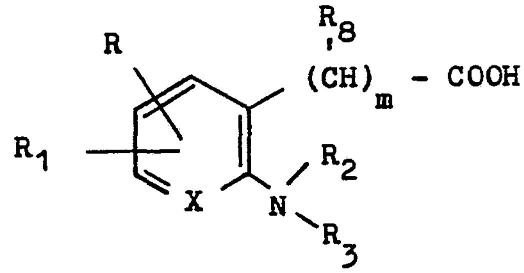

,(XI)

in der
R, $R_1$, $R_2$, $R_3$, $R_8$, X und m wie eingangs definiert sind, oder deren gegebenenfalls im Reaktionsgemisch hergestelltes reaktionsfähiges Derivat mit einem Amin der allgemeinen Formel

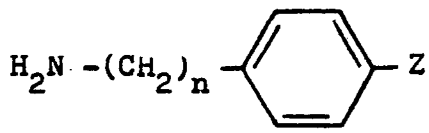

,(XII)

in der
Z und n wie eingangs definiert sind, oder mit einem gegebenenfalls im Reaktionsgemisch gebildeten N-aktivierten Amin der allgemeinen Formel XII, wenn eine Carbonsäure der allgemeinen Formel XI eingesetzt wird und wenn Z in einem N-aktivierten Amin der allgemeinen Formel XII keine Carboxygruppe darstellt.

Das Verfahren betrifft somit die Acylierung eines Amins der allgemeinen Formel XII mit einer Carbonsäure der allgemeinen Formel XI in Gegenwart eines die Säure aktivierenden oder eines wasserentziehenden Mittels oder mit deren funktionellen Derivaten oder
die Umsetzung einer Carbonsäure der allgemeinen Formel XI mit einem Amin der allgemeinen Formel XII, in der Z keine Carboxygruppe darstellt, in Gegenwart eines die Aminogruppe aktivierenden Mittels oder mit dessen reaktionsfähigen Derivaten.

Als gegebenenfalls im Reaktionsgemisch hergestellte funktionelle Derivate einer Carbonsäure der allgemeinen Formel XI kommen beispielsweise deren Alkyl-, Aryl- oder Aralkylester oder -thioester wie der Methyl-, Äthyl-, Phenyl- oder Benzylester, deren Imidazolide, deren Säurehalogenide wie das Säurechlorid oder -bromid, deren Anhydride, deren gemischte Anhydride mit aliphatischen oder aromatischen Carbon-, Sulfen-, Sulfin- oder Sulfonsäuren oder Kohlensäureestern, z.B. der Essigsäure, Propionsäure, p-Toluolsulfonsäure oder der O-Äthyl-kohlensäure, deren O-Triphenylphosphonium- oder Kupfer-Komplexe, deren N-Acyloxyimide, deren Azide oder Nitrile oder die entsprechenden Amino-thiocarbonsäure-Derivate, und als gegebenenfalls im Reaktionsgemisch hergestellte reaktionsfähige Derivate eines Amins der allgemeinen Formel XII, wenn Z keine Carboxygruppe darstellt, deren Phosphazoderivate in Betracht.

Als säureaktivierende und/oder wasserentziehende Mittel kommen beispielsweise ein Chlorameisensäureester wie Chlorameisensäureäthylester, Thionylchlorid, Phosphortrichlorid, Phosphorpentoxid, N,N'-Dicyclohexylcarbodiimid, N,N'-Carbonyldiimidazol, N,N'-Thionyldiimidazol, Bortrifluoridätherat oder Triphenylphosphin/Tetrachlorkohlenstoff in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart einer anorganischen Base wie Natriumkarbonat oder einer tertiären organischen Base wie Triäthylamin oder Pyridin, welche gleichzeitig als Lösungsmittel dienen kann, und gegebenenfalls in Gegenwart eines säureaktivierenden Mittels bei Temperaturen zwischen -25 und 250°C, vorzugsweise jedoch bei Temperaturen zwischen -10°C und der Siedetemperatur des verwendeten Lösungsmittels, durchgeführt. Hierbei braucht ein gegebenenfalls im Reaktionsgemisch entstandenes funktionelles Derivat einer Verbindung der allgemeinen Formel XI oder XII nicht isoliert zu werden, ferner kann die Umsetzung auch ohne Lösungsmittel durchgeführt werden. Desweiteren kann während der Umsetzung entstehendes Wasser durch azeotrope Destillation, z.B. durch Erhitzen mit Toluol am Wasserabscheider, oder durch Zugabe eines Trockenmittels wie Magnesiumsulfat oder Molekularsieb abgetrennt werden.

b) Zur Herstellung von Verbindungen der allgemeinen Formel I, in der X eine CH-Gruppe und m die Zahl O darstellt:

Umsetzung einer Verbindung der allgemeinen Formel

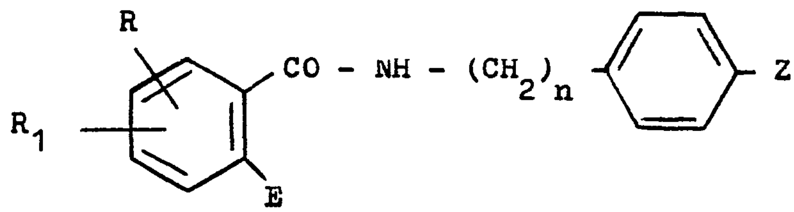
,(XIII)

in der R, $R_1$, Z und n wie eingangs definiert sind und E einen austauschbaren Rest wie ein Halogenatom darstellt, mit einem Amin der allgemeinen Formel

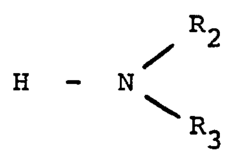
,(XIV)

in der $R_2$ und $R_3$ wie eingangs definiert sind.

Unter den bei der Definition des austauschbaren Restes E verwendeten Begriff "ein Halogenatom" ist insbesondere ein Chlor- oder Bromatom zu verstehen.

Die Umsetzung wird zweckmäßigerweise in einem Lösungsmittel wie Wasser, Wasser/Methanol, Wasser/Äthanol, Wasser/Isopropanol, Dimethylformamid oder in einem Überschuß des eingesetzten Amins der allgemeinen Formel XIV gegebenenfalls in Gegenwart einer anorganischen oder tertiären organischen Base, gegebenenfalls in Gegenwart eines Reaktionsbeschleunigers wie Kupfer und gegebenenfalls in einem

Druckgefäß bei Temperaturen zwischen 20 und 150°C, vorzugsweise jedoch bei der Siedetemperatur des Reaktionsgemisches, z.B. bei 100°C, durchgeführt. Die Umsetzung kann jedoch auch ohne Lösungsmittel durchgeführt werden.

c) Zur Herstellung von Verbindungen der allgemeinen Formel I, in der Z eine Carboxygruppe darstellt:

Oxidation einer Verbindung der allgemeinen Formel

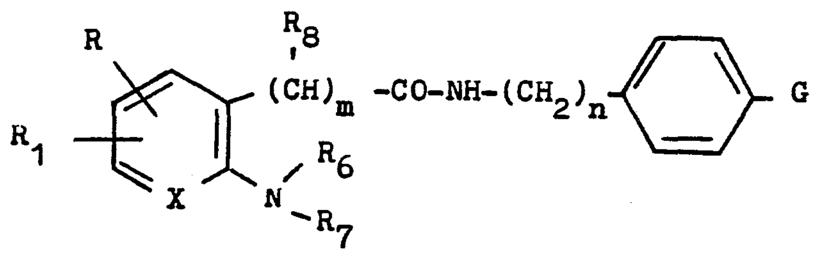

,(XV)

in der
R, $R_1$ bis $R_3$, $R_8$, X, m und n wie eingangs definiert sind und
G eine durch Oxidation in eine Carboxygruppe überführbare Gruppe bedeutet.

Als eine derartige oxidierbare Gruppe kommen beispielsweise die Formylgruppe und deren Acetale, die Hydroxymethylgruppe und deren Äther, eine unsubstituierte oder substituierte Acylgruppe wie die Acetyl-, Chloracetyl-, Propionyl- oder Malonsäure-(1)-yl-gruppe oder eine Malonester-(1)-yl-gruppe in Betracht.

Die Umsetzung wird mit einem Oxidationsmittel in einem geeigneten Lösungsmittel wie Wasser, Eisessig, Pyridin oder Tetrachlorkohlenstoff bei Temperaturen zwischen 0 und 100°C, zweckmäßigerweise jedoch bei Temperaturen zwischen 20 und 50°C, durchgeführt. Die Umsetzung wird jedoch vorzugsweise mit Silberoxid/Natronlauge, Mangandioxid/Aceton

oder Methylenchlorid, Wasserstoffperoxid/Natronlauge, Brom oder Chlor/Natron- oder Kalilauge oder Chromtrioxid/ Pyridin durchgeführt.

d) Zur Herstellung von Verbindungen der allgemeinen Formel I, in der Z eine Carboxygruppe darstellt:

Hydrolyse einer Verbindung der allgemeinen Formel

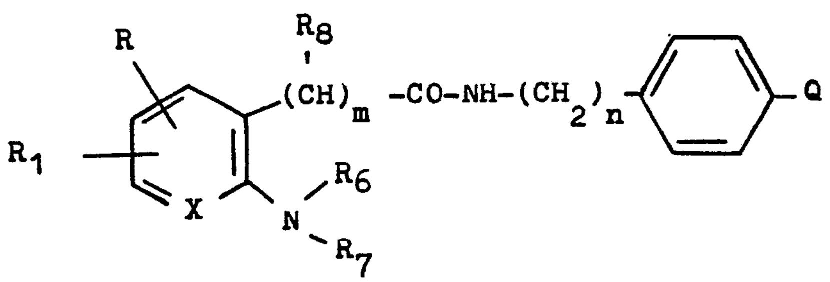
,(XVI)

in der

R, $R_1$ bis $R_3$, $R_8$, X, m und n wie eingangs definiert sind und

Q eine durch Hydrolyse in eine Carboxygruppe überführbare Gruppe darstellt.

Als derartige hydrolysierbare Gruppen kommen beispielsweise die Nitrilgruppe, funktionelle Derivate der Carboxygruppe wie deren unsubstituierte oder substituierte Amide, Ester, Thioester, Orthoester, Iminoäther, Amidine oder Anhydride, eine Malonester-(1)-yl-gruppe, die Tetrazolylgruppe, eine gegebenenfalls substituierte 1,3-Oxazol-(2)-yl- oder Dihydro-1,3-oxazol-(2)-yl-gruppe in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Äthanol, Wasser/Äthanol, Wasser/

Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Bedeutet in einer Verbindung der allgemeinen Formel XVI, Q die Cyangruppe, so wird die Umsetzung zweckmäßigerweise in Gegenwart von Äthanol/Chlorwasserstoff durchgeführt, hierbei bildet sich im Reaktionsgemisch der entsprechende Imino- und Orthoester bzw. nach Wasserzugabe der entsprechende Ester, welcher nach Zugabe von Wasser hydrolysiert wird.

e) Zur Herstellung von Verbindungen der allgemeinen Formel I, in der
X eine CH-Gruppe und m die Zahl O darstellt:

Umsetzung eines Amids der allgemeinen Formel

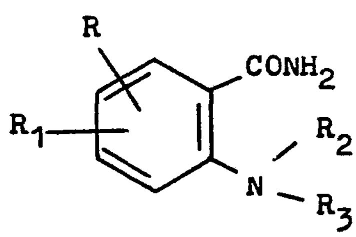

,(XVII)

in der
R und $R_1$ bis $R_3$ wie eingangs definiert sind, oder dessen Alkalisalz mit einer Verbindung der allgemeinen Formel

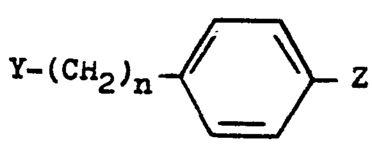

,(XVIII)

in der
Z und n wie eingangs definiert sind und
Y eine nukleophile Austrittsgruppe wie ein Halogenatom oder eine Sulfonyloxygruppe darstellt.

Die Umsetzung wird zweckmäßigerweise in einem Lösungsmittel wie Tetrahydrofuran, Dioxan, Toluol, Dimethylformamid, Dimethylsulfoxid oder Hexamethylphosphorsäuretriamid gegebenenfalls in Gegenwart einer Base wie Natriumhydrid oder Kalium-tert.butylat bei Temperaturen zwischen 20 und 180°C, vorzugsweise jedoch bei Temperaturen zwischen 50 und 150°C, durchgeführt.

f) Zur Herstellung von Verbindungen der allgemeinen Formel I, in der
X eine CH-Gruppe und
Z eine Carboxygruppe darstellen:

Acylierung einer Verbindung der allgemeinen Formel

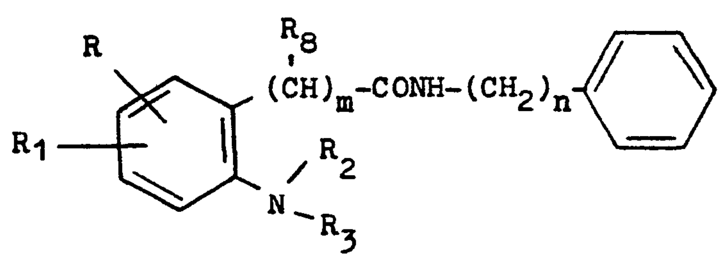

,(XIX)

in der
R, $R_1$ bis $R_3$, $R_8$, m und n wie eingangs definiert sind,
mit einem Oxalylhalogenid oder Phosgen in Gegenwart einer Lewis-Säure.

Die Friedel-Crafts-Acylierung wird zweckmäßigerweise in einem Lösungsmittel wie Nitrobenzol oder Schwefelkohlenstoff in Gegenwart einer Lewis-Säure wie Aluminiumchlorid bei Temperaturen zwischen 0 und 80°C, vorzugsweise jedoch bei Temperaturen zwischen 20 und 60°C, durchgeführt.

g) Zur Herstellung von Verbindungen der allgemeinen Formel I, in der
X eine CH-Gruppe und
Z die Carboxygruppe bedeutet:

Umsetzung einer Verbindung der allgemeinen Formel

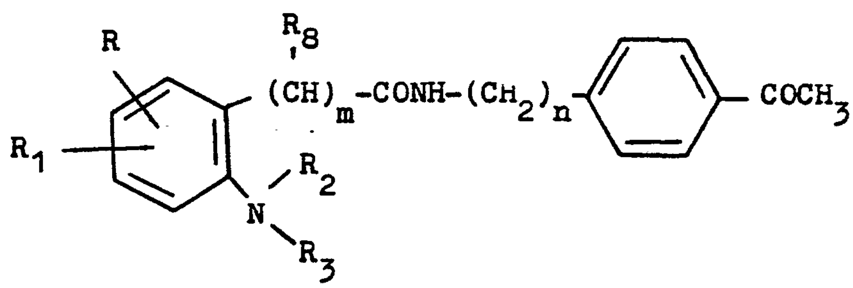
,(XX)

in der
R, $R_1$ bis $R_3$, $R_8$, m und n wie eingangs definiert sind,
mit einem gegebenenfalls im Reaktionsgemisch hergestellten Hypohalogenit.

Die Umsetzung wird zweckmäßigerweise in einem Lösungsmittel wie Wasser/Tetrahydrofuran oder Wasser/Dioxan bei Temperaturen zwischen 0 und 80°C, vorzugsweise jedoch bei Temperaturen zwischen 25 und 50°C, durchgeführt.

Erhält man erfindungsgemäß ein Carbonsäureamid der allgemeinen Formel I, in der Z eine Carboxygruppe darstellt, so kann dieses gewünschtenfalls mittels Veresterung in eine entsprechende Verbindung der allgemeinen Formel I, in der Z eine veresterte Carboxygruppe darstellt, übergeführt werden.

Die nachträgliche Veresterung wird zweckmäßigerweise in einem geeigneten Lösungsmittel, z.B. in einem entsprechenden Alkohol, Pyridin, Toluol oder Dioxan, in Gegenwart eines säureaktivierenden und/oder wasserentziehenden Mittels wie Thionylchlorid, Chlorameisensäureäthylester, N,N'-Dicyclohexylcarbodiimid oder Carbonyldiimidazol oder durch Umesterung, z.B. mit einem entsprechenden Kohlensäurediester, bei Temperaturen zwischen 0 und $50^{o}C$, vorzugsweise jedoch bei Raumtemperatur, durchgeführt.

Die erhaltenen Verbindungen der allgemeinen Formeln I und Ia lassen sich ferner in ihre physiologisch verträglichen Salze mit anorganischen oder organischen Säuren oder auch Basen, wenn W oder Z eine Carboxygruppe darstellt, überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure oder Fumarsäure und als Basen Natriumhydroxid, Kaliumhydroxid oder Cylohexylamin in Betracht.

Die als Ausgangsstoffe verwendeten Verbindungen der allgemeinen Formeln II bis XX sind literaturbekannt, bzw. man erhält sie nach an sich bekannten Verfahren. So erhält man beispielsweise eine Verbindung der allgemeinen Formel II durch Reduktion einer entsprechenden Nitroverbindung und gegebenenfalls anschließender Sandmeyer-Reaktion und gegebenenfalls anschließender Alkylierung.

Eine Verbindung der allgemeinen Formel III erhält man beispielsweise durch Nitrierung einer entsprechenden Verbindung, durch Umsetzung einer entsprechenden metallorganischen Verbindung mit Kohlendioxid oder durch Umsetzung eines entsprechenden Benzylhalogenids mit Kaliumcyanid und gegebenenfalls anschließender Hydrolyse und/oder Veresterung.

Eine Verbindung der allgemeinen Formel IV oder XI erhält man beispielsweise durch Umsetzung einer entsprechenden 2-Chlor- oder 2-Brom-nitro-carbonsäure oder deren Derivate mit einem entsprechenden Amin, anschließende Reduktion der Nitrogruppe in

einer so erhaltenen 2-Aminoverbindung mittels katalytisch angeregtem Wasserstoff, mittels nascierendem Wasserstoff, mittels Metallen oder Metallsalzen und Überführung der so erhaltenen Aminogruppe über ein entsprechendes Diazoniumsalz in eine entsprechende Verbindung der allgemeinen Formel IV oder XI. Zur Herstellung einer Ausgangsverbindung der allgemeinen Formel XI, in der $R_1$ eine Alkoxygruppe darstellt, wird eine so hergestellte Hydroxy-carbonsäure anschließend alkyliert und erforderlichenfalls falls anschließend hydrolysiert.

Beispielsweise erhält man eine Verbindung der allgemeinen Formel V durch Sandmeyer-Reaktion einer entsprechenden Aminoverbindung, eine Verbindung der allgemeinen Formel VI durch Metallierung einer entsprechenden Halogenverbindung, eine Verbindung der allgemeinen Formel VII durch Halogenierung einer entsprechenden Pyridinverbindung und eine Verbindung der allgemeinen Formel IX durch Verkochen eines entsprechenden Diazoniumsalzes.

Eine Verbindung der allgemeinen Formel XI, in der $R_1$ eine Alkyl- oder Trifluormethylgruppe darstellt, erhält man beispielsweise durch Reduktion eines entsprechenden Nitrobenzolderivates, Sandmeyer-Reaktion der erhaltenen Aminoverbindung mit Kupfer(I)-bromid, Metallierung der erhaltenen Bromverbindung mit Butyllithium und anschließende Umsetzung mit Kohlendioxid.

Eine Verbindung der allgemeinen Formel XII erhält man beispielsweise durch Umsetzung eines entsprechenden 4-Brommethyl-benzol-Derivates mit Natriumcyanid und anschließende katalytische Hydrierung der so erhaltenen Cyanoverbindung.

Eine als Ausgangsstoff verwendete Verbindung der allgemeinen Formeln XIII, XV bis XVII, XIX und XX erhält man durch Umsetzung einer entsprechenden Carbonsäure mit einem entsprechenden Amin in Gegenwart eines die Säure aktivierenden oder wasserentziehenden Mittels.

Eine als Ausgangsstoff verwendete Verbindung der allgemeinen Formeln XVIII erhält man durch Halogenierung eines entsprechenden Alkohols oder Umsetzung eines Sulfonsäurehalogenids mit einem entsprechenden Alkohol in Gegenwart einer Base.

Wie bereits eingangs erwähnt, weisen die neuen Verbindungen der allgemeinen Formeln I und Ia wertvolle pharmakologische Eigenschaften auf, nämlich eine Wirkung auf den Intermediärstoffwechsel. So besitzen die Verbindungen der allgemeinen Formel I insbesondere eine blutzuckersenkende und/oder lipidsenkende Wirkung und die Verbindungen der allgemeinen Formel Ia eine lipidsenkende Wirkung. Außerdem stellen die Verbindungen der allgemeinen Formel Ia wertvolle Zwischenprodukte zur Herstellung der neuen Verbindungen der allgemeinen Formel I dar.

Beispielsweise wurden die Verbindungen

A = 4-[2-[2-(2-Azabicyclo[3.3.1]nonan-2-yl)-5-chlor-benzoylamino]-äthyl]benzoesäure,

B = 4-[2-[5-Fluor-2-(octahydro-1H-azonino)-benzoylamino]äthyl]-benzoesäure,

C = 4-[2-[5-Chlor-2-(cis-3,5-dimethylpiperidino)-nicotinoylamino]-äthyl]benzoesäure und

D = 4-[2-[5-Methyl-2-(octahydro-1H-azonino)-benzoylamino]-äthyl]-benzoesäure

im Vergleich zu

E = 4-[2-(2-Äthylamino-5-chlor-benzoylamino)-äthyl]benzoesäure (siehe Beispiel 5 der BE-PS 837 311)

auf ihre blutzuckersenkenden Eigenschaften und die Verbindungen

F = 5-Chlor-2-[4-(3-pentyl)-piperidino]benzoesäure,

G = 5-Brom-2-(octahydro-isoindol-2-yl)-benzoesäure,

H = 5-Chlor-2-(2,3,3a,4,7,7a-hexahydro-1H-isoindol-2-yl)-benzoesäure und

I = 5-Chlor-2-(octahydro-isoindol-2-yl)-nicotinsäure

auf ihre lipidsenkenden Eigenschaften wie folgt untersucht:

1. <u>Blutzuckersenkende Wirkung:</u>

Die blutzuckersenkende Wirkung der zu untersuchenden Substanzen wurde an weiblichen Ratten eigener Zucht mit dem Gewicht von 180-220 g geprüft, welche 24 Stunden vor Versuchsbeginn nüchtern gesetzt wurden. Die zu untersuchenden Substanzen wurden unmittelbar vor Versuchsbeginn in 1,5%-iger Methylcellulose suspendiert und per Schlundsonde appliziert.

Die Blutentnahme erfolgte unmittelbar vor Substanzapplikation, sowie 1, 2, 3 und 4 Stunden danach jeweils aus dem retroorbitalen Venenplexus. Hiervon wurden jeweils 50 µl mit 0,5 ml 0,33 N Perchlorsäure enteiweißt und zentrifugiert. Im Überstand wurde Glukose nach der Hexokinase-Methode mit Hilfe eines Analysenphotometers bestimmt. Die statistische Auswertung erfolgte nach dem t-Test nach Student mit $p = 0,05$ als Signifikanzgrenze.

Die nachfolgende Tabelle enthält die gefundenen Werte in Prozent gegenüber Kontrolle:

Tabelle 1

| Substanz | 25 mg/kg | | | | 5 mg/kg | | | |
|---|---|---|---|---|---|---|---|---|
| | 1 | 2 | 3 | 4 | 1 | 2 | 3 | 4 |
| | Stunden | | | | Stunden | | | |
| A | | | | | -42 | -43 | -36 | -32 |
| B | | | | | -31 | -36 | -34 | -34 |
| C | | | | | -37 | -39 | -40 | -38 |
| D | | | | | -30 | -34 | -35 | -26 |
| E | n.s. | n.s. | n.s. | n.s. | | | | |

n.s. ≙ statistisch nicht signifikant

2. Lipidsenkende Wirkung:

Literatur: P. E. Schurr et al. in Atherosclerosis Drug Discovery (1976), Herausgeber: C. E. Day; Plenum, New York, Seite 215.

Junge männliche Ratten mit einem durchschnittlichen Gewicht von 100 g wurden durch viertägige Gabe einer Diät (bestehend aus 10 % Kokosfett, 1,5 % Cholesterin, 0,5 % Cholsäure, 0,2 % Cholinchlorid und 15 % Sucrose) hyperlipämisch gemacht. Unter Beibehaltung der Diät appliziert man an zwei aufeinanderfolgenden Tagen die zu untersuchende Substanz in Methylcellulose-Suspension per Schlundsonde. Anschließend wurden die Tiere über Nacht nüchtern gehalten, 4 und 24 Stunden nach der letzten Substanzapplikation wurde Blut zur Serumgewinnung entnommen.

Im Serum wurden Gesamtcholesterin (Boehringer Mannheim Testkombination 187.313) und Triglyceride (Boehringer Mannheim Testkombination 126.039) enzymatisch bestimmt. Die ß-Lipoproteine wurden nach Fällung mit $Ca^{++}$ und Heparin im Autoanalyzer nephelometrisch bestimmt.

Die Berechnung der prozentualen Senkung erfolgte gegen eine Kontrollgruppe.

Die folgende Tabelle enthält die gefundenen Werte:

| Substanz | Dosis [mg/kg] | Änderung in % gegenüber Kontrolle nach zweimaliger Applikation | | | |
|---|---|---|---|---|---|
| | | Serum-Gesamt cholesterin | | Serum ß-Lipo-proteine | |
| | | 4 Stunden | 24 Stunden | 4 Stunden | 24 Stunden |
| F | 5 | - 18 | — | — | - 39 |
| | 20 | - 49 | - 54 | - 54 | - 69 |
| | 50 | - 54 | - 43 | - 61 | - 57 |
| G | 5 | - | - 28 | - | - 42 |
| | 20 | - 37 | - 51 | - 57 | - 66 |
| | 50 | - 40 | - 47 | - 47 | - 42 |
| H | 5 | — | - 34 | — | - 36 |
| | 20 | - 43 | - 61 | - 41 | - 62 |
| | 50 | - 50 | - 65 | - 54 | - 72 |
| I | 5 | - 43 | - 45 | - | - 47 |
| | 20 | - 48 | - 50 | - 45 | - 62 |
| | 50 | - 53 | - 48 | - 60 | - 67 |

Die Veränderungen sind statistisch signifikant bei p= 0,05.

3. <u>Akute Toxizität:</u>

Bei weiblichen und männlichen Mäusen eigener Zucht mit dem Gewicht von 20-26 g wurde die toxische Wirkung der Substanzen nach oraler Gabe (Suspension in 1%iger Methylcellulose) einer einmaligen Dosis bei einer Nachbeobachtungszeit von mindestens 7 Tagen geprüft. Die nachfolgende Tabelle enthält die gefundenen Werte:

| Substanz | orientierende Toxizität |
|---|---|
| C | > 1 000 mg/kg (0 von 10 Tieren gestorben) |
| F | > 1 000 mg/kg (0 von 10 Tieren gestorben) |

Aufgrund ihrer pharmakologischen Eigenschaften eignen sich die erfindungsgemäß hergestellten Verbindungen der allgemeinen Formel I und deren physiologisch verträgliche Salze zur Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1-50 mg, vorzugsweise jedoch 2,5 - 20 mg, 1 oder 2 mal täglich.

Desweiteren eignen sich die erfindungsgemäß hergestellten Verbindungen der allgemeinen Formel Ia aufgrund ihrer pharmakologischen Eigenschaften zur Behandlung von Hyperlipidämien, insbesondere des Typs IIa, IIb und IV, und dadurch bedingten atherosklerotischen Veränderungen des Gefäßsystems und lassen sich zur pharmazeutischen Anwendung, gegebenenfalls in Kombination mit anderen Wirksubstanzen, in die üblichen pharmazeutischen Zubereitungen wie Dragées, Tabletten, Kapseln, Suppositorien, Suspensionen oder Lösungen einarbeiten, die Einzeldosis beträgt hierbei 5 bis 200 mg, vorzugsweise jedoch 5 bis 50 mg, und die Tagesdosis 10 bis 500 mg, vorzugsweise 15-150 mg.

Die nachfolgenden Beispiele sollen die Erfindung näher erläutern:

Beispiel A

2-(2-Azabicyclo/3.3.1/nonan-2-yl)-5-nitro-benzoesäure

In 600 ml Äthanol werden 40 g (320 mMol) 2-Azabicyclo/3.3.1/-nonan, 64,5 g (320 mMol) 2-Chlor-5-nitro-benzoesäure und 74,3 g Natriumcarbonat 4 Tage auf Rückflußtemperatur erhitzt. Nach Abdestillieren des Äthanols wird der Abdampfrückstand in 800 ml Wasser gelöst, mit 2 N Salzsäure auf pH 4 eingestellt und anschließend mit Chloroform ausgeschüttelt. Die Chloroformphasen werden über Natriumsulfat getrocknet und der Abdampfrückstand wird über eine Kieselgelsäule mit Toluol/Essigsäureäthylester (6:4) als Fließmittel chromatographisch gereinigt.

Ausbeute: 11,6 g (12,5 % der Theorie),

Schmelzpunkt: 167°C

| | | | | | | |
|---|---|---|---|---|---|---|
| Ber.: | C | 62,05 | H | 6,25 | N | 9,65 |
| Gef.: | | 61,72 | | 5,98 | | 9,83 |

Beispiel B

5-Nitro-2-/4-(3-pentyl)-piperidino/benzoesäure

Hergestellt analog Beispiel A aus 2-Chlor-5-nitro-benzoesäure und 4-(3-Pentyl)-piperidin.

Ausbeute: 60 % der Theorie,

Schmelzpunkt: 146°C

| | | | | | | |
|---|---|---|---|---|---|---|
| Ber.: | C | 63,73 | H | 7,55 | N | 8,74 |
| Gef.: | | 63,65 | | 7,60 | | 8,82 |

Beispiel C

5-Nitro-2-/4-(5-nonyl)-piperidino/benzoesäure

Hergestellt analog Beispiel A aus 2-Chlor-5-nitro-benzoesäure und 4-(5-Nonyl)-piperidin.

Ausbeute: 73 % der Theorie,
Schmelzpunkt: 150-152°C

| | | | | | | |
|---|---|---|---|---|---|---|
| Ber.: | C | 66,99 | H | 8,57 | N | 7,44 |
| Gef.: | | 66,87 | | 8,43 | | 7,23 |

Beispiel D

5-Nitro-2-(octahydro-isoindol-2-yl)-benzoesäure

Hergestellt analog Beispiel A aus 2-Chlor-5-nitro-benzoesäure und Octahydro-isoindol.
Ausbeute: 77,5 % der Theorie,
Schmelzpunkt: 188-190°C

| | | | | | | |
|---|---|---|---|---|---|---|
| Ber.: | C | 62,05 | H | 6,25 | N | 9,64 |
| Gef.: | | 62,20 | | 6,19 | | 9,63 |

Beispiel E

3-Brom-4-(octahydro-1H-azonino)-toluol

12,0 g (52 mMol) 5-Methyl-2-(octahydro-1H-azonino)-anilin (hergestellt durch Reduktion von 3-Nitro-4-(octahydro-1H-azonino)-toluol) werden in 200 ml 48%iger wässriger Bromwasserstoffsäure gelöst und bei 5°C durch tropfenweise Zugabe einer Lösung von 3,8 g (55 mMol) Natriumnitrit in 10 ml Wasser diazotiert. Die so erhaltene Diazoniumsalzlösung wird dann rasch zu einer Suspension von 10,75 g (75 mMol) Kupfer-I-bromid und 4,0 g Kupferpulver in 100 ml 48%iger wässriger Bromwasserstoffsäure getropft. Man rührt eine Stunde bei 40°C nach, stellt dann alkalisch, extrahiert mit Methylenchlorid, trocknet die Extrakte über Natriumsulfat, filtriert und engt ein.
Ausbeute: 10,1 g (66 % der Theorie),
Schmelzpunkt: $<$ 20°C.

Beispiel F

3-Brom-4-(octahydro-isoindol-2-yl)-toluol

Hergestellt durch Sandmeyer-Reaktion analog Beispiel E ausgehend von 3-Amino-4-(octahydro-isoindol-2-yl)-toluol.
Ausbeute: 20 % der Theorie,
Schmelzpunkt: 116°C

| | | | | | | | | |
|---|---|---|---|---|---|---|---|---|
| Ber.: | C | 61,23 | H | 6,84 | N | 4,75 | Br | 27,15 |
| Gef.: | | 61,17 | | 7,00 | | 4,81 | | 26,75 |

Beispiel G

(5-Nitro-2-piperidino-phenyl)essigsäure

55 g (260 mMol) (2-Chlor-5-nitrophenyl)essigsäure /hergestellt durch Nitrierung von (2-Chlorphenyl)-essigsäure mit Nitriersäure, Schmelzpunkt: 127°C/ werden mit 230 ml Piperidin 48 Stunden auf Rückflußtemperatur erhitzt. Nach Zusatz von 400 ml Wasser wird mit Salzsäure auf pH 5,5 eingestellt. Dabei fällt das Reaktionsprodukt aus, das nach dem Absaugen mit Aceton und Äther nachgewaschen wird.
Ausbeute: 64 g (93 % der Theorie),
Schmelzpunkt: 119°C

| | | | | | | |
|---|---|---|---|---|---|---|
| Ber.: | C | 59,08 | H | 6,10 | N | 10,60 |
| Gef.: | | 58,90 | | 6,15 | | 10,46 |

Beispiel H

(2-Octahydro-1H-azonino-5-nitro-phenyl)essigsäure

50 g (240 mMol) (2-Chlor-5-nitro-phenyl)essigsäure werden mit 260 ml Octahydro-1H-azonin 24 Stunden auf 110-120°C erhitzt. Nach dem Eindampfen zur Trockne wird in 400 ml Wasser gelöst, mit Salzsäure auf pH 2-3 eingestellt und mit Chloroform ausgeschüttelt. Die vereinigten Chloroformphasen werden mit verdünnter Natronlauge ausgeschüttelt und anschließend die wässrige

Phase nach erneutem Ansäuern mit Chloroform extrahiert; nach Trocknung und Abdestillieren des Chloroforms wird das entstandene Kristallisat mit Petroläther/Äther = 5:1 gewaschen.

Ausbeute: 31 g (42 % der Theorie),

Schmelzpunkt: 100-103°C

| | | | | | | |
|---|---|---|---|---|---|---|
| Ber.: | C | 62,72 | H | 7,24 | N | 9,04 |
| Gef.: | | 62,59 | | 7,44 | | 9,15 |

Beispiel I

/2-(Octahydro-isoindol-2-yl)-5-nitro-phenyl7-essigsäure

Hergestellt analog Beispiel H aus (2-Chlor-5-nitro-phenyl)-essigsäure und Octahydro-isoindol.

Ausbeute: 53 % der Theorie,

Schmelzpunkt: 228°C

| | | | | | | |
|---|---|---|---|---|---|---|
| Ber.: | C | 63,14 | H | 6,62 | N | 9,20 |
| Gef.: | | 62,99 | | 6,51 | | 9,13 |

Beispiel K

2-(2,3,3a,4,7,7a-Hexahydro-1H-isoindol-2-yl)-5-nitro-benzoesäure

Hergestellt analog Beispiel A aus 2-Chlor-5-nitro-benzoesäure und 2,3,3a,4,7,7a-Hexahydro-1H-isoindol.

Ausbeute: 85 % der Theorie,

Schmelzpunkt: 231°C

| | | | | | | |
|---|---|---|---|---|---|---|
| Ber.: | C | 62,49 | H | 5,59 | N | 9,71 |
| Gef.: | | 62,50 | | 5,67 | | 9,61 |

Beispiel L

2-(1,3-Dihydro-isoindol-2-yl)-5-nitro-benzoesäure

Hergestellt analog Beispiel A aus 2-Chlor-5-nitro-benzoesäure und 1,3-Dihydroisoindol.

Ausbeute: 52 % der Theorie,
Schmelzpunkt: 185-186°C

| | | | | | | |
|---|---|---|---|---|---|---|
| Ber.: | C | 63,37 | H | 4,25 | N | 9,85 |
| Gef.: | | 63,53 | | 4,30 | | 9,90 |

<u>Beispiel M</u>

<u>DL-2-(2-Chlor-5-nitro-phenyl)propionsäure</u>

Zu 2,9 g (157 mMol) DL-2-(2-Chlorphenyl)propionsäure, gelöst in 70 ml konzentrierter Schwefelsäure, werden bei 15°C 23,5 ml Nitriersäure (7,5 ml rauchende Salpetersäure der Dichte 1,5 und 16 ml konzentrierter Schwefelsäure) so langsam zugegeben, daß die Temperatur nicht über 0°C steigt. Nach beendeter Zugabe und einstündigem Nachrühren bei Raumtemperatur wird auf Eiswasser gegossen. Die ausgefallene Säure wird abgesaugt, in Chloroform gelöst und mit Wasser gewaschen. Die Chloroformextrakte werden getrocknet und der Eindampfrückstand mit Petroläther/Äther zur Kristallisation gebracht.
Ausbeute: 95 % der Theorie,
Schmelzpunkt: 129-131°C

| | | | | | | | | |
|---|---|---|---|---|---|---|---|---|
| Ber.: | C | 47,07 | H | 3,51 | N | 6,10 | Cl | 15,43 |
| Gef.: | | 46,82 | | 3,77 | | 6,15 | | 14,88 |

Analog wurden folgende Verbindungen hergestellt:

DL-2-/2-(Octahydro-isoindol-2-yl)-5-nitro-phenyl/propionsäure

DL-2-(2-Octahydro-1H-azonino-5-nitro-phenyl)-propionsäure

DL-2-(5-Nitro-2-piperidino-phenyl)-propionsäure

Beispiel N

2-(5-Chlor-1,3-dihydro-isoindol-2-yl)-5-nitro-benzoesäure

Hergestellt analog Beispiel A aus 2-Chlor-5-nitro-benzoesäure und 5-Chlor-1,3-dihydro-isoindol.
Ausbeute: 27 % der Theorie,
Schmelzpunkt: 163°C

| | | | | | | |
|---|---|---|---|---|---|---|
| Ber.: | C | 56,52 | H | 3,48 | N | 8,79 |
| Gef.: | | 56,83 | | 3,57 | | 8,85 |

Beispiel O

2-(1,3-Dihydro-5-methoxy-isoindol-2-yl)-5-nitro-benzoesäure

Hergestellt analog Beispiel A aus 2-Chlor-5-nitro-benzoesäure und 5-Methoxy-1,3-dihydro-isoindol.
Ausbeute: 57 % der Theorie,
Schmelzpunkt: 152°C

| | | | | | | |
|---|---|---|---|---|---|---|
| Ber.: | C | 61,40 | H | 4,48 | N | 8,91 |
| Gef.: | | 61,24 | | 4,32 | | 9,05 |

Beispiel P

(5-Nitro-2-pyrrolidino-phenyl)essigsäure

Hergestellt aus (2-Chlor-5-nitro-phenyl)-essigsäure und Pyrrolidin analog Beispiel G.
Ausbeute: 90 % der Theorie,
Schmelzpunkt: 208°C

| | | | | | | |
|---|---|---|---|---|---|---|
| Ber.: | C | 57,59 | H | 5,64 | N | 11,20 |
| Gef.: | | 57,24 | | 5,50 | | 11,29 |

Beispiel Q

(2-Hexamethylenimino-5-nitro-phenyl)essigsäure

Hergestellt aus (2-Chlor-5-nitro-phenyl)essigsäure und Hexamethylenimin analog Beispiel H.
Ausbeute: 96 % der Theorie,
Schmelzpunkt: 144°C

| | | | | | | |
|---|---|---|---|---|---|---|
| Ber.: | C | 60,42 | H | 6,52 | N | 10,07 |
| Gef.: | | 60,48 | | 6,82 | | 10,17 |

Beispiel R

(2-Heptamethylenimino-5-nitro-phenyl)essigsäure

Hergestellt aus (2-Chlor-5-nitro-phenyl)essigsäure und Heptamethylenimin analog Beispiel H.
Ausbeute: 95 % der Theorie,
Schmelzpunkt: 136°C
Ber.: Molpeak m/e = 292
Gef.: Molpeak m/e = 292

Beispiel S

2,5-Dichlor-nicotinsäure

a) 2,5-Dichlor-nicotinsäurechlorid

Eine Mischung aus 52,1 g (0,3 Mol) 5-Chlor-2-hydroxy-nicotinsäure und 68,7 g (0,33 Mol) Phosphorpentachlorid wird 30 Minuten bei 140°C gerührt. Dann versetzt man mit 500 ml Toluol, saugt vom Ungelösten ab und engt das Filtrat im Vakuum ein. Der Rückstand wird im Hochvakuum destilliert.
Ausbeute: 40,1 g (63,5 % der Theorie),
Siedepunkt: 72-74°C bei 0,05 mbar.

b) 2,5-Dichlor-nicotinsäure

72,2 g (0,343 Mol) 2,5-Dichlor-nicotinsäurechlorid tropft man unter Kühlung und kräftigem Rühren in wässrige Natronlauge (aus 13,7 g (0,343 Mol) Natriumhydroxid und 200 ml Wasser). Nach 1,5 Stunden wird vom Niederschlag abgesaugt und mit Wasser gewaschen. Die erhaltenen Kristalle werden mit Acetonitril verrieben und abgesaugt.
Ausbeute: 62,7 g (95,2 % der Theorie),
Schmelzpunkt: 153-155°C

| | | | | |
|---|---|---|---|---|
| Ber.: | C 37,53 | H 1,57 | N 7,30 | Cl 36,93 |
| Gef.: | 37,76 | 1,67 | 7,31 | 36,30 |

Beispiel T

/2-Piperidino-pyridyl-(3)/acetonitril

a) /2-Chlor-pyridyl-(3)/acetonitril

Eine Lösung von 35,8 g (0,267 Mol) 3-Cyanomethyl-pyridin-N-oxid in 360 ml Phosphoroxychlorid wird unter Rühren langsam auf Rückfluß erhitzt. Nach 2 Stunden wird im Vakuum eingeengt, auf Eiswasser gegeben und mit Natronlauge auf pH = 3 gebracht. Nach Extraktion mit Chloroform wird über Kieselgel im Fließmittel Toluol/Essigsäureäthylester = 10:1 säulenchromatographisch gereinigt.
Ausbeute: 8,5 g (21 % der Theorie),
Schmelzpunkt: 82-84°C

b) /2-Piperidino-pyridyl-(3)/acetonitril

Hergestellt aus /2-Chlor-pyridyl-(3)/acetonitril und Piperidin analog Beispiel H.
Ausbeute: 57 % der Theorie,
Schmelzpunkt: < 20°C

| | | | | | | |
|---|---|---|---|---|---|---|
| Ber.: | C | 71,61 | H | 7,52 | N | 20,88 |
| Gef.: | | 71,69 | | 7,79 | | 20,86 |

Ber.: Molpeak: m/e = 201

Gef.: Molpeak: m/e = 201

Beispiel 1

4-[2-[2-(2-Azabicyclo[3.3.1]nonan-2-yl)-5-chlor-benzoylamino]-äthyl]benzoesäure-äthylester

Zu einer Lösung von 3,7 g (13 mMol) 2-(2-Azabicyclo[3.3.1]nonan-2-yl)-5-chlor-benzoesäure in 20 ml absolutem Pyridin werden 2,3 g (13 mMol) N,N'-Carbonyl-diimidazol zugesetzt. Nach 5-stündigem Erhitzen auf 80°C hat sich das Imidazolid quantitativ gebildet. Anschließend werden 3,1 g (16 mMol) 4-(2-Amino-äthyl)-benzoesäure-äthylester, gelöst in 50 ml Pyridin, zugegeben und 16 Stunden lang unter Rühren auf 90°C erhitzt. Nach Abdestillieren des Pyridins am Rotationsverdampfer wird der Ester über eine Kieselgelsäule mit Toluol/Essigsäureäthylester (9:1) als Laufmittel chromatographisch gereinigt.

Ausbeute: 1,8 g (30 % der Theorie),

Schmelzpunkt: $< 20°C$

| | | | | | | |
|---|---|---|---|---|---|---|
| Ber.: | C | 68,63 | H | 6,87 | N | 6,16 |
| Gef.: | | 68,63 | | 6,83 | | 6,05 |

Beispiel 2

4-[2-[5-Chlor-2-(4-(3-pentyl)-piperidino)-benzoylamino]äthyl]-benzoesäure-äthylester

Hergestellt analog Beispiel 1 aus 5-Chlor-2-(4-(3-pentyl)-piperidino)-benzoesäure und 4-(2-Amino-äthyl)-benzoesäure-äthylester.

Ausbeute: 62 % der Theorie,

Schmelzpunkt: 95-96°C

| | | | | | | |
|---|---|---|---|---|---|---|
| Ber.: | C | 69,33 | H | 7,69 | N | 5,78 |
| Gef.: | | 69,10 | | 7,57 | | 5,52 |

Beispiel 3

4-[2-[5-Chlor-2-(4-(5-nonyl)-piperidino)-benzoylamino]äthyl]-benzoesäure-äthylester

Hergestellt analog Beispiel 1 aus 5-Chlor-2-(4-(5-nonyl)-piperidino)-benzoesäure und 4-(2-Amino-äthyl)-benzoesäure-äthylester.
Ausbeute: 79 % der Theorie,
Schmelzpunkt: 49-50°C

| | | | | | | |
|---|---|---|---|---|---|---|
| Ber.: | C | 71,02 | H | 8,38 | N | 5,18 |
| Gef.: | | 71,00 | | 8,39 | | 5,26 |

Beispiel 4

4-[2-[5-Brom-2-(octahydro-isoindol-2-yl)-benzoylamino]äthyl]-benzoesäure-äthylester

Hergestellt analog Beispiel 1 aus 5-Brom-2-(octahydro-isoindol-2-yl)-benzoesäure und 4-(2-Amino-äthyl)-benzoesäure-äthylester.
Ausbeute: 67,7 % der Theorie,
Schmelzpunkt: 178-179°C

| | | | | | | |
|---|---|---|---|---|---|---|
| Ber.: | C | 62,52 | H | 6,25 | N | 5,60 |
| Gef.: | | 62,55 | | 6,49 | | 5,66 |

Beispiel 5

4-[2-[2-(2-Azabicyclo[3.3.1]nonan-2-yl)-5-chlor-benzoylamino]-äthyl]benzoesäure

1,3 g (2,9 mMol) 4-[2-[2-(2-Azabicyclo[3.3.1]nonan-2-yl)-5-chlor-benzoylamino]äthyl]benzoesäure-äthylester werden in 40 ml einer Mischung aus Methanol/Dioxan (1:1) gelöst. Nach Zugabe von 0,65 g (12 mMol) Kaliumhydroxid, gelöst in 5 ml Wasser, werden bei Raumtemperatur 100 ml Wasser so langsam zugetropft, daß es zu keiner Ausfällung des Esters kommt. Nach einigen Stunden wird das organische Lösungsmittel am Rotationsverdampfer abdestilliert, die wässrige Phase mit Chloroform ausgeschüttelt

und die wässrige Phase mit 2 N Salzsäure auf pH 5,5 eingestellt. Nach Extraktion mit Chloroform, Trocknung über Natriumsulfat, Abdestillieren des Chloroforms und Digerieren mit Petroläther wird die Säure erhalten.

Ausbeute: 1 g (80 % der Theorie),
Schmelzpunkt: 217°C

| | | | | | | |
|---|---|---|---|---|---|---|
| Ber.: | C | 67,51 | H | 6,38 | N | 6,56 |
| Gef.: | | 67,55 | | 6,51 | | 6,57 |

Beispiel 6

4-/2-/5-Chlor-2-(4-(3-pentyl)-piperidino)-benzoylamino/äthyl/-benzoesäure

Hergestellt analog Beispiel 5 durch alkalische Hydrolyse von 4-/2-/5-Chlor-2-(4-(3-pentyl)-piperidino)-benzoylamino/äthyl/-benzoesäure-äthylester.

Ausbeute: 20 % der Theorie,
Schmelzpunkt: 130-132°C

| | | | | | | |
|---|---|---|---|---|---|---|
| Ber.: | C | 68,33 | H | 7,28 | N | 6,13 |
| Gef.: | | 68,40 | | 7,05 | | 6,12 |

Beispiel 7

4-/2-/5-Chlor-2-(4-(5-nonyl)-piperidino)-benzoylamino/äthyl/-benzoesäure

Hergestellt analog Beispiel 5 durch alkalische Hydrolyse von 4-/2-/5-Chlor-2-(4-(5-nonyl)-piperidino)-benzoylamino/äthyl/-benzoesäure-äthylester.

Ausbeute: 68 % der Theorie,
Schmelzpunkt: 129-130°C

| | | | | | | |
|---|---|---|---|---|---|---|
| Ber.: | C | 70,22 | H | 8,05 | N | 5,46 |
| Gef.: | | 70,20 | | 8,07 | | 5,46 |

Beispiel 8

4-/2-/5-Brom-2-(octahydro-isoindol-2-yl)-benzoylamino/äthyl/-benzoesäure

Hergestellt analog Beispiel 5 durch alkalische Hydrolyse von 4-/2-/5-Brom-2-(octahydro-isoindol-2-yl)-benzoylamino/äthyl/-benzoesäure-äthylester.

Ausbeute: 86,3 % der Theorie,
Schmelzpunkt: 235°C

| | | | | | | |
|---|---|---|---|---|---|---|
| Ber.: | C | 61,15 | H | 5,77 | N | 5,94 |
| Gef.: | | 61,24 | | 5,83 | | 5,79 |

Beispiel 9

4-/2-(5-Jod-2-(octahydro-1H-azonino)-benzoylamino)äthyl/benzoesäure-äthylester

2,9 g (7,8 mMol) 5-Jod-2-(octahydro-1H-azonino)-benzoesäure werden in 20 ml absolutem Pyridin gelöst und bei 50°C mit 1,5 g (8,5 mMol) N,N'-Carbonyl-diimidazol innerhalb von 3 Stunden quantitativ in das Imidazolid überführt. Nach Zusatz von 1,75 g (9 mMol) 4-(2-Amino-äthyl)-benzoesäure-äthylester wird das Reaktionsgemisch für 8 Stunden auf 90°C erwärmt und das Pyridin anschließend am Rotationsverdampfer abdestilliert. Der Ester wird über eine Kieselgelsäule mit Toluol/Essigsäureäthylester (9:1) als Fließmittel chromatographisch gereinigt.

Ausbeute: 1,3 g (30 % der Theorie),
Schmelzpunkt: < 20°C

| | | | | | | |
|---|---|---|---|---|---|---|
| Ber.: | C | 56,93 | H | 6,06 | N | 5,11 |
| Gef.: | | 57,10 | | 6,22 | | 5,22 |

Beispiel 10

4-/2-(5-Fluor-2-(octahydro-1H-azonino)-benzoylamino)äthyl/benzoesäure-äthylester

Hergestellt analog Beispiel 9 aus 5-Fluor-2-(octahydro-1H-azonino)-benzoesäure und 4-(2-Amino-äthyl)-benzoesäure-äthylester.
Ausbeute: 60 % der Theorie,
Schmelzpunkt: < 20°C

| | | | | | | |
|---|---|---|---|---|---|---|
| Ber.: | C | 70,88 | H | 7,55 | N | 6,36 |
| Gef.: | | 71,01 | | 7,68 | | 6,25 |

Beispiel 11

4-/2-(5-Methyl-2-(octahydro-1H-azonino)-benzoylamino)äthyl/-benzoesäure-äthylester

Hergestellt analog Beispiel 9 aus 5-Methyl-2-(octahydro-1H-azonino)-benzoesäure und 4-(2-Amino-äthyl)-benzoesäure-äthylester.
Ausbeute: 90 % der Theorie,
Schmelzpunkt: < 20°C

| | | | | | | |
|---|---|---|---|---|---|---|
| Ber.: | C | 74,28 | H | 8,31 | N | 6,42 |
| Gef.: | | 74,25 | | 8,33 | | 6,33 |

Beispiel 12

4-/2-/5-(2-Tolyl)-2-(octahydro-1H-azonino)-benzoylamino/äthyl/-benzoesäure-äthylester

Hergestellt analog Beispiel 9 aus 5-(2-Tolyl)-2-(octahydro-1H-azonino)-benzoesäure und 4-(2-Amino-äthyl)-benzoesäure-äthylester.
Ausbeute: 11 % der Theorie,
Schmelzpunkt: < 20°C

| | | | | | | |
|---|---|---|---|---|---|---|
| Ber.: | C | 77,31 | H | 7,86 | N | 5,47 |
| Gef.: | | 77,25 | | 8,16 | | 5,17 |

Beispiel 13

4-/2-/5-(4-Chlorphenyl)-2-piperidino-benzoylamino/-äthyl/benzoesäure-äthylester

Hergestellt analog Beispiel 9 aus 5-(4-Chlorphenyl)-2-piperidino-benzoesäure und 4-(2-Amino-äthyl)-benzoesäure-äthylester.
Ausbeute: 24 % der Theorie,
Schmelzpunkt: 129-130°C

| | | | | | | | | |
|---|---|---|---|---|---|---|---|---|
| Ber.: | C | 70,93 | H | 6,36 | N | 5,71 | Cl | 7,22 |
| Gef.: | | 71,28 | | 6,28 | | 5,66 | | 7,42 |

Beispiel 14

4-/2-/5-Trifluormethyl-2-(octahydro-1H-azonino)-benzoylamino/-äthyl/benzoesäure-äthylester

Hergestellt analog Beispiel 9 aus 5-Trifluormethyl-2-(octahydro-1H-azonino)-benzoesäure und 4-(2-Amino-äthyl)-benzoesäure-äthylester.
Ausbeute: 80 % der Theorie,
Schmelzpunkt: < 20°C

| | | | | | | |
|---|---|---|---|---|---|---|
| Ber.: | C | 66,10 | H | 6,78 | N | 5,71 |
| Gef.: | | 65,80 | | 6,88 | | 5,62 |

Analog den vorstehenden Beispielen 9-14 wurden folgende Verbindungen hergestellt:

4-/2-(5-Isopropyl-2-(octahydro-1H-azonino)-benzoylamino)äthyl/-benzoesäure-äthylester

4-/2-/5-tert.Butyl-2-(octahydro-1H-azonino)-benzoylamino/äthyl/-benzoesäure-äthylester

4-/2-/5-Butyl-2-(octahydro-1H-azonino)-benzoylamino/äthyl/-benzoesäure-äthylester

4-/2-/5-Fluor-2-(decahydro-azecino)-benzoylamino/äthyl/benzoesäure-äthylester

4-/2-/5-Brom-2-(decahydro-azecino)-benzoylamino/äthyl/benzoesäure-äthylester

4-/2-/5-Jod-2-(decahydro-azecino)-benzoylamino/äthyl/benzoesäure-äthylester

4-/2-/5-Cyano-2-(decahydro-azecino)-benzoylamino/äthyl/benzoesäure-äthylester

4-/2-/5-Methyl-2-(decahydro-azecino)-benzoylamino/äthyl/benzoesäure-äthylester

4-/2-/5-Methoxy-2-(decahydro-azecino)-benzoylamino/äthyl/benzoesäure-äthylester

Beispiel 15

4-/2-/5-Jod-2-(octahydro-1H-azonino)-benzoylamino/äthyl/benzoesäure

1 g (1,8 mMol) 4-/2-/5-Jod-2-(octahydro-1H-azonino)-benzoylamino/äthyl/benzoesäure-äthylester werden in 20 ml Dioxan/Methanol (1:1) bei Raumtemperatur mit 0,4 g (7,2 mMol) Kalilauge, gelöst in 50 ml Wasser, verseift. Nach Abdestillieren der organischen Lösungsmittel wird mit Chloroform ausgeschüttelt, die wässrige Phase mit 2 N Salzsäure auf pH 4,5 gestellt und mit Chloroform ausgeschüttelt. Der Trockenrückstand der Chloroformextrakte wird mit Petroläther verrieben, wobei das Produkt auskristallisiert.

Ausbeute: 0,45 g (48 % der Theorie),
Schmelzpunkt: sintern ab 73°C

| | | | | | | |
|---|---|---|---|---|---|---|
| Ber.: | C | 55,39 | H | 5,62 | N | 5,38 |
| Gef.: | | 55,30 | | 5,61 | | 5,26 |

Beispiel 16

4-/2-/5-Fluor-2-(octahydro-1H-azonino)-benzoylamino/äthyl/-benzoesäure

Hergestellt analog Beispiel 15 durch alkalische Hydrolyse von 4-/2-(5-Fluor-2-(octahydro-1H-azonino)-benzoylamino)äthyl/-benzoesäure-äthylester.
Ausbeute: 85 % der Theorie,
Schmelzpunkt: 166-168°C

| | | | | | | |
|---|---|---|---|---|---|---|
| Ber.: | C | 69,88 | H | 7,09 | N | 6,79 |
| Gef.: | | 69,49 | | 7,09 | | 6,64 |

Beispiel 17

4-/2-/5-Methyl-2-(octahydro-1H-azonino)-benzoylamino/äthyl/-benzoesäure

Hergestellt analog Beispiel 15 durch alkalische Hydroylse von 4-/2-(5-Methyl-2-(octahydro-1H-azonino)-benzoylamino)äthyl/-benzoesäure-äthylester.
Ausbeute: 81 % der Theorie,
Schmelzpunkt: 145-147°C

| | | | | | | |
|---|---|---|---|---|---|---|
| Ber.: | C | 73,50 | H | 7,89 | N | 6,86 |
| Gef.: | | 73,22 | | 8,19 | | 6,59 |

Beispiel 18

4-/2-/5-(4-Chlorphenyl)-2-piperidino-benzoylamino/äthyl/benzoesäure

Hergestellt analog Beispiel 15 durch alkalische Hydrolyse von 4-/2-/5-(4-Chlorphenyl)-2-piperidino-benzoylamino/äthyl/benzoesäure-äthylester.

Ausbeute: 73 % der Theorie,

Schmelzpunkt: 249-252°C

| | | | | | | | | |
|---|---|---|---|---|---|---|---|---|
| Ber.: | C | 70,04 | H | 5,88 | Cl | 7,66 | N | 6,05 |
| Gef.: | | 69,89 | | 5,43 | | 7,53 | | 5,98 |

Beispiel 19

4-/2-/5-Trifluormethyl-2-(octahydro-1H-azonino)-benzoylamino/-äthyl/benzoesäure

Hergestellt analog Beispiel 15 durch alkalische Hydrolyse von 4-/2-/5-Trifluormethyl-2-(octahydro-1H-azonino)-benzoylamino/-äthyl/benzoesäure-äthylester.

Ausbeute: 48 % der Theorie,

Schmelzpunkt: 149-150°C

| | | | | | | |
|---|---|---|---|---|---|---|
| Ber.: | C | 64,92 | H | 6,32 | N | 6,06 |
| Gef.: | | 65,20 | | 6,34 | | 6,00 |

Analog den Beispielen 15-19 wurden folgende Verbindungen hergestellt:

4-/2-/5-Isopropyl-2-(octahydro-1H-azonino)-benzoylamino/äthyl/-benzoesäure

4-/2-/5-tert.Butyl-2-(octahydro-1H-azonino)-benzoylamino/äthyl/-benzoesäure

4-[2-[5-Butyl-2-(octahydro-1H-azonino)-benzoylamino]äthyl]-benzoesäure

4-[2-[5-(2-Tolyl)-2-piperidino-benzoylamino]äthyl]benzoesäure

4-[2-[5-Fluor-2-(decahydro-azecino)-benzoylamino]äthyl]benzoesäure

4-[2-[5-Brom-2-(decahydro-azecino)-benzoylamino]äthyl]benzoesäure

4-[2-[5-Jod-2-(decahydro-azecino)-benzoylamino]äthyl]benzoesäure

4-[2-[5-Cyano-2-(decahydro-azecino)-benzoylamino]äthyl]benzoesäure

4-[2-[5-Methyl-2-(decahydro-azecino)-benzoylamino]äthyl]benzoesäure

4-[2-[5-Methoxy-2-(decahydro-azecino)-benzoylamino]äthyl]benzoesäure

## Beispiel 20

**4-[2-(5-Chlor-6-methyl-2-piperidino-nicotinoylamino)-äthyl]-benzoesäure-methylester**

2,5 g (13,7 mMol) 5-Chlor-6-methyl-2-piperidino-nicotinsäure, 3,0 g (13,7 mMol) 4-(2-Amino-äthyl)-benzoesäure-methylester-hydrochlorid und 5,0 g (19,25 mMol) Triphenylphosphin werden in 150 ml absolutem Acetonitril gerührt und nacheinander mit 1,4 ml (14 mMol) Tetrachlorkohlenstoff und 8,3 ml (60 mMol) Triäthylamin versetzt. Die so erhaltene Suspension wird 2 Tage bei Raumtemperatur gerührt, dann eingeengt, mit Wasser versetzt und mit Chloroform mehrmals extrahiert. Die Chloroformextrakte

werden über Natriumsulfat getrocknet und eingeengt und über Kieselgel mit Toluol/Essigsäureäthylester (2:1) als Fließmittel säulenchromatographisch gereinigt.
Ausbeute: 3,35 g (58,8 % der Theorie),
Schmelzpunkt: 88-90°C

| | | | | | | | | |
|---|---|---|---|---|---|---|---|---|
| Ber.: | C | 63,53 | H | 6,30 | Cl | 8,52 | N | 10,10 |
| Gef.: | | 63,60 | | 6,42 | | 8,57 | | 10,21 |

Beispiel 21

4-/2-(6-Methyl-2-piperidino-nicotinoylamino)äthyl/benzoesäure-methylester

Hergestellt analog Beispiel 20 aus 6-Methyl-2-piperidino-nicotinsäure und 4-(2-Amino-äthyl)-benzoesäure-methylester-hydrochlorid.
Ausbeute: 72,4 % der Theorie,
Schmelzpunkt: 92-94°C

| | | | | | | |
|---|---|---|---|---|---|---|
| Ber.: | C | 69,27 | H | 7,13 | N | 11,01 |
| Gef.: | | 69,50 | | 7,20 | | 11,22 |

Beispiel 22

4-/2-(5-Chlor-2-(octahydro-1H-azonino)-nicotinoylamino)äthyl/-benzoesäure-äthylester

Hergestellt analog Beispiel 20 aus 5-Chlor-2-(octahydro-1H-azonino)-nicotinsäure und 4-(2-Amino-äthyl)-benzoesäure-äthylester-hydrochlorid.
Ausbeute: 31 % der Theorie,
Schmelzpunkt: $<$ 20°C

| | | | | | | | | |
|---|---|---|---|---|---|---|---|---|
| Ber.: | C | 65,56 | H | 7,04 | Cl | 7,74 | N | 9,18 |
| Gef.: | | 65,40 | | 6,99 | | 7,53 | | 9,02 |

Beispiel 23

4-[2-(5-Brom-2-piperidino-nicotinoylamino)äthyl]benzoesäure-äthylester

Hergestellt analog Beispiel 20 aus 5-Brom-2-piperidino-nicotinsäure und 4-(2-Amino-äthyl)-benzoesäure-äthylester-hydrochlorid.
Ausbeute: 41,8 % der Theorie,
Schmelzpunkt: 93-95°C

| | | | | | | | | |
|---|---|---|---|---|---|---|---|---|
| Ber.: | C | 57,40 | H | 5,69 | Br | 17,36 | N | 9,13 |
| Gef.: | | 57,62 | | 5,53 | | 17,25 | | 9,01 |

Beispiel 24

4-[2-[5-Chlor-2-(cis-3,5-dimethyl-piperidino)-nicotinoylamino]-äthyl]benzoesäure-äthylester

Hergestellt analog Beispiel 20 aus 5-Chlor-2-(cis-3,5-dimethyl-piperidino)-nicotinsäure und 4-(2-Amino-äthyl)-benzoesäure-äthylester-hydrochlorid.
Ausbeute: 42,4 % der Theorie,
Schmelzpunkt: 94-96°C

| | | | | | | | | |
|---|---|---|---|---|---|---|---|---|
| Ber.: | C | 64,92 | H | 6,81 | Cl | 7,99 | N | 9,47 |
| Gef.: | | 65,12 | | 6,92 | | 7,73 | | 9,12 |

Beispiel 25

4-[2-(5-Chlor-2-piperidino-nicotinoylamino)äthyl]benzoesäure-methylester

Hergestellt analog Beispiel 20 aus 5-Chlor-2-piperidino-nicotinsäure und 4-(2-Amino-äthyl)-benzoesäure-methylester-hydrochlorid.
Ausbeute: 49 % der Theorie,
Schmelzpunkt: 113-115°C

| | | | | | | | | |
|---|---|---|---|---|---|---|---|---|
| Ber.: | C | 62,76 | H | 6,02 | Cl | 8,82 | N | 10,46 |
| Gef.: | | 62,95 | | 6,13 | | 8,54 | | 10,23 |

Beispiel 26

4-/2-(5-Brom-6-methyl-2-piperidino-nicotinoylamino)äthyl/benzoesäure-äthylester

Hergestellt analog Beispiel 20 aus 4-(2-Amino-äthyl)-benzoesäure-äthylester-hydrochlorid und 5-Brom-6-methyl-2-piperidino-nicotinsäure.

Ausbeute: 54,6 % der Theorie,

Schmelzpunkt: < 20°C

| | | | | | | | | |
|---|---|---|---|---|---|---|---|---|
| Ber.: | C | 58,23 | H | 5,95 | Br | 16,84 | N | 8,86 |
| Gef.: | | 58,51 | | 6,01 | | 16,73 | | 8,53 |

Beispiel 27

4-/2-/5-Chlor-2-(3,5-cis-dimethyl-piperidino)-6-methyl-nicotinoylamino/äthyl/benzoesäure-äthylester

Hergestellt analog Beispiel 20 aus 5-Chlor-2-(3,5-cis-dimethyl-piperidino)-6-methyl-nicotinsäure und 4-(2-Amino-äthyl)-benzoesäure-äthylester-hydrochlorid.

Ausbeute: 51 % der Theorie,

Schmelzpunkt: < 20°C

| | | | | | | | | |
|---|---|---|---|---|---|---|---|---|
| Ber.: | C | 65,56 | H | 7,04 | Cl | 7,74 | N | 9,17 |
| Gef.: | | 65,73 | | 7,21 | | 7,51 | | 9,02 |

Beispiel 28

4-/2-/5-Brom-2-(3,5-cis-dimethyl-piperidino)-6-methyl-nicotinoylamino/äthyl/benzoesäure-äthylester

Hergestellt analog Beispiel 20 aus 5-Brom-2-(3,5-cis-dimethyl-piperidino)-6-methyl-nicotinsäure und 4-(2-Amino-äthyl)-benzoesäure-äthylester-hydrochlorid.

Ausbeute: 43 % der Theorie,
Schmelzpunkt: < 20°C

| | | | | | | | | |
|---|---|---|---|---|---|---|---|---|
| Ber.: | C | 59,76 | H | 6,42 | Br | 15,90 | N | 8,37 |
| Gef.: | | 59,91 | | 6,62 | | 15,75 | | 8,21 |

Beispiel 29

4-/2-(5-Chlor-6-methyl-2-piperidino-nicotinoylamino)äthyl/-benzoesäure

1,65 g (3,97 mMol) 4-/2-(5-Chlor-6-methyl-2-piperidino-nicotinoylamino)äthyl/benzoesäure-methylester werden in 100 ml Methanol gelöst und nach Zugabe von 25 ml 1 N Natronlauge 12 Stunden bei 40°C gerührt. Anschließend wird im Vakuum eingeengt. Der Einengungsrückstand wird in Wasser gelöst, filtriert, und das Filtrat mit 25 ml 1 N Salzsäure versetzt. Der gebildete Niederschlag wird abgesaugt und aus Acetonitril umkristallisiert.
Ausbeute: 1,1 g (58,75 % der Theorie),
Schmelzpunkt: 185-187°C

| | | | | | | | | |
|---|---|---|---|---|---|---|---|---|
| Ber.: | C | 62,76 | H | 6,02 | Cl | 8,82 | N | 10,46 |
| Gef.: | | 62,57 | | 5,80 | | 8,81 | | 10,59 |

Beispiel 30

4-/2-(6-Methyl-2-piperidino-nicotinoylamino)äthyl/benzoesäure

Hergestellt analog Beispiel 29 durch alkalische Verseifung von 4-/2-(6-Methyl-2-piperidino-nicotinoylamino)äthyl/benzoesäure-methylester.
Ausbeute: 92 % der Theorie,
Schmelzpunkt: 152-154°C

| | | | | | | |
|---|---|---|---|---|---|---|
| Ber.: | C | 68,64 | H | 6,86 | N | 11,44 |
| Gef.: | | 68,47 | | 6,80 | | 11,57 |

Beispiel 31

4-/2-(5-Chlor-2-(octahydro-1H-azonino)-nicotinoylamino)äthyl/-benzoesäure

Hergestellt analog Beispiel 29 aus 4-/2-(5-Chlor-2-(octahydro-1H-azonino)-nicotinoylamino)äthyl/benzoesäure-äthylester durch alkalische Verseifung.
Ausbeute: 83,3 % der Theorie.
Schmelzpunkt: 169-170°C

| | | | | | | | | |
|---|---|---|---|---|---|---|---|---|
| Ber.: | C | 64,25 | H | 6,56 | Cl | 8,24 | N | 9,77 |
| Gef.: | | 64,45 | | 6,69 | | 8,13 | | 9,66 |

Beispiel 32

4-/2-(5-Brom-2-piperidino-nicotinoylamino)äthyl/benzoesäure

Hergestellt analog Beispiel 29 aus 4-/2-(5-Brom-2-piperidino-nicotinoylamino)äthyl/benzoesäure-äthylester durch alkalische Verseifung.
Ausbeute: 93,5 % der Theorie,
Schmelzpunkt: 175-177°C

| | | | | | | | | |
|---|---|---|---|---|---|---|---|---|
| Ber.: | C | 55,56 | H | 5,13 | Br | 18,48 | N | 9,72 |
| Gef.: | | 55,30 | | 5,16 | | 18,60 | | 9,59 |

Beispiel 33

4-/2-/5-Chlor-2-(cis-3,5-dimethyl-piperidino)-nicotinoylamino/-äthyl/benzoesäure

Hergestellt analog Beispiel 29 durch alkalische Verseifung von 4-/2-/5-Chlor-2-(cis-3,5-dimethyl-piperidino)-nicotinoylamino/-äthyl/benzoesäure-äthylester.
Ausbeute: 76 % der Theorie,
Schmelzpunkt: 191-193°C

| | | | | | | | | |
|---|---|---|---|---|---|---|---|---|
| Ber.: | C | 63,53 | H | 6,30 | Cl | 8,52 | N | 10,10 |
| Gef.: | | 63,28 | | 6,27 | | 8,57 | | 10,07 |

Beispiel 34

4-/2-(5-Chlor-2-piperidino-nicotinoylamio)äthyl/benzoesäure

Hergestellt analog Beispiel 29 durch alkalische Verseifung von 4-/2-(5-Chlor-2-piperidino-nicotinoylamino)äthyl/benzoesäure-methylester.
Ausbeute: 82 % der Theorie,
Schmelzpunkt: 164-166°C

| | | | | | | | | |
|---|---|---|---|---|---|---|---|---|
| Ber.: | C | 61,93 | H | 5,72 | Cl | 9,14 | N | 10,83 |
| Gef.: | | 62,00 | | 5,92 | | 9,02 | | 10,99 |

Beispiel 35

4-/2-(5-Brom-6-methyl-2-piperidino-nicotinoylamino)äthyl/benzoesäure

Hergestellt analog Beispiel 29 aus 4-/2-(5-Brom-6-methyl-2-piperidino-nicotinoylamino)äthyl/benzoesäure-äthylester durch alkalische Verseifung.
Ausbeute: 85,5 % der Theorie,
Schmelzpunkt: 199-201°C

| | | | | | | | | |
|---|---|---|---|---|---|---|---|---|
| Ber.: | C | 56,51 | H | 5,42 | Br | 17,90 | N | 9,41 |
| Gef.: | | 56,78 | | 5,46 | | 17,80 | | 9,46 |

Beispiel 36

4-/2-/5-Chlor-2-(3,5-cis-dimethyl-piperidino)-6-methyl-nicotinoylamino/äthyl/benzoesäure

Hergestellt analog Beispiel 29 aus 4-/2-/5-Chlor-2-(3,5-cis-dimethyl-piperidino)-6-methyl-nicotinoylamino/äthyl/benzoesäure-äthylester durch alkalische Verseifung.
Ausbeute: 71 % der Theorie,
Schmelzpunkt: 192-193°C

| | | | | | | | | |
|---|---|---|---|---|---|---|---|---|
| Ber.: | C | 64,25 | H | 6,56 | Cl | 8,24 | N | 9,77 |
| Gef.: | | 64,60 | | 6,47 | | 8,31 | | 9,86 |

Beispiel 37

4-[2-[5-Brom-2-(3,5-cis-dimethyl-piperidino)-6-methyl-nicotinoylamino]äthyl]benzoesäure

Hergestellt analog Beispiel 29 aus 4-[2-[5-Brom-2-(3,5-cis-dimethyl-piperidino)-6-methyl-nicotinoylamino]äthyl]benzoesäure-äthylester durch alkalische Verseifung.

Ausbeute: 99 % der Theorie,

Schmelzpunkt: 203-204°C

| | | | | | | | | |
|---|---|---|---|---|---|---|---|---|
| Ber.: | C | 58,23 | H | 5,95 | Br | 16,84 | N | 8,86 |
| Gef.: | | 58,30 | | 5,98 | | 16,95 | | 9,20 |

Beispiel 38

4-[2-[(2-Piperidino-pyridyl-3)acetylamino]äthyl]benzoesäure-äthylester

Hergestellt aus [2-Piperidino-pyridyl-(3)]-essigsäure und 4-(2-Amino-äthyl)benzoesäure-äthylester analog Beispiel 20.

Ausbeute: 84 % der Theorie,

Schmelzpunkt: 103-104°C

| | | | | | | |
|---|---|---|---|---|---|---|
| Ber.: | C | 69,85 | H | 7,39 | N | 10,63 |
| Gef.: | | 69,82 | | 7,18 | | 10,89 |

Beispiel 39

4-[(2-Piperidino-pyridyl-3)acetylamino-methyl]benzoesäure-äthylester

Hergestellt aus [2-Piperidino-pyridyl-(3)]-essigsäure und 4-Amino-methyl-benzoesäure-äthylester analog Beispiel 20.

Ausbeute: 86 % der Theorie,

Schmelzpunkt: 68-71°C

| | | | | | | |
|---|---|---|---|---|---|---|
| Ber.: | C | 69,27 | H | 7,14 | N | 11,02 |
| Gef.: | | 69,65 | | 6,86 | | 11,32 |

Beispiel 40

4-/2-/(5-Chlor-2-piperidino-pyridyl-3)acetylamino/äthyl/benzoesäure-äthylester

Hergestellt aus /5-Chlor-2-piperidino-pyridyl-(3)/-essigsäure und 4-(2-Aminoäthyl)-benzoesäure-äthylester analog Beispiel 20.
Ausbeute: 86 % der Theorie,
Schmelzpunkt: 115-117°C

| | | | | | | | | |
|---|---|---|---|---|---|---|---|---|
| Ber.: | C | 64,25 | H | 6,56 | N | 9,77 | Cl | 8,25 |
| Gef.: | | 64,48 | | 6,74 | | 9,88 | | 8,12 |

Beispiel 41

4-/2-/(2-Piperidino-pyridyl-3)acetylamino/äthyl/benzoesäure

Hergestellt aus 4-/2-/(2-Piperidino-pyridyl-3)acetylamino/-äthyl/benzoesäure-äthylester durch alkalische Verseifung analog Beispiel 29.
Ausbeute: 60 % der Theorie,
Schmelzpunkt: 153-155°C

| | | | | | | |
|---|---|---|---|---|---|---|
| Ber.: | C | 68,64 | H | 6,86 | N | 11,44 |
| Gef.: | | 68,85 | | 6,71 | | 11,57 |

Beispiel 42

4-/(2-Piperidino-pyridyl-3)acetylaminomethyl/benzoesäure

Hergestellt durch alkalische Verseifung von 4-/(2-Piperidino-pyridyl-3)acetylaminomethyl/benzoesäure-äthylester analog Beispiel 29.
Ausbeute: 74 % der Theorie,
Schmelzpunkt: 180-182°C

| | | | | | | |
|---|---|---|---|---|---|---|
| Ber.: | C | 67,97 | H | 6,56 | N | 11,89 |
| Gef.: | | 68,20 | | 6,60 | | 12,15 |

## Beispiel 43

**4-[2-[(5-Chlor-2-piperidino-pyridyl-3)acetylamino]äthyl]benzoesäure**

Hergestellt durch alkalische Verseifung von 4-[2-[(5-Chlor-2-piperidino-pyridyl-3)acetylamino]äthyl]benzoesäure-äthylester analog Beispiel 29.

Ausbeute: 85 % der Theorie,

Schmelzpunkt: 182-184°C

| | | | | | | | | |
|---|---|---|---|---|---|---|---|---|
| Ber.: | C | 62,76 | H | 6,02 | N | 10,46 | Cl | 8,82 |
| Gef.: | | 62,56 | | 5,88 | | 10,21 | | 8,75 |

## Beispiel 44

**4-[(5-Chlor-2-piperidino-phenyl)-acetylamino-methyl]benzoesäure-äthylester**

Hergestellt analog Beispiel 1 aus (5-Chlor-2-piperidino-phenyl)-essigsäure und 4-Aminomethyl-benzoesäure-äthylester.

Ausbeute: 64 % der Theorie,

Schmelzpunkt: 112°C

| | | | | | | |
|---|---|---|---|---|---|---|
| Ber.: | C | 67,20 | H | 6,81 | N | 6,53 |
| Gef.: | | 67,40 | | 6,90 | | 6,50 |

## Beispiel 45

**4-[2-[(5-Chlor-2-piperidino-phenyl)-acetylamino]-äthyl]benzoesäure-äthylester**

Hergestellt analog Beispiel 1 aus (5-Chlor-2-piperidino-phenyl)-essigsäure und 4-(2-Amino-äthyl)-benzoesäure-äthylester.

Ausbeute: 74 % der Theorie,

Schmelzpunkt: 187-189°C

| | | | | | | |
|---|---|---|---|---|---|---|
| Ber.: | C | 69,53 | H | 8,75 | N | 10,14 |
| Gef.: | | 69,45 | | 8,74 | | 10,40 |

Beispiel 46

4-/(5-Chlor-2-octahydro-1H-azonino-phenyl)-acetylaminomethyl/-benzoesäure-äthylester

Hergestellt analog Beispiel 1 aus (5-Chlor-2-octahydro-1H-azonino-phenyl)-essigsäure und 4-Amino-methyl-benzoesäure-äthylester.
Ausbeute: 85 % der Theorie,
Schmelzpunkt: < 20°C

| | | | | | | | |
|---|---|---|---|---|---|---|---|
| Ber.: | C | 68,33 | H | 7,28 | N | 6,13 | m/e = 456/8 |
| Gef.: | | 68,15 | | 7,04 | | 5,99 | m/e = 456/8 |

Beispiel 47

4-/2-/(5-Chlor-2-octahydro-1H-azonino-phenyl)-acetylamino/äthyl/-benzoesäure-äthylester

Hergestellt analog Beispiel 1 aus (5-Chlor-2-octahydro-1H-azonino-phenyl)-essigsäure und 4-(2-Amino-äthyl)-benzoesäure-äthylester.
Ausbeute: 59 % der Theorie,
Schmelzpunkt: < 20°C
Ber.: Molpeak: m/e = 470/2
Gef.: Molpeak: m/e = 470/2

| | | | | | | |
|---|---|---|---|---|---|---|
| Ber.: | C | 68,84 | H | 7,49 | N | 5,95 |
| Gef.: | | 68,17 | | 7,14 | | 5,75 |

Beispiel 48

4-/(5-Chlor-2-(octahydro-isoindol-2-yl)-phenyl)-acetylamino-methyl/benzoesäure-äthylester

Hergestellt analog Beispiel 1 aus /5-Chlor-2-(octahydro-isoindol-2-yl)-phenyl/-essigsäure und 4-Amino-methyl-benzoesäure-äthylester.

Ausbeute: 45 % der Theorie,
Schmelzpunkt: 172°C

| | | | | | | |
|---|---|---|---|---|---|---|
| Ber.: | C | 68,63 | H | 6,86 | N | 6,15 |
| Gef.: | | 68,63 | | 6,84 | | 5,97 |

Beispiel 49

4-/2-/(5-Chlor-2-(octahydro-isoindol-2-yl)-phenyl)-acetylamino7-äthyl7benzoesäure-äthylester

Hergestellt analog Beispiel 1 aus /5-Chlor-2-(octahydro-isoindol-2-yl)-phenyl7-essigsäure und 4-(2-Aminoäthyl)benzoesäure-äthylester.
Ausbeute: 12,5 % deßTheorie,
Schmelzpunkt: 102°C
Ber.: Molpeak: m/e = 468/2
Gef.: Molpeak: m/e = 468/2

Beispiel 50

DL-4-/(2-(5-Chlor-2-piperidino-phenyl)propionylamino)methyl7-benzoesäure-äthylester

Hergestellt analog Beispiel 20 aus 2-(5-Chlor-2-piperidino-phenyl)propionsäure und 4-Aminomethyl-benzoesäure-äthylester.
Ausbeute: 46 % der Theorie,
Schmelzpunkt: < 20°C
Ber.: Molpeak: m/e = 428/30
Gef.: Molpeak: m/e = 428/30

| | | | | | | | | |
|---|---|---|---|---|---|---|---|---|
| Ber.: | C | 67,20 | H | 6,81 | N | 6,53 | Cl | 8,26 |
| Gef.: | | 67,30 | | 6,92 | | 6,60 | | 8,67 |

Beispiel 51

DL-4-/2-/2-(5-Chlor-2-piperidino-phenyl)propionylamino/äthyl/-benzoesäure-äthylester

Hergestellt analog Beispiel 20 aus 2-(5-Chlor-2-piperidino-phenyl)propionsäure und 4-(2-Amino-äthyl)-benzoesäure-äthylester.
Ausbeute: 52 % der Theorie,
Schmelzpunkt: < 20°C
Ber.: Molpeak: m/e = 442/44
Gef.: Molpeak: m/e = 442/44

| | | | | | | |
|---|---|---|---|---|---|---|
| Ber.: | C | 67,78 | H | 7,05 | N | 6,32 |
| Gef.: | | 67,82 | | 7,15 | | 6,46 |

Beispiel 52

DL-4-/2-/2-(5-Chlor-2-octahydro-1H-azonino-phenyl)-propionylamino/äthyl/benzoesäure-äthylester

Hergestellt analog Beispiel 1 aus 2-(2-Octahydro-1H-azonino-5-chlor-phenyl)propionsäure und 4-(2-Aminoäthyl)benzoesäure-äthylester.
Ausbeute: 43 % der Theorie,
Schmelzpunkt: < 20°C

| | | | | | | | |
|---|---|---|---|---|---|---|---|
| Ber.: | C | 69,33 | H | 7,68 | N | 5,77 | m/e = 484/86 |
| Gef.: | | 69,53 | | 7,77 | | 5,82 | m/e = 484/86 |

Beispiel 53

DL-4-/2-/(2-Piperidino-diphenyl)-acetylamino/äthyl/benzoesäure-äthylester

Hergestellt aus (2-Piperidino-diphenyl)-essigsäure und 4-(2-Aminoäthyl)benzoesäure-äthylester analog Beispiel 20.

Ausbeute: 36 % der Theorie,
Schmelzpunkt: 84-85°C

| | | | | | | |
|---|---|---|---|---|---|---|
| Ber.: | C | 76,56 | H | 7,28 | N | 5,95 |
| Gef.: | | 76,57 | | 7,38 | | 6,01 |

**Beispiel 54**

**DL-4-/(2-Piperidino-diphenyl)-acetylamino-methyl/benzoesäure-äthylester**

Hergestellt aus (2-Piperidino-diphenyl)-essigsäure und 4-Amino-methyl-benzoesäure-äthylester analog Beispiel 20.
Ausbeute: 43 % der Theorie,
Schmelzpunkt: 137-139°C

| | | | | | | |
|---|---|---|---|---|---|---|
| Ber.: | C | 76,28 | H | 7,06 | N | 6,14 |
| Gef.: | | 76,50 | | 7,02 | | 5,97 |

**Beispiel 55**

**4-/2-/(5-Chlor-2-pyrrolidino-phenyl)-acetylamino/-äthyl/benzoesäure-äthylester**

Hergestellt analog Beispiel 1 aus (5-Chlor-2-pyrrolidino-phenyl)-essigsäure und 4-(2-Aminoäthyl)benzoesäure-äthylester.
Ausbeute: 75 % der Theorie,
Schmelzpunkt: 126-127°C

| | | | | | | |
|---|---|---|---|---|---|---|
| Ber.: | C | 66,57 | H | 6,56 | N | 6,75 |
| Gef.: | | 66,77 | | 6,60 | | 6,73 |

**Beispiel 56**

**4-/2-/(5-Chlor-2-hexamethylenimino-phenyl)-acetylamino/äthyl/-benzoesäure-äthylester**

Hergestellt analog Beispiel 1 aus (5-Chlor-2-hexamethylenimino-phenyl)-essigsäure und 4-(2-Amino-äthyl)benzoesäure-äthylester.

Ausbeute: 51 % der Theorie,
Schmelzpunkt: 102-104°C

| | | | | | | |
|---|---|---|---|---|---|---|
| Ber.: | C | 67,78 | H | 7,05 | N | 6,33 |
| Gef.: | | 67,90 | | 7,15 | | 6,35 |

Beispiel 57

4-/2-/(5-Chlor-2-heptamethylenimino-phenyl)acetylamino/äthyl/-benzoesäure-äthylester

Hergestellt analog Beispiel 1 aus (5-Chlor-2-heptamethylenimino-phenyl)-essigsäure und 4-(2-Amino-äthyl)benzoesäure-äthylester.
Ausbeute: 57 % der Theorie,
Schmelzpunkt: 95-96°C

| | | | | | | |
|---|---|---|---|---|---|---|
| Ber.: | C | 68,33 | H | 7,27 | N | 6,12 |
| Gef.: | | 68,50 | | 7,28 | | 6,09 |

Beispiel 58

4-/2-(2-Octahydro-1H-azonino-benzoylamino)äthyl/benzoesäure-äthylester

Hergestellt analog Beispiel 1 aus 2-Octahydro-1H-azonino-benzoesäure und 4-(2-Amino-äthyl)benzoesäure-äthylester.
Ausbeute: 53 % der Theorie,
Schmelzpunkt: < 20°C
Ber.: Molpeak: m/e = 422
Gef.: Molpeak: m/e = 422

| | | | | | | |
|---|---|---|---|---|---|---|
| Ber.: | C | 73,90 | H | 8,11 | N | 6,62 |
| Gef.: | | 73,80 | | 8,05 | | 6,52 |

Beispiel 59

4-/2-(5-Chlor-2-(2,3,3a,4,7,7a-hexahydro-1H-isoindol-2-yl)-benzoylamino)äthyl/benzoesäure-äthylester

Hergestellt analog Beispiel 1 aus 5-Chlor-2-(2,3,3a,4,7,7a-hexahydro-1H-isoindol-2-yl)-benzoesäure und 4-(2-Amino-äthyl)-benzoesäure-äthylester.
Ausbeute: 57 % der Theorie,
Schmelzpunkt: 172°C

| | | | | | | |
|---|---|---|---|---|---|---|
| Ber.: | C | 68,94 | H | 6,45 | N | 6,18 |
| Gef.: | | 68,87 | | 6,44 | | 6,13 |

Beispiel 60

4-/2-(5-Chlor-2-(1,3-dihydro-isoindol-2-yl)-benzoylamino)äthyl7-benzoesäure-äthylester

Hergestellt analog Beispiel 1 aus 5-Chlor-2-(1,3-dihydro-isoindol-2-yl)-benzoesäure und 4-(2-Amino-äthyl)benzoesäure-äthylester.
Ausbeute: 40 % der Theorie,
Schmelzpunkt: 158°C

| | | | | | | | | |
|---|---|---|---|---|---|---|---|---|
| Ber.: | C | 69,56 | H | 5,61 | N | 6,23 | Cl | 7,89 |
| Gef.: | | 69,94 | | 5,58 | | 6,05 | | 7,78 |

Beispiel 61

4-/(5-Chlor-2-piperidino-phenyl)-acetylaminomethyl7benzoesäure

Hergestellt analog Beispiel 5 durch alkalische Hydrolyse von 4-/(5-Chlor-2-piperidino-phenyl)-acetylaminomethyl7benzoesäure-äthylester.
Ausbeute: 92 % der Theorie,
Schmelzpunkt: 187-189°C

| | | | | | | |
|---|---|---|---|---|---|---|
| Ber.: | C | 65,19 | H | 5,99 | N | 7,24 |
| Gef.: | | 64,87 | | 6,11 | | 7,35 |

Beispiel 62

4-/(2-Piperidino-phenyl)-acetylamino-methyl/benzoesäure

1 g (2,6 mMol) 4-/(5-Chlor-2-piperidino-phenyl)-acetylamino-methyl/benzoesäure werden in 100 ml Methanol gelöst und bei einem Wasserstoffdruck von 5 bar bei Raumtemperatur hydriert. Als Katalysator wird 10%ige Palladium/Kohle verwendet. Nach abgeschlossener Wasserstoffaufnahme wird der Katalysator abgetrennt, das Lösungsmittel am Rotationsverdampfer abdestilliert und der Trockenrückstand in Wasser gelöst. Bei pH 6 wird mit Chloroform extrahiert und nach Trocknung der Chloroform-Abdampfrückstand mit Petroläther/Äther behandelt.

Ausbeute: 0,5 g (55 % der Theorie),
Schmelzpunkt: 180°C

| | | | | | | |
|---|---|---|---|---|---|---|
| Ber.: | C | 71,57 | H | 6,86 | N | 7,95 |
| Gef.: | | 71,25 | | 6,80 | | 7,87 |

Beispiel 63

4-/2-/(5-Chlor-2-piperidino-phenyl)-acetylamino/äthyl/benzoesäure

Hergestellt analog Beispiel 5 durch alkalische Hydrolyse von 4-/2-/(5-Chlor-2-piperidino-phenyl)-acetylamino/äthyl/benzoesäure-äthylester.

Ausbeute: 95 % der Theorie,
Schmelzpunkt: 169-170°C

| | | | | | | |
|---|---|---|---|---|---|---|
| Ber.: | C | 65,91 | H | 6,29 | N | 6,99 |
| Gef.: | | 65,50 | | 6,22 | | 6,99 |

Beispiel 64

4-/2-/(2-Piperidino-phenyl)-acetylamino/äthyl/benzoesäure

Hergestellt analog Beispiel 62 durch katalytische Enthalogenierung von 4-/2-/(5-Chlor-2-piperidino-phenyl)-acetylamino/äthyl/-benzoesäure.

Ausbeute: 55 % der Theorie,

Schmelzpunkt: 154°C

| | | | | | | |
|---|---|---|---|---|---|---|
| Ber.: | C | 73,50 | H | 7,90 | N | 6,86 |
| Gef.: | | 73,97 | | 7,95 | | 6,84 |

Beispiel 65

4-/(5-Chlor-2-octahydro-1H-azonino-phenyl)-acetylaminomethyl/-benzoesäure

Hergestellt analog Beispiel 5 durch alkalische Hydrolyse von 4-/(5-Chlor-2-octahydro-1H-azonino-phenyl)-acetylaminomethyl/-benzoesäure-äthylester.

Ausbeute: 91 % der Theorie,

Schmelzpunkt: 191°C

| | | | | | | |
|---|---|---|---|---|---|---|
| Ber.: | C | 67,19 | H | 6,81 | N | 6,53 |
| Gef.: | | 67,08 | | 6,64 | | 6,32 |

Beispiel 66

4-/(2-Octahydro-1H-azonino-phenyl)-acetylaminomethyl/benzoesäure

Hergestellt analog Beispiel 62 durch katalytische Enthalogenierung von 4-/(5-Chlor-2-octahydro-1H-azonino-phenyl)-acetylaminomethyl/benzoesäure.

Ausbeute: 55 % der Theorie,

Schmelzpunkt: 150°C

| | | | | | | |
|---|---|---|---|---|---|---|
| Ber.: | C | 73,06 | H | 7,67 | N | 7,10 |
| Gef.: | | 72,79 | | 7,34 | | 7,19 |

Beispiel 67

4-/2-/(5-Chlor-2-octahydro-1H-azonino-phenyl)-acetylamino/-äthyl/benzoesäure

Hergestellt analog Beispiel 5 durch alkalische Hydrolyse von 4-/2-/(5-Chlor-2-octahydro-1H-azonino-phenyl)-acetylamino/-äthyl/benzoesäure-äthylester.
Ausbeute: 88 % der Theorie,
Schmelzpunkt: 179 °C

| | | | | | | |
|---|---|---|---|---|---|---|
| Ber.: | C | 67,78 | H | 7,05 | N | 6,33 |
| Gef.: | | 67,36 | | 7,12 | | 6,18 |

Beispiel 68

4-/2-/(2-Octahydro-1H-azonino-phenyl)-acetylamino/äthyl/benzoesäure

Hergestellt analog Beispiel 62 durch katalytische Enthalogenierung von 4-/2-/(5-Chlor-2-octahydro-1H-azonino-phenyl)-acetylamino/äthyl/benzoesäure.
Ausbeute: 55 % der Theorie,
Schmelzpunkt: 154 °C

| | | | | | | |
|---|---|---|---|---|---|---|
| Ber.: | C | 73,50 | H | 7,90 | N | 6,86 |
| Gef.: | | 73,97 | | 7,95 | | 6,84 |

Beispiel 69

4-/(5-Chlor-2-(octahydro-isoindol-2-yl)-phenyl)-acetylamino-methyl/benzoesäure

Hergestellt analog Beispiel 5 durch alkalische Hydrolyse von 4-/(5-Chlor-2-(octahydro-isoindol-2-yl)-phenyl)-acetylamino-methyl/benzoesäure-äthylester.
Ausbeute: 78 % der Theorie,
Schmelzpunkt: 200 °C

| | | | | | | |
|---|---|---|---|---|---|---|
| Ber.: | C | 67,51 | H | 6,37 | N | 6,56 |
| Gef.: | | 66,87 | | 6,53 | | 6,56 |

Beispiel 70

DL-4-/(2-(5-Chlor-2-piperidino-phenyl)propionylamino)methyl/-benzoesäure

Hergestellt analog Beispiel 5 durch alkalische Hydrolyse von 4-/(2-(5-Chlor-2-piperidino-phenyl)propionylamino)methyl/benzoesäure-äthylester.

Ausbeute: 65 % der Theorie,

Schmelzpunkt: 170°C

| | | | | | | | | |
|---|---|---|---|---|---|---|---|---|
| Ber.: | C | 65,91 | H | 6,28 | N | 6,98 | Cl | 8,84 |
| Gef.: | | 66,03 | | 6,24 | | 6,90 | | 9,10 |

Beispiel 71

DL-4-/(2-(2-Piperidino-phenyl)propionylamino)methyl/benzoesäure

Hergestellt analog Beispiel 62 durch katalytische Hydrierung von 4-/(2-(5-Chlor-2-piperidino-phenyl)propionylamino)methyl/-benzoesäure.

Ausbeute: 60 % der Theorie,

Schmelzpunkt: 125°C

| | | | | | | |
|---|---|---|---|---|---|---|
| Ber.: | C | 72,10 | H | 7,15 | N | 7,64 |
| Gef.: | | 72,35 | | 7,14 | | 7,46 |

Beispiel 72

DL-4-/2-/2-(5-Chlor-2-piperidino-phenyl)propionylamino/äthyl/-benzoesäure

Hergestellt analog Beispiel 5 durch alkalische Hydrolyse von DL-4-/2-/2-(5-Chlor-2-piperidino)-phenyl)propionylamino/äthyl/-benzoesäure-äthylester.

Ausbeute: 73 % de Theorie,
Schmelzpunkt: 174 °C

| | | | | | | |
|---|---|---|---|---|---|---|
| Ber.: | C | 66,57 | H | 6,55 | N | 6,75 |
| Gef.: | | 66,31 | | 6,92 | | 6,79 |

Beispiel 73

DL-4-/2-/2-(2-Piperidino-phenyl)propionylamino/äthyl/benzoesäure

Hergestellt analog Beispiel 62 durch katalytische Enthalogenierung von DL-4-/2-/2-(5-Chlor-2-piperidino-phenyl)propionylamino/äthyl/benzoesäure.
Ausbeute: 70 % der Theorie,
Schmelzpunkt: 151 °C

| | | | | | | |
|---|---|---|---|---|---|---|
| Ber.: | C | 72,60 | H | 7,41 | N | 7,36 |
| Gef.: | | 72,48 | | 7,38 | | 7,28 |

Beispiel 74

DL-4-/2-/2-(5-Chlor-2-octahydro-1H-azonino-phenyl)-propionylamino/äthyl/benzoesäure

Hergestellt analog Beispiel 5 durch alkalische Hydrolyse von DL-4-/2-/2-(5-Chlor-2-octahydro-1H-azonino-phenyl)propionylamino/äthyl/benzoesäure-äthylester.
Ausbeute: 65 % der Theorie,
Schmelzpunkt: 168-170 °C

| | | | | | | | | |
|---|---|---|---|---|---|---|---|---|
| Ber.: | C | 68,33 | H | 7,27 | N | 6,13 | Cl | 7,75 |
| Gef.: | | 68,00 | | 7,16 | | 6,30 | | 7,74 |

Beispiel 75

DL-4-/2-/2-(2-Octahydro-1H-azonino-phenyl)propionylamino/äthyl/-benzoesäure

Hergestellt analog Beispiel 62 durch katalytische Enthalogenierung von DL-4-/2-/2-(5-Chlor-2-octahydro-1H-azonino-phenyl)-propionylamino/äthyl/benzoesäure.

Ausbeute: 64 % der Theorie,

Schmelzpunkt: 152-154°C

| | | | | | | |
|---|---|---|---|---|---|---|
| Ber.: | C | 73,90 | H | 8,81 | N | 6,62 |
| Gef.: | | 73,70 | | 8,18 | | 6,82 |

Beispiel 76

DL-4-/2-/(2-Piperidino-diphenyl)-acetylamino/äthyl/benzoesäure

Hergestellt durch alkalische Verseifung von 4-/2-/(2-Piperidino-diphenyl)-acetylamino/äthyl/benzoesäure-äthylester analog Beispiel 29.

Ausbeute: 64 % der Theorie,

Schmelzpunkt: 166-168°C

| | | | | | | |
|---|---|---|---|---|---|---|
| Ber.: | C | 75,99 | H | 6,83 | N | 6,33 |
| Gef.: | | 75,54 | | 6,87 | | 6,21 |

Beispiel 77

DL-4-(2-Piperidino-diphenyl)-acetylaminomethyl)benzoesäure

Hergestellt durch alkalische Verseifung von 4-(2-Piperidino-diphenyl)-acetylaminomethyl)benzoesäure-äthylester analog Beispiel 29.

Ausbeute: 75 % der Theorie,

Schmelzpunkt: 220-222°C

| | | | | | | |
|---|---|---|---|---|---|---|
| Ber.: | C | 75,67 | H | 6,58 | N | 6,54 |
| Gef.: | | 75,71 | | 6,63 | | 6,54 |

Beispiel 78

4-/2-/(5-Chlor-2-hexamethylenimino-phenyl)-acetylamino/äthyl/-benzoesäure

Hergestellt analog Beispiel 5 durch alkalische Hydrolyse von 4-/2-/(5-Chlor-2-hexamethylenimino-phenyl)-acetylamino/äthyl/-benzoesäure-äthylester.

Ausbeute: 42 % der Theorie,

Schmelzpunkt: 168°C

| | | | | | | |
|---|---|---|---|---|---|---|
| Ber.: | C | 66,57 | H | 6,56 | N | 6,75 |
| Gef.: | | 66,71 | | 6,73 | | 6,53 |

Beispiel 79

4-/2-/(2-Hexamethylenimino-phenyl)-acetylamino/-äthyl/benzoesäure

Hergestellt analog Beispiel 62 durch katalytische Enthalogenierung von 4-/2-/(5-Chlor-2-hexamethylenimino-phenyl)-acetylamino/-äthyl/benzoesäure.

Ausbeute: 70 % der Theorie,

Schmelzpunkt: 144°C

| | | | | | | |
|---|---|---|---|---|---|---|
| Ber.: | C | 72,60 | H | 7,42 | N | 7,36 |
| Gef.: | | 72,64 | | 7,48 | | 7,38 |

Beispiel 80

4-/2-/(5-Chlor-2-pyrrolidino-phenyl)-acetylamino/äthyl/benzoesäure

Hergestellt analog Beispiel 5 durch alkalische Hydrolyse von 4-/2-/(5-Chlor-2-pyrrolidino-phenyl)-acetylamino/äthyl/benzoesäure-äthylester.

Ausbeute: 86 % der Theorie,
Schmelzpunkt: 174-176°C

| | | | | | | |
|---|---|---|---|---|---|---|
| Ber.: | C | 65,15 | H | 5,99 | N | 7,24 |
| Gef.: | | 65,28 | | 6,18 | | 7,20 |

Beispiel 81

4-/2-/(2-Pyrrolidino-phenyl)-acetylamino/-äthyl/benzoesäure

Hergestellt analog Beispiel 62 durch katalytische Enthalogenierung von 4-/2-/(5-Chlor-2-pyrrolidino-phenyl)-acetylamino/-äthyl/benzoesäure.
Ausbeute: 37 % der Theorie,
Schmelzpunkt: 150-152°C

| | | | | | | |
|---|---|---|---|---|---|---|
| Ber.: | C | 71,57 | H | 6,86 | N | 7,95 |
| Gef.: | | 71,65 | | 6,93 | | 7,68 |

Beispiel 82

4-/2-/(5-Chlor-2-heptamethylenimino-phenyl)-acetylamino/äthyl/-benzoesäure

Hergestellt analog Beispiel 5 durch alkalische Hydrolyse von 4-/2-/(5-Chlor-2-heptamethylenimino-phenyl)-acetylamino/äthyl/-benzoesäure-äthylester.
Ausbeute: 43 % der Theorie,
Schmelzpunkt: 188°C

| | | | | | | |
|---|---|---|---|---|---|---|
| Ber.: | C | 67,20 | H | 6,81 | N | 6,52 |
| Gef.: | | 66,92 | | 6,71 | | 6,45 |

Beispiel 83

4-/2-/(2-Heptamethylenimino-phenyl)-acetylamino/äthyl/benzoesäure

Hergestellt analog Beispiel 62 durch katalytische Enthalogenierung von 4-/2-/(5-Chlor-2-heptamethylenimino-phenyl)-acetylamino/-äthyl/benzoesäure.

| | | | | | | |
|---|---|---|---|---|---|---|
| Ausbeute: | 73 % der Theorie, | | | | | |
| Schmelzpunkt: | 152-154°C | | | | | |
| Ber.: | C | 73,06 | H | 7,66 | N | 7,10 |
| Gef.: | | 73,16 | | 7,80 | | 7,15 |

**Beispiel 84**

**DL-4-/2-/2-(2-Pyrrolidino-phenyl)propionylamino/äthyl/benzoesäure-äthylester**

Hergestellt aus DL-2-(2-Pyrrolidino-phenyl)propionsäure und 4-(2-Aminoäthyl)benzoesäureäthylester analog Beispiel 1.

| | | | | | | |
|---|---|---|---|---|---|---|
| Ausbeute: | 64 % der Theorie, | | | | | |
| Schmelzpunkt: | < 20°C | | | | | |
| Ber.: | Molpeak: m/e = 394 | | | | | |
| Gef.: | Molpeak: m/e = 394 | | | | | |
| Ber.: | C | 73,07 | H | 7,66 | N | 7,10 |
| Gef.: | | 73,30 | | 7,81 | | 6,95 |

**Beispiel 85**

**DL-4-//2-(2-Pyrrolidino-phenyl)propionylamino/-methyl/benzoesäure-äthylester**

Hergestellt aus DL-2-(2-Pyrrolidino-phenyl)propionsäure und 4-Amino-methyl-benzoesäure-äthylester analog Beispiel 1.

| | | | | | | |
|---|---|---|---|---|---|---|
| Ausbeute: | 58 % der Theorie, | | | | | |
| Schmelzpunkt: | < 20°C | | | | | |
| Ber.: | Molpeak: m/e = 380 | | | | | |
| Gef.: | Molpeak: m/e = 380 | | | | | |
| Ber.: | C | 72,60 | H | 7,41 | N | 7,36 |
| Gef.: | | 72,52 | | 7,55 | | 7,40 |

Beispiel 86

DL-4-/2-/2-(2-Hexamethylenimino-phenyl)propionylamino/äthyl/-benzoesäure-äthylester

Hergestellt aus DL-2-(2-Hexamethylenimino-phenyl)propionsäure und 4-(2-Amino-äthyl)benzoesäure-äthylester analog Beispiel 1.

Ausbeute: 72 % der Theorie,

Schmelzpunkt: $< 20^{\circ}C$

Ber.: Molpeak: m/e = 422

Gef.: Molpeak: m/e = 422

| | | | | | | |
|---|---|---|---|---|---|---|
| Ber.: | C | 73,90 | H | 8,11 | N | 6,63 |
| Gef.: | | 73,80 | | 8,05 | | 6,75 |

Beispiel 87

DL-4-//2-(2-Hexamethylenimino-phenyl)propionylamino/methyl/-benzoesäure-äthylester

Hergestellt aus DL-2-(2-Hexamethylenimino-phenyl)propionsäure und 4-(Aminomethyl)benzoesäure-äthylester analog Beispiel 1.

Ausbeute: 69 % der Theorie,

Schmelzpunkt: $< 20^{\circ}C$

Ber.: Molpeak: m/e = 408

Gef.: Molpeak: m/e = 408

| | | | | | | |
|---|---|---|---|---|---|---|
| Ber.: | C | 73,49 | H | 7,89 | N | 6,85 |
| Gef.: | | 73,62 | | 7,92 | | 6,78 |

Beispiel 88

DL-4-/2-/(2-Pyrrolidino-diphenyl)-acetylamino/äthyl/benzoesäure-äthylester

Hergestellt aus (2-Pyrrolidino-diphenyl)-essigsäure und 4-(2-Aminoäthyl)benzoesäure-äthylester analog Beispiel 20.

Ausbeute: 51 % der Theorie,

Schmelzpunkt: $89-92^{\circ}C$

| | | | | | | |
|---|---|---|---|---|---|---|
| Ber.: | C | 76,28 | H | 7,06 | N | 6,14 |
| Gef.: | | 76,01 | | 6,85 | | 6,17 |

Beispiel 89

DL-4-/2-/(2-Hexamethylenimino-diphenyl)-acetylamino/äthyl/-benzoesäure-äthylester

Hergestellt aus (2-Hexamethylenimino-diphenyl)-essigsäure und 4-(2-Aminoäthyl)-benzoesäure-äthylester analog Beispiel 20.
Ausbeute: 49 % der Theorie,
Schmelzpunkt: 80-83 °C

| | | | | | | |
|---|---|---|---|---|---|---|
| Ber.: | C | 76,82 | H | 7,48 | N | 5,78 |
| Gef.: | | 77,01 | | 7,61 | | 5,54 |

Beispiel 90

4-/2-(2-Octahydro-1H-azonino-benzoylamino)äthyl/benzoesäure

Hergestellt analog Beispiel 5 durch alkalische Hydrolyse von 4-/2-(2-Octahydro-1H-azonino-benzoylamino)äthyl/benzoesäure-äthylester.
Ausbeute: 71 % der Theorie,
Schmelzpunkt: 154-156 °C

| | | | | | | |
|---|---|---|---|---|---|---|
| Ber.: | C | 73,06 | H | 7,66 | N | 7,10 |
| Gef.: | | 73,27 | | 7,76 | | 6,92 |

Beispiel 91

4-/2-(5-Chlor-2-(2,3,3a,4,7,7a-hexahydro-1H-isoindol-2-yl)-benzoylamino)äthyl/benzoesäure-semihydrat

Hergestellt analog Beispiel 5 durch alkalische Hydrolyse von 4-/2-(5-Chlor-2-(2,3,3a,4,7,7a-hexahydro-1H-isoindol-2-yl)-benzoylamino)äthyl/benzoesäure-äthylester.

Ausbeute: 86 % der Theorie,
Schmelzpunkt: ab 90°C Zersetzung

| | | | | | | |
|---|---|---|---|---|---|---|
| Ber.: | C | 66,43 | H | 6,03 | N | 6,45 |
| Gef.: | | 66,07 | | 5,78 | | 6,14 |

Beispiel 92

4-/2-(5-Chlor-2-(1,3-dihydro-isoindol-2-yl)-benzoylamino)-äthyl7/benzoesäure

Hergestellt analog Beispiel 5 durch alkalische Hydrolyse von 4-/2-(5-Chlor-2-(1,3-dihydro-isoindol-2-yl)-benzoylamino)äthyl7-benzoesäure-äthylester.
Ausbeute: 74 % der Theorie,
Schmelzpunkt: 224-226°C

| | | | | | | |
|---|---|---|---|---|---|---|
| Ber.: | C | 68,48 | H | 5,02 | N | 6,65 |
| Gef.: | | 68,22 | | 4,98 | | 6,43 |

Beispiel 93

5-Amino-2-(2-azabicyclo/3.3.17nonan-2-yl)-benzoesäure

In 100 ml Dimethylformamid werden 11 g 2-(2-Azabicyclo/3.3.17-nonan-2-yl)-5-nitro-benzoesäure gelöst und bei einem Wasserstoffdruck von 5 bar mit 10%iger Palladiumkohle als Katalysator bei Raumtemperatur hydriert. Nach beendeter Wasserstoffaufnahme wird der Katalysator abfiltriert, das Lösungsmittel im Vakuum abdestilliert und der Rückstand mit Äthanol/Petroläther zur Kristallisation gebracht.

Ausbeute: 7,5 g (76 % der Theorie),
Schmelzpunkt: 215-217°C

| | | | | | | |
|---|---|---|---|---|---|---|
| Ber.: | C | 69,20 | H | 7,74 | N | 10,76 |
| Gef.: | | 69,09 | | 7,44 | | 10,96 |

Beispiel 94

5-Amino-2-[4-(3-pentyl)-piperidino]benzoesäure

Hergestellt analog Beispiel 93 durch katalytische Hydrierung von 5-Nitro-2-[4-(3-pentyl)-piperidino]benzoesäure.
Ausbeute: 94,1 % der Theorie,
Schmelzpunkt: 218°C

| | | | | | | |
|---|---|---|---|---|---|---|
| Ber.: | C | 70,31 | H | 9,02 | N | 9,65 |
| Gef.: | | 70,42 | | 8,87 | | 9,53 |

Beispiel 95

5-Amino-2-[4-(5-nonyl)-piperidino]benzoesäure

Hergestellt analog Beispiel 93 durch katalytische Hydrierung von 5-Nitro-2-[4-(5-nonyl)-piperidino]benzoesäure.
Ausbeute: 85 % der Theorie,
Schmelzpunkt: 130°C

| | | | | | | |
|---|---|---|---|---|---|---|
| Ber.: | C | 72,79 | H | 9,89 | N | 8,24 |
| Gef.: | | 72,63 | | 10,19 | | 8,35 |

Beispiel 96

5-Amino-2-(octahydro-isoindol-2-yl)-benzoesäure

Hergestellt analog Beispiel 93 durch katalytische Hydrierung von 5-Nitro-2-(octahydro-isoindol-2-yl)-benzoesäure.
Ausbeute: 78,7 % der Theorie,
Schmelzpunkt: 244-245°C

| | | | | | | |
|---|---|---|---|---|---|---|
| Ber.: | C | 69,20 | H | 7,74 | N | 10,75 |
| Gef.: | | 69,20 | | 7,86 | | 10,82 |

Beispiel 97

5-Amino-2-(1,3-dihydro-isoindol-2-yl)-benzoesäure

Hergestellt durch katalytische Hydrierung von 2-(1,3-Dihydro-isoindol-2-yl)-5-nitro-benzoesäure analog Beispiel 93.
Ausbeute: 54 % der Theorie,
Schmelzpunkt: 260°C

| | | | | | | |
|---|---|---|---|---|---|---|
| Ber.: | C | 70,85 | H | 5,55 | N | 11,01 |
| Gef.: | | 70,95 | | 5,59 | | 11,08 |

Beispiel 98

5-Amino-2-(2,3,3a,4,7,7a-hexahydro-1H-isoindol-2-yl)-benzoesäure

3,4 g (11,8 mMol) 2-(2,3,3a,4,7,7a-hexahydro-1H-isoindol-2-yl)-5-nitro-benzoesäure werden in 32 ml einer Mischung von konzentrierter Salzsäure und Methanol 1:1 suspendiert und bei 0°C portionsweise mit 15,6 g (69 mMol) Zinn-II-chlorid-dihydrat reduziert. Nach beendeter Zugabe wird einige Stunden bei Raumtemperatur nachgerührt, mit 300 ml $H_2O$ verdünnt, das pH mit Natronlauge auf pH 4 gestellt und mit Chloroform extrahiert. Die Chloroformextrakte werden getrocknet und nach Abdestillieren des Chloroforms im Vakuum wird der Rückstand in Aceton/Äther zur Kristallisation gebracht.
Ausbeute: 2,3 g (75 % der Theorie),
Schmelzpunkt: 287°C

Ber.: Molpeak: m/e = 258
Gef.: m/e = 258

| | | | | | | |
|---|---|---|---|---|---|---|
| Ber.: | C | 69,74 | H | 7,02 | N | 10,84 |
| Gef.: | | 69,20 | | 7,01 | | 11,07 |

Beispiel 99

(5-Amino-2-pyrrolidino-phenyl)essigsäure

Hergestellt durch katalytische Hydrierung von (5-Nitro-2-pyrrolidino-phenyl)essigsäure analog Beispiel 93.
Ausbeute: 72 % der Theorie,
Schmelzpunkt: 158°C

| | | | | | | |
|---|---|---|---|---|---|---|
| Ber.: | C | 65,43 | H | 7,32 | N | 12,72 |
| Gef.: | | 65,70 | | 7,33 | | 12,72 |

Beispiel 100

(5-Amino-2-heptamethylenimino-phenyl)essigsäure

Hergestellt durch katalytische Hydrierung von (5-Nitro-2-heptamethylenimino-phenyl)essigsäure analog Beispiel 93.
Ausbeute: 42 % der Theorie
Schmelzpunkt: 152-154°C

| | | | | | | |
|---|---|---|---|---|---|---|
| Ber.: | C | 68,67 | H | 8,45 | N | 10,67 |
| Gef.: | | 68,50 | | 8,25 | | 10,64 |

Beispiel 101

[5-Amino-2-(octahydro-isoindol-2-yl)-phenyl]-essigsäure

Hergestellt durch katalytische Hydrierung von [2-(Octahydro-isoindol-2-yl)-5-nitro-phenyl]-essigsäure analog Beispiel 93.
Ausbeute: 92 % der Theorie,
Schmelzpunkt: 202-203°C

| | | | | | | |
|---|---|---|---|---|---|---|
| Ber.: | C | 70,04 | H | 8,08 | N | 10,20 |
| Gef.: | | 69,90 | | 7,93 | | 10,46 |

Beispiel 102

/5-Amino-2-(octahydro-1H-azonino)-phenyl7-essigsäure

Hergestellt durch katalytische Hydrierung von /2-(Octahydro-1H-azonino)-5-nitro-phenyl7-essigsäure analog Beispiel 93.
Ausbeute: 74 % der Theorie,
Schmelzpunkt: 187-189°C

| | | | | | | |
|---|---|---|---|---|---|---|
| Ber.: | C | 69,53 | H | 8,75 | N | 10,14 |
| Gef.: | | 69,45 | | 8,74 | | 10,40 |

Beispiel 103

(5-Amino-2-hexamethylenimino-phenyl)-essigsäure

Hergestellt durch katalytische Hydrierung von (2-Hexamethylenimino-5-nitrophenyl)-essigsäure analog Beispiel 93.
Ausbeute: 48 % der Theorie,
Schmelzpunkt: 156°C

| | | | | | | |
|---|---|---|---|---|---|---|
| Ber.: | C | 67,71 | H | 8,12 | N | 11,28 |
| Gef.: | | 67,55 | | 8,10 | | 11,51 |

Beispiel 104

(5-Amino-2-piperidino-phenyl)-essigsäure

Hergestellt durch katalytische Hydrierung von (5-Nitro-2-piperidino-phenyl)-essigsäure analog Beispiel 93.
Ausbeute: 65 % der Theorie,
Schmelzpunkt: 179-181°C

| | | | | | | |
|---|---|---|---|---|---|---|
| Ber.: | C | 66,64 | H | 7,74 | N | 11,96 |
| Gef.: | | 66,95 | | 7,51 | | 12,21 |

Beispiel 105

DL-2-(5-Amino-2-piperidino-phenyl)-propionsäure

Hergestellt aus DL-2-(5-Nitro-2-piperidino-phenyl)-propionsäure durch katalytische Hydrierung analog Beispiel 93.
Ausbeute: 40 % der Theorie,
Schmelzpunkt: 207°C

| | | | | | | |
|---|---|---|---|---|---|---|
| Ber.: | C | 67,71 | H | 8,11 | N | 11,28 |
| Gef.: | | 67,66 | | 8,41 | | 11,35 |

Beispiel 106

DL-2-(5-Amino-2-octahydro-1H-azonino-phenyl)-propionsäure

Hergestellt aus DL-2-(5-Nitro-2-octahydro-1H-azonino-phenyl)-propionsäure durch katalytische Hydrierung analog Beispiel 93.
Ausbeute: 19 % der Theorie,
Schmelzpunkt: 218°C

| | | | | | | |
|---|---|---|---|---|---|---|
| Ber.: | C | 70,31 | H | 9,02 | N | 9,64 |
| Gef.: | | 70,51 | | 9,14 | | 9,78 |

Beispiel 107

DL-2-/5-Amino-2-(octahydro-isoindol-2-yl)-phenyl/-propionsäure

Hergestellt durch katalytische Hydrierung von DL-2-/5-Nitro-2-(octahydro-isoindol-2-yl)-phenyl/-propionsäure analog Beispiel 93.
Ausbeute: 21 % der Theorie,
Schmelzpunkt: 243°C

| | | | | | | |
|---|---|---|---|---|---|---|
| Ber.: | C | 70,80 | H | 8,38 | N | 9,71 |
| Gef.: | | 71,20 | | 8,47 | | 9,88 |

Beispiel 108

5-Chlor-2-(2-azabicyclo/3.3.1/nonan-2-yl)-benzoesäure

6,5 g (25 mMol) 5-Amino-2-(2-azabicyclo/3.3.1/nonan-2-yl)-benzoesäure werden in 90 ml halbkonzentrierter Salzsäure gelöst und bei 0°C mit einer Lösung von 1,5 g (27 mMol) Natriumnitrit in 18 ml Wasser diazotiert. Die Diazoniumlösung wird unter Rühren zu einer Lösung von 3 g (30 mMol) Kupfer-I-chlorid in 45 ml konzentrierter Salzsäure zugetropft. Nach beendeter Stickstoffentwicklung wird 2 bis 3 Stunden nachgerührt. Die Chlorverbindung wird mit Chloroform ausgeschüttelt und über eine Kieselgelsäule mit Essigsäureäthylester als Fließmittel gereinigt.
Ausbeute: 5,2 g (75 % der Theorie),
Schmelzpunkt: 85-87°C

| | | | | | | |
|---|---|---|---|---|---|---|
| Ber.: | C | 64,39 | H | 6,48 | N | 5,01 |
| Gef.: | | 64,60 | | 6,12 | | 5,04 |

Beispiel 109

5-Chlor-2-/4-(3-pentyl)-piperidino/-benzoesäure

Hergestellt analog Beispiel 108 durch Sandmeyer-Reaktion ausgehend von 5-Amino-2-/4-(3-pentyl)-piperidino/benzoesäure.
Ausbeute: 60,6 % der Theorie,
Schmelzpunkt: 150°C

| | | | | | | |
|---|---|---|---|---|---|---|
| Ber.: | C | 65,90 | H | 7,80 | N | 4,52 |
| Gef.: | | 65,80 | | 7,67 | | 4,62 |

Beispiel 110

5-Chlor-2-/4-(5-nonyl)-piperidino/benzoesäure

Hergestellt analog Beispiel 108 durch Sandmeyer-Reaktion ausgehend von 5-Amino-2-/4-(5-nonyl)-piperidino/benzoesäure.

Ausbeute: 58 % der Theorie,
Schmelzpunkt: 148-150°C.

| | | | | | | |
|---|---|---|---|---|---|---|
| Ber.: | C | 68,92 | H | 8,82 | N | 3,83 |
| Gef.: | | 68,70 | | 8,76 | | 3,78 |

Beispiel 111

5-Brom-2-(octahydro-isoindol-2-yl)-benzoesäure

Hergestellt analog Beispiel 108 durch Sandmeyer-Reaktion ausgehend von 5-Amino-2-(octahydro-isoindol-2-yl)-benzoesäure.
Ausbeute: 21 % der Theorie,
Schmelzpunkt: 176°C

| | | | | | | |
|---|---|---|---|---|---|---|
| Ber.: | C | 55,56 | H | 5,59 | N | 4,31 |
| Gef.: | | 55,70 | | 5,70 | | 4,53 |

Beispiel 112

5-Jod-2-(octahydro-isoindol-2-yl)-benzoesäure

Hergestellt analog Beispiel 108 durch Sandmeyer-Reaktion ausgehend von 5-Amino-2-(octahydro-isoindol-2-yl)-benzoesäure.
Ausbeute: 19 % der Theorie,
Schmelzpunkt: 174°C

| | | | | | | | | |
|---|---|---|---|---|---|---|---|---|
| Ber.: | H | 48,53 | H | 4,88 | N | 3,77 | J | 34,18 |
| Gef.: | | 48,56 | | 4,69 | | 3,76 | | 33,80 |

Beispiel 113

5-Cyan-2-(octahydro-isoindol-2-yl)-benzoesäure

Hergestellt analog Beispiel 108 durch Sandmeyer-Reaktion ausgehend von 5-Amino-2-(octahydro-isoindol-2-yl)-benzoesäure.

Ausbeute: 33 % der Theorie,
Schmelzpunkt: 190°C

| | | | | | | |
|---|---|---|---|---|---|---|
| Ber.: | C | 71,08 | H | 6,71 | N | 10,36 |
| Gef.: | | 70,80 | | 6,47 | | 10,02 |

Beispiel 114

5-Fluor-2-(octahydro-isoindol-2-yl)-benzoesäure

Hergestellt ausgehend von 5-Amino-2-(octahydro-isoindol-2-yl)-benzoesäure über das /5-Diazonium-2-(octahydro-isoindol-2-yl)-benzoesäure/tetrafluorborat /Ausbeute 73 %, Schmelzpunkt: 173°C (Zersetzung)/ und anschließender thermischer Zersetzung.
Ausbeute: 2 % der Theorie,
Ber.: Molpeak m/e = 263
Gef.: Molpeak m/e = 263

Beispiel 115

5-Hydroxy-2-(octahydro-isoindol-2-yl)-benzoesäure

Hergestellt aus 5-Amino-2-(octahydro-isoindol-2-yl)-benzoesäure durch Diazotierung und anschließende Verkochung des Diazoniumsalzes in 50%iger Schwefelsäure bei 80-90°C.
Ausbeute: 61 % der Theorie,
Schmelzpunkt: 222-223°C

| | | | | | | |
|---|---|---|---|---|---|---|
| Ber.: | C | 68,94 | H | 7,37 | N | 5,35 |
| Gef.: | | 68,70 | | 7,36 | | 5,30 |

Beispiel 116

5-Methoxy-2-(octahydro-isoindol-2-yl)-benzoesäure-methylester

1 g (3,8 mMol) 5-Hydroxy-2-(octahydro-isoindol-2-yl)-benzoesäure werden in 10 ml absolutem Dimethylformamid mit 0,19 g (8 mMol) 50%iger Natriumhydrid-Dispersion in das Dinatriumsalz überführt und anschließend mit 1,66 g (11,7 mMol) Methyljodid alkyliert.
Ausbeute: 0,9 g (82 % der Theorie),
Schmelzpunkt: 66-68°C

| | | | | | | |
|---|---|---|---|---|---|---|
| Ber.: | C | 70,56 | H | 8,01 | N | 4,83 |
| Gef.: | | 70,63 | | 8,03 | | 4,89 |

Beispiel 117

5-Methoxy-2-(octahydro-isoindol-2-yl)-benzoesäure

Hergestellt analog Beispiel 5 durch alkalische Hydrolyse von 5-Methoxy-2-(octahydro-isoindol-2-yl)-benzoesäure-methylester.
Ausbeute: 91 % der Theorie,
Schmelzpunkt: 132-134°C

| | | | | | | |
|---|---|---|---|---|---|---|
| Ber.: | C | 69,79 | H | 7,68 | N | 5,08 |
| Gef.: | | 69,30 | | 7,60 | | 5,03 |

Beispiel 118

5-Methyl-2-(octahydro-isoindol-2-yl)-benzoesäure

Hergestellt analog Beispiel 135 aus 3-Brom-4-(octahydro-isoindol-2-yl)-toluol (Schmelzpunkt: 110-112°C) mit Butyllithium und anschließender Carboxylierung.
Ausbeute: 46 % der Theorie,
Schmelzpunkt: 157-159°C

| | | | | | | |
|---|---|---|---|---|---|---|
| Ber.: | C | 74,10 | H | 8,15 | N | 5,39 |
| Gef.: | | 74,40 | | 8,29 | | 5,42 |

Beispiel 119

5-Chlor-2-(2,3,3a,4,7,7a-hexahydro-1H-isoindol-2-yl)-benzoesäure

Hergestellt durch Sandmeyer-Reaktion analog Beispiel 108 ausgehend von 5-Amino-2-(2,3,3a,4,7,7a-hexahydro-1H-isoindol-2-yl)-benzoesäure.
Ausbeute: 12 % der Theorie,
Schmelzpunkt: 201°C

| | | | | | | |
|---|---|---|---|---|---|---|
| Ber.: | C | 67,34 | H | 6,71 | N | 14,72 |
| Gef.: | | 67,25 | | 6,74 | | 14,70 |

Beispiel 120

2-(2,3,3a,4,7,7a-Hexahydro-1H-isoindol-2-yl)-benzoesäure

Hergestellt aus 5-Amino-2-(2,3,3a,4,7,7a-hexahydro-1H-isoindol-2-yl)-benzoesäure durch Diazotierung mit Natriumnitrit und anschließende Reduktion mit Kupfer/Salzsäure.
Ausbeute: 5 % der Theorie,
Schmelzpunkt: 156°C

| | | | | | | |
|---|---|---|---|---|---|---|
| Ber.: | C | 74,05 | H | 7,04 | N | 5,75 |
| Gef.: | | 74,24 | | 7,08 | | 6,00 |

Beispiel 121

5-Acetamino-2-(2,3,3a,4,7,7a-hexahydro-1H-isoindol-2-yl)-benzoesäure

Hergestellt aus 5-Amino-2-(2,3,3a,4,7,7a-hexahydro-1H-isoindol-2-yl)benzoesäure durch Acylierung mit Acetylchlorid in Pyridin.

Ausbeute: 76 % der Theorie,
Schmelzpunkt: 226-227°C

| | | | | | | |
|---|---|---|---|---|---|---|
| Ber.: | C | 68,00 | H | 6,71 | N | 9,32 |
| Gef.: | | 67,90 | | 6,71 | | 9,38 |

Beispiel 122

5-Chlor-2-(1,3-dihydro-isoindol-2-yl)-benzoesäure

Hergestellt analog Beispiel 108 durch Sandmeyer-Reaktion ausgehend von 5-Amino-2-(1,3-dihydro-isoindol-2-yl)-benzoesäure.
Ausbeute: 37 % der Theorie,
Schmelzpunkt: 162°C

| | | | | | | | | |
|---|---|---|---|---|---|---|---|---|
| Ber.: | C | 65,82 | H | 4,41 | N | 5,11 | Cl | 12,95 |
| Gef.: | | 65,73 | | 4,64 | | 5,05 | | 12,86 |

Beispiel 123

5-Brom-2-(1,3-dihydro-isoindol-2-yl)-benzoesäure

Hergestellt analog Beispiel 108 durch Sandmeyer-Reaktion ausgehend von 5-Amino-2-(1,3-dihydro-isoindol-2-yl)-benzoesäure.
Ausbeute: 63 % der Theorie,
Schmelzpunkt: 178°C

| | | | | | | |
|---|---|---|---|---|---|---|
| Ber.: | C | 56,62 | H | 3,80 | N | 4,40 |
| Gef.: | | 56,90 | | 3,82 | | 4,43 |

Beispiel 124

2-(1,3-Dihydro-isoindol-2-yl)-benzoesäure

Hergestellt analog Beispiel 62 durch katalytische Enthalogenierung von 5-Chlor-2-(1,3-dihydro-isoindol-2-yl)-benzoesäure.

Ausbeute: 68 % der Theorie,
Schmelzpunkt: 146-148°C

| | | | | | | |
|---|---|---|---|---|---|---|
| Ber.: | C | 75,29 | H | 5,47 | N | 5,85 |
| Gef.: | | 75,02 | | 5,26 | | 5,75 |

Beispiel 125

5-Amino-2-(1,3-dihydro-5-chlor-isoindol-2-yl)-benzoesäure

Hergestellt analog Beispiel 93 durch Reduktion von 2-(1,3-Dihydro-5-chlor-isoindol-2-yl)-5-nitro-benzoesäure mit Zinn-II-chlorid.
Ausbeute: 23 % der Theorie,
Schmelzpunkt: 235°C

| | | | | | | |
|---|---|---|---|---|---|---|
| Ber.: | C | 62,39 | H | 4,54 | N | 9,70 |
| Gef.: | | 62,47 | | 4,67 | | 9,90 |

Beispiel 126

5-Chlor-2-(1,3-dihydro-5-chlor-isoindol-2-yl)-benzoesäure

Hergestellt analog Beispiel 108 durch Sandmeyer-Reaktion ausgehend von 5-Amino-2-(1,3-dihydro-5-chlor-isoindol-2-yl)-benzoesäure.
Ausbeute: 18 % der Theorie,
Schmelzpunkt: 157°C

| | | | | | | |
|---|---|---|---|---|---|---|
| Ber.: | C | 58,46 | H | 3,60 | N | 4,55 |
| Gef.: | | 58,64 | | 3,82 | | 4,70 |

Beispiel 127

5-Amino-2-(1,3-dihydro-5-methoxy-isoindol-2-yl)-benzoesäure

Hergestellt analog Beispiel 93 durch katalytische Hydrierung

von 2-(1,3-Dihydro-5-methoxy-isoindol-2-yl)-5-nitro-benzoesäure.
Ausbeute: 75 % der Theorie,
Schmelzpunkt: 202°C

| | | | | | | |
|---|---|---|---|---|---|---|
| Ber.: | C | 67,59 | H | 5,67 | N | 9,85 |
| Gef.: | | 67,65 | | 5,75 | | 9,98 |

Beispiel 128

5-Chlor-2-(1,3-dihydro-5-methoxy-isoindol-2-yl)-benzoesäure

Hergestellt analog Beispiel 108 durch Sandmeyer-Reaktion ausgehend von 5-Amino-2-(1,3-dihydro-5-methoxy-isoindol-2-yl)-benzoesäure.
Ausbeute: 34 % der Theorie,
Schmelzpunkt: 148°C

| | | | | | | |
|---|---|---|---|---|---|---|
| Ber.: | C | 63,27 | H | 4,64 | N | 4,60 |
| Gef.: | | 63,34 | | 4,53 | | 4,71 |

Beispiel 129

2-(1,3-Dihydro-5-methoxy-isoindol-2-yl)-benzoesäure

Hergestellt analog Beispiel 62 durch katalytische Enthalogenierung von 5-Chlor-2-(1,3-dihydro-5-methoxy-isoindol-2-yl)-benzoesäure.
Ausbeute: 64 % der Theorie,
Schmelzpunkt: 140°C

| | | | | | | |
|---|---|---|---|---|---|---|
| Ber.: | C | 71,36 | H | 5,61 | N | 5,19 |
| Gef.: | | 71,56 | | 5,82 | | 5,34 |

Beispiel 130

2-/4-(3-Pentyl)-piperidino/-benzoesäure

Hergestellt analog Beispiel 62 durch katalytische Enthalogenierung von 5-Chlor-2-/4-(3-pentyl)-piperidino/benzoesäure.
Ausbeute: 31 % der Theorie,
Schmelzpunkt: 134-136°C

| | | | | | | |
|---|---|---|---|---|---|---|
| Ber.: | C | 74,14 | H | 9,15 | N | 5,08 |
| Gef.: | | 73,80 | | 9,26 | | 5,24 |

Beispiel 131

5-Brom-2-/4-(3-pentyl)-piperidino/benzoesäure

Hergestellt analog Beispiel 108 durch Sandmeyer-Reaktion ausgehend von 5-Amino-2-/4-(3-pentyl)-piperidino/benzoesäure
Ausbeute: 21 % der Theorie,
Schmelzpunkt: 157-158°C

| | | | | | | | | |
|---|---|---|---|---|---|---|---|---|
| Ber.: | C | 57,63 | H | 6,82 | N | 3,95 | Br | 21,40 |
| Gef.: | | 57,50 | | 6,76 | | 3,91 | | 21,55 |

Beispiel 132

5-Cyan-2-/4-(3-pentyl)-piperidino/-benzoesäure

Hergestellt analog Beispiel 108 durch Sandmeyer-Reaktion ausgehend von 5-Amino-2-/4-(3-pentyl)-piperidino/benzoesäure.
Ausbeute: 19 % der Theorie,
Schmelzpunkt: 171°C

| | | | | | | |
|---|---|---|---|---|---|---|
| Ber.: | C | 71,97 | H | 8,04 | N | 9,32 |
| Gef.: | | 72,42 | | 8,09 | | 9,36 |

Beispiel 133

4-[4-(3-Pentyl)-piperidino]-isophthalsäure

Hergestellt durch alkalische Verseifung von 5-Cyan-2-[4-(3-pentyl)-piperidino]-benzoesäure analog Beispiel 5.

Ausbeute: 27 % der Theorie,

Schmelzpunkt: 265°C

| | | | | | | |
|---|---|---|---|---|---|---|
| Ber.: | C | 67,68 | H | 7,88 | N | 4,38 |
| Gef.: | | 67,25 | | 7,69 | | 4,41 |

Beispiel 134

5-Acetamino-2-[4-(3-pentyl)-piperidino]-benzoesäure

Hergestellt aus 5-Amino-2-[4-(3-pentyl)-piperidino]-benzoesäure durch Acetylierung mit Essigsäureanhydrid.

Ausbeute: 23 % der Theorie,

Schmelzpunkt: 260-263°C

| | | | | | | |
|---|---|---|---|---|---|---|
| Ber.: | C | 69,02 | H | 8,71 | N | 8,42 |
| Gef.: | | 68,64 | | 8,48 | | 8,44 |

Beispiel 135

5-Methyl-2-(octahydro-1H-azonino)-benzoesäure

3,1 g (10,5 mMol) 3-Brom-4-(octahydro-1H-azonino)-toluol werden in 100 ml absolutem Äther gelöst. Unter Stickstoff werden bei -50°C 20 ml einer 15%igen Butyllithiumlösung in Hexan zugetropft. Dann läßt man auf Raumtemperatur kommen. Anschließend wird die Reaktionsmischung rasch zu einer Suspension von festem Kohlendioxid in absolutem Äther getropft und über Nacht gerührt. Dann wird mit Wasser zersetzt und mit verdünnter Salzsäure auf pH 4-5 eingestellt. Nach Extraktion mit Chloroform wird über Kieselgel

säulenchromatographisch gereinigt (Fließmittel: Toluol/Essigsäureäthylester = 1:1).
Ausbeute: 1,35 g (49,3 % der Theorie),
Schmelzpunkt: 87°C

| | | | | | | |
|---|---|---|---|---|---|---|
| Ber.: | C | 73,53 | H | 8,87 | N | 5,36 |
| Gef.: | | 73,71 | | 8,95 | | 5,12 |

Beispiel 136

DL-2-(5-Chlor-2-piperidino-phenyl)-propionsäure

Hergestellt analog Beispiel 108 durch Sandmeyer-Reaktion ausgehend von DL-2-(5-Amino-2-piperidino-phenyl)-propionsäure.
Ausbeute: 45 % der Theorie,
Schmelzpunkt: 134-135°C

| | | | | | | | | |
|---|---|---|---|---|---|---|---|---|
| Ber.: | C | 62,80 | H | 6,77 | N | 5,23 | Cl | 13,23 |
| Gef.: | | 63,03 | | 6,53 | | 5,27 | | 12,93 |

Beispiel 137

DL-2-(5-Chlor-2-octahydro-1H-azonino-phenyl)-propionsäure

Hergestellt analog Beispiel 108 durch Sandmeyer-Reaktion ausgehend von DL-2-(5-Amino-2-octahydro-1H-azonino-phenyl)-propionsäure.
Ausbeute: 17 % der Theorie,
Schmelzpunkt: 139-140°C

| | | | | | | | | |
|---|---|---|---|---|---|---|---|---|
| Ber.: | C | 65,90 | H | 7,80 | N | 4,52 | Cl | 11,44 |
| Gef.: | | 65,35 | | 7,64 | | 4,44 | | 11,40 |

Beispiel 138

DL-2-/5-Chlor-2-(octahydro-isoindol-2-yl)-phenyl/-propionsäure

Hergestellt analog Beispiel 108 durch Sandmeyer-Reaktion ausgehend von DL-2-/5-Amino-2-(octahydro-isoindol-2-yl)-phenyl/-propionsäure.
Ausbeute: 27 % der Theorie,
Schmelzpunkt: 151-152°C

| | | | | | | |
|---|---|---|---|---|---|---|
| Ber.: | C | 66,33 | H | 7,20 | N | 4,55 |
| Gef.: | | 66,59 | | 7,19 | | 4,70 |

Beispiel 139

(5-Chlor-2-octahydro-1H-azonino-phenyl)-essigsäure

Hergestellt analog Beispiel 108 durch Sandmeyer-Reaktion ausgehend von (5-Amino-2-octahydro-1H-azonino-phenyl)-essigsäure.
Ausbeute: 58 % der Theorie,
Schmelzpunkt: 63-65°C

| | | | | | | |
|---|---|---|---|---|---|---|
| Ber.: | C | 64,96 | H | 7,50 | N | 4,74 |
| Gef.: | | 64,88 | | 7,41 | | 4,53 |

Beispiel 140

(5-Chlor-2-heptamethylenimino-phenyl)-essigsäure

Hergestellt analog Beispiel 108 durch Sandmeyer-Reaktion ausgehend von (5-Amino-2-heptamethylenimino-phenyl)-essigsäure.
Ausbeute: 43 % der Theorie,
Schmelzpunkt: 78°C
Ber.: Molpeak m/e = 280/2
Gef.: Molpeak m/e = 280/2

| | | | | | | |
|---|---|---|---|---|---|---|
| Ber.: | C | 63,94 | H | 7,15 | N | 4,96 |
| Gef.: | | 64,30 | | 7,13 | | 4,84 |

Beispiel 141

(5-Chlor-2-hexamethylenimino-phenyl)-essigsäure

Hergestellt analog Beispiel 108 durch Sandmeyer-Reaktion ausgehend von (5-Amino-2-hexamethylenimino-phenyl)-essigsäure.
Ausbeute: 77 % der Theorie,
Schmelzpunkt: < 20°C

| | | | | | | |
|---|---|---|---|---|---|---|
| Ber.: | C | 62,80 | H | 6,77 | N | 5,23 |
| Gef.: | | 62,85 | | 6,58 | | 5,40 |

Beispiel 142

(5-Chlor-2-piperidino-phenyl)-essigsäure

Hergestellt analog Beispiel 108 durch Sandmeyer-Reaktion ausgehend von (5-Amino-2-piperidino-phenyl)-essigsäure.
Ausbeute: 63 % der Theorie,
Schmelzpunkt: 105°C

| | | | | | | |
|---|---|---|---|---|---|---|
| Ber.: | C | 61,53 | H | 6,36 | N | 5,52 |
| Gef.: | | 61,81 | | 6,58 | | 5,56 |

Beispiel 143

(5-Chlor-2-pyrrolidino-phenyl)-essigsäure

Hergestellt analog Beispiel 108 durch Sandmeyer-Reaktion ausgehend von (5-Amino-2-pyrrolidino-phenyl)-essigsäure.
Ausbeute: 54 % der Theorie,
Schmelzpunkt: 105-107°C

| | | | | | | |
|---|---|---|---|---|---|---|
| Ber.: | C | 60,13 | H | 5,89 | N | 5,84 |
| Gef.: | | 60,11 | | 5,81 | | 5,76 |

Beispiel 144

/5-Chlor-2-(octahydro-isoindol-2-yl)-phenyl/-essigsäure

Hergestellt analog Beispiel 108 durch Sandmeyer-Reaktion ausgehend von /5-Amino-2-(octahydro-isoindol-2-yl)-phenyl/-essigsäure.
Ausbeute: 40 % der Theorie,
Schmelzpunkt: 156-157°C

| | | | | | | |
|---|---|---|---|---|---|---|
| Ber.: | C | 65,41 | H | 6,86 | N | 4,76 |
| Gef.: | | 65,54 | | 6,63 | | 4,70 |

Beispiel 145

/2-(Octahydro-isoindol-2-yl)-phenyl/-essigsäure

Hergestellt analog Beispiel 120 aus /5-Amino-2-(octahydro-isoindol-2-yl)-phenyl/-essigsäure.
Ausbeute: 15 % der Theorie,
Schmelzpunkt: 135-136°C

| | | | | | | |
|---|---|---|---|---|---|---|
| Ber.: | C | 74,10 | H | 8,15 | N | 5,39 |
| Gef.: | | 74,29 | | 8,31 | | 5,30 |

Beispiel 146

/5-Chlor-2-(4-(3-pentyl)-piperidino)-phenyl/-essigsäure

Hergestellt analog Beispiel 108 durch Sandmeyer-Reaktion ausgehend von /5-Amino-2-(4-(3-pentyl)-piperidino)-phenyl/-essigsäure.
Ausbeute: 17 % der Theorie,
Schmelzpunkt: < 20°C

| | | | | | | | | |
|---|---|---|---|---|---|---|---|---|
| Ber.: | C | 66,76 | H | 8,09 | N | 4,33 | Cl | 10,95 |
| Gef.: | | 66,80 | | 8,24 | | 4,21 | | 11,23 |

Beispiel 147

(2-Piperidino-diphenyl)essigsäure-hydrochlorid

a) (2-Piperidino-diphenyl)acetonitril

Zu 17,0 g (52,8 mMol) 2-Piperidino-benzhydryl-chlorid-hydrochlorid (hergestellt aus 2-Piperidino-benzhydrylalkohol durch Behandeln mit Thionylchlorid) in 200 ml Dimethylsulfoxid gibt man 8,5 g (0,17 Mol) Natriumcyanid und läßt 2 Stunden bei 60-70°C nachrühren. Dann gibt man auf kalte verdünnte Natronlauge und extrahiert mit Methylenchlorid. Die eingeengten Extrakte werden nach Zusatz von etwas Aktivkohle mit Petroläther (90-110°C) heiß extrahiert. Nach dem Einengen der Petrolätherextrakte hinterbleibt ein gelbliches Öl.
Ausbeute: 12,7 g (87 % der Theorie), Öl

b) (2-Piperidino-diphenyl)-essigsäure-hydrochlorid

1,5 g (5,4 mMol) (2-Piperidino-diphenyl)-acetonitril werden mit 50 ml konz. Salzsäure 3 Stunden am Rückfluß gekocht. Dann engt man ein bis zur Trockne und kristallisiert aus 2N-Salzsäure um.
Ausbeute: 1,2 g (75,2 % der Theorie),
Schmelzpunkt: 195-200°C (Zersetzung)

| | | | | | | | | |
|---|---|---|---|---|---|---|---|---|
| Ber.: | C | 68,77 | H | 6,68 | N | 4,22 | Cl | 10,68 |
| Gef.: | | 69,05 | | 6,84 | | 4,50 | | 10,50 |

Beispiel 148

2-(Octahydro-isoindol-2-yl)-nicotinsäure

3,15 g (20 mMol) 2-Chlor-nicotinsäure und 2,5 g (20 mMol) Octahydro-isoindol werden in 50 ml Äthanol mit 5,2 g (40 mMol) N-Äthyldiisopropylamin 18 Stunden am Rückfluß erhitzt. Dann wird

eingeengt, in Wasser aufgenommen und mit 20 mMol Eisessig versetzt. Anschließend wird mit Chloroform extrahiert. Die getrockneten Chloroformextrakte werden eingeengt und der erhaltene kristalline Rückstand aus Acetonitril umkristallisiert.

Ausbeute: 1,8 g (37 % der Theorie),

Schmelzpunkt: 217-218°C

| | | | | | | |
|---|---|---|---|---|---|---|
| Ber.: | C | 68,27 | H | 7,37 | N | 11,38 |
| Gef.: | | 68,75 | | 7,33 | | 11,14 |

Beispiel 149

5-Chlor-2-(octahydro-isoindol-2-yl)-nicotinsäure

Hergestellt aus 2,5-Dichlor-nicotinsäure und Octahydro-isoindol analog Beispiel 148.

Ausbeute: 36 % der Theorie,

Schmelzpunkt: 233-235°C

| | | | | | | |
|---|---|---|---|---|---|---|
| Ber.: | C | 59,89 | H | 6,11 | N | 9,98 |
| Gef.: | | 59,89 | | 6,05 | | 10,32 |

Beispiel 150

6-Methyl-2-piperidino-nicotinsäure

Hergestellt aus 2-Chlor-6-methyl-nicotinsäure und Piperidin analog Beispiel 148.

Ausbeute: 68 % der Theorie,

Schmelzpunkt: 120-122°C

| | | | | | | |
|---|---|---|---|---|---|---|
| Ber.: | C | 65,43 | H | 7,32 | N | 12,72 |
| Gef.: | | 65,31 | | 7,27 | | 12,41 |

Beispiel 151

5-Chlor-6-methyl-2-piperidinonicotinsäure

Hergestellt aus 2,5-Dichlor-6-methyl-nicotinsäure und Piperidin analog Beispiel 148.
Ausbeute: 56 % der Theorie,
Schmelzpunkt: 130-132°C

| | | | | | | | | |
|---|---|---|---|---|---|---|---|---|
| Ber.: | C | 56,58 | H | 5,93 | N | 11,00 | Cl | 13,92 |
| Gef.: | | 56,32 | | 5,67 | | 10,98 | | 13,71 |

Beispiel 152

5-Chlor-2-octahydro-1H-azonino-nicotinsäure

Hergestellt aus 2,5-Dichlor-nicotinsäure und Octahydro-1H-azonin analog Beispiel 148.
Ausbeute: 57 % der Theorie,
Schmelzpunkt: 154-156°C

| | | | | | | | | |
|---|---|---|---|---|---|---|---|---|
| Ber.: | C | 59,47 | H | 6,77 | N | 9,91 | Cl | 12,54 |
| Gef.: | | 59,66 | | 6,54 | | 10,08 | | 12,45 |

Beispiel 153

5-Chlor-2-(cis-3,5-dimethyl-piperidino)-nicotinsäure

Hergestellt aus 2,5-Dichlornicotinsäure und cis-3,5-Dimethyl-piperidin analog Beispiel 148.
Ausbeute: 40 % der Theorie,
Schmelzpunkt: 78-80°C

| | | | | | | | | |
|---|---|---|---|---|---|---|---|---|
| Ber.: | C | 58,10 | H | 6,37 | N | 10,43 | Cl | 13,19 |
| Gef.: | | 58,32 | | 6,49 | | 10,21 | | 13,02 |

Beispiel 154

5-Chlor-2-piperidino-nicotinsäure

Hergestellt aus 2,5-Dichlor-nicotinsäure und Piperidin analog Beispiel 148.
Ausbeute: 63 % der Theorie,
Schmelzpunkt: 149-152°C

| | | | | | | | | |
|---|---|---|---|---|---|---|---|---|
| Ber.: | C | 54,89 | H | 5,44 | N | 11,64 | Cl | 14,73 |
| Gef.: | | 54,91 | | 5,56 | | 11,47 | | 14,51 |

Beispiel 155

5-Brom-6-methyl-2-piperidino-nicotinsäure

Hergestellt aus 5-Brom-2-chlor-6-methyl-nicotinsäure und Piperidin analog Beispiel 148.
Ausbeute: 35 % der Theorie,
Schmelzpunkt: 108-111°C

| | | | | | | | | |
|---|---|---|---|---|---|---|---|---|
| Ber.: | C | 48,18 | H | 5,05 | N | 9,31 | Br | 26,70 |
| Gef.: | | 48,31 | | 5,16 | | 9,07 | | 26,43 |

Beispiel 156

5-Brom-2-piperidino-nicotinsäure

Hergestellt aus 5-Brom-2-chlor-nicotinsäure und Piperidin analog Beispiel 148.
Ausbeute: 43 % der Theorie,
Schmelzpunkt: 172-174°C

| | | | | | | | | |
|---|---|---|---|---|---|---|---|---|
| Ber.: | C | 46,33 | H | 4,59 | N | 9,82 | Br | 28,02 |
| Gef.: | | 46,45 | | 4,81 | | 9,55 | | 27,80 |

Beispiel 157

/2-Piperidino-pyridyl-(3)7essigsäure

Hergestellt durch alkalische Verseifung von /2-Piperidino-pyridyl-(3)7essigsäure-äthylester vom Schmelzpunkt < 20°C (erhalten aus /2-Piperidino-pyridyl-(3)7-acetonitril durch Kochen mit äthanolischer Salzsäure) analog Beispiel 5.
Ausbeute: 81 % der Theorie,
Schmelzpunkt: 112-115°C

| | | | | | | |
|---|---|---|---|---|---|---|
| Ber.: | C | 65,43 | H | 7,32 | N | 12,72 |
| Gef.: | | 64,98 | | 7,71 | | 12,56 |

Beispiel 158

/5-Chlor-2-piperidino-pyridyl-(3)7essigsäure

Hergestellt durch alkalische Verseifung von /5-Chlor-2-piperidino-pyridyl-(3)7essigsäure-äthylester vom Schmelzpunkt < 20°C (erhalten aus /5-Chlor-2-piperidino-pyridyl-(3)7acetonitril durch Kochen mit äthanolischer Salzsäure) analog Beispiel 5.
Ausbeute: 89 % der Theorie,
Schmelzpunkt: 124-126°C

| | | | | | | | | |
|---|---|---|---|---|---|---|---|---|
| Ber.: | C | 56,58 | H | 5,93 | N | 11,06 | Cl | 13,92 |
| Gef.: | | 56,41 | | 5,98 | | 10,57 | | 13,51 |

Beispiel 159

5-Chlor-6-methyl-2-(cis-3,5-dimethyl-piperidino)nicotinsäure

Hergestellt aus 2,5-Dichlor-6-methyl-nicotinsäure und cis-3,5-Dimethyl-piperidin analog Beispiel 148.
Ausbeute: 18 % der Theorie,
Schmelzpunkt: < 20°C

| | | | | | | | | |
|---|---|---|---|---|---|---|---|---|
| Ber.: | C | 59,46 | H | 6,77 | N | 9,91 | Cl | 12,54 |
| Gef.: | | 59,31 | | 6,68 | | 9,75 | | 12,31 |

Beispiel 160

5-Brom-6-methyl-2-(cis-3,5-dimethyl-piperidino)nicotinsäure

Hergestellt aus 5-Brom-2-chlor-6-methyl-nicotinsäure und cis-3,5-Dimethyl-piperidin analog Beispiel 148.
Ausbeute: 28 % der Theorie,
Schmelzpunkt: $< 20^{\circ}C$

| | | | | | | | | |
|---|---|---|---|---|---|---|---|---|
| Ber.: | C | 51,39 | H | 5,85 | N | 8,56 | Br | 24,42 |
| Gef.: | | 51,12 | | 5,76 | | 8,47 | | 24,31 |

Beispiel 161

3-Methyl-2-piperidino-benzoesäure

Hergestellt analog Beispiel 135 durch Umsetzung von 3-Brom-2-piperidino-toluol mit Butyl-lithium und anschließender Carboxylierung mit Kohlendioxid.
Ausbeute: 64 % der Theorie,
Schmelzpunkt: $126-128^{\circ}C$
Ber.: Molpeak: m/e = 219
Gef.: Molpeak: m/e = 219

| | | | | | | |
|---|---|---|---|---|---|---|
| Ber.: | C | 71,20 | H | 7,81 | N | 6,39 |
| Gef.: | | 71,35 | | 7,74 | | 6,27 |

Beispiel 162

3-Chlor-2-piperidino-benzoesäure

Hergestellt analog Beispiel 108 durch Sandmeyer-Reaktion aus 3-Amino-2-piperidino-benzoesäure.
Ausbeute: 54 % der Theorie,
Ber.: Molpeak: m/e = 239/241 (1 Cl)
Gef.: Molpeak: m/e = 239/241 (1 Cl)

| | | | | | | | | |
|---|---|---|---|---|---|---|---|---|
| Ber.: | C | 60,13 | H | 5,89 | Cl | 14,79 | N | 5,84 |
| Gef.: | | 59,91 | | 5,78 | | 14,93 | | 5,81 |

Beispiel 163

3-Methyl-2-(octahydro-isoindol-2-yl)-benzoesäure

Hergestellt analog Beispiel 135 durch Umsetzung von 3-Brom-2-(octahydro-isoindol-2-yl)-toluol mit Butyllithium und anschließender Carboxylierung mit Kohlendioxyd.
Ausbeute: 53 % der Theorie,

| | | | | | | |
|---|---|---|---|---|---|---|
| Ber.: | C | 74,10 | H | 8,16 | N | 5,40 |
| Gef.: | | 74,27 | | 8,29 | | 5,53 |

Ber.: Molpeak m/e = 259
Gef.: Molpeak m/e = 259

Beispiel 164

3-Chlor-2-(octahydro-isoindol-2-yl)-benzoesäure

Hergestellt analog Beispiel 108 durch Sandmeyer-Reaktion ausgehend von 3-Amino-2-(octahydro-isoindol-2-yl)-benzoesäure.
Ausbeute: 59 % der Theorie,

| | | | | | | | | |
|---|---|---|---|---|---|---|---|---|
| Ber.: | C | 64,40 | H | 6,48 | Cl | 12,67 | N | 5,00 |
| Gef.: | | 64,27 | | 6,40 | | 12,49 | | 5,12 |

Ber.: Molpeak m/e = 279/281 (1 Cl)
Gef.: Molpeak m/e = 279/281 (1 Cl)

Beispiel I

Tabletten mit 5 mg 4-/2-/2-(2-Azabicyclo/3.3.1/nonan-2-yl)-5-chlor-benzoylamino/äthyl/benzoesäure

Zusammensetzung:
1 Tablette enthält:

| | | |
|---|---|---|
| Wirksubstanz | (1) | 5,0 mg |
| Maisstärke | (2) | 62,0 mg |
| Milchzucker | (3) | 48,0 mg |
| Polyvinylpyrrolidon | (4) | 4,0 mg |
| Magnesiumstearat | (5) | 1,0 mg |
| | | 120,0 mg |

Herstellungsverfahren:

1, 2, 3 und 4 werden gemischt und mit Wasser befeuchtet. Die feuchte Mischung wird durch ein Sieb mit 1,5 mm-Maschenweite gedrückt und bei ca. 45 °C getrocknet. Das trockene Granulat wird durch ein Sieb mit 1,0 mm-Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel II

Dragées mit 2,5 mg 4-/2-/2-(2-Azabicyclo/3.3.1/nonan-2-yl)-5-chlor-benzoylamino/äthyl/benzoesäure

1 Drageekern enthält:

| | | |
|---|---|---|
| Wirksubstanz | (1) | 2,5 mg |
| Kartoffelstärke | (2) | 44,0 mg |
| Milchzucker | (3) | 30,0 mg |

| | | |
|---|---|---|
| Polyvinylpyrrolidon | (4) | 3,0 mg |
| Magnesiumstearat | (5) | 0,5 mg |
| | | 80,0 mg |

Herstellungsverfahren:

1, 2, 3 und 4 werden gut gemischt und mit Wasser befeuchtet. Die feuchte Masse drückt man durch ein Sieb mit 1 mm Maschenweite, trocknet bei ca. 45°C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 120 mg

Beispiel III

Suppositorien zu 30 mg 5-Chlor-2-/4-(3-pentyl)-piperidino/-benzoesäure

Zusammensetzung:
1 Zäpfchen enthält:

| | |
|---|---|
| Wirksubstanz | 0,030 g |
| Zäpfchenmasse (z.B. Witepsol W 45 und | 1,336 g |
| Witepsol E 75) | 0,334 g |
| | 1,700 g |

Herstellung:

Der gemahlene Wirkstoff wird unter Rühren in das auf 40°C temperierte aufgeschmolzene Gemisch der Zäpfchenmassen eingetragen und die Schmelze in gekühlte Gießformen ausgegossen. Nach dem völligen Erstarren werden die Suppositorien den Formen entnommen und in geeigneter Weise verpackt.

**Beispiel IV**

Gelatine-Steckkapseln zu 5 mg 5-Chlor-2-[4-(3-pentyl)-piperidino]benzoesäure

1 Kapsel enthält:

| | |
|---|---|
| Wirksubstanz | 5,0 mg |
| Maisstärke getr. | 100,0 mg |
| Maisstärke pulv. | 93,0 mg |
| Magnesiumstearat | 2,0 mg |
| | 200,0 mg |

**Herstellung:**

Wirkstoff und Hilfsstoffe werden gemischt, durch ein Sieb der Maschenweite 0,75 mm gegeben und in einem geeigneten Mischer homogen verteilt. Das Pulver wird auf einem Kapselfüll- und Schließautomaten in Hartgelatine-Steckkapseln der Größe 3 (Parke Davis) abgefüllt.

**Beispiel V**

Tabletten zu 25 mg 5-Chlor-2-[4-(3-pentyl)-piperidino]benzoesäure

1 Tablette enthält:

| | |
|---|---|
| Wirksubstanz | 25,0 mg |
| Milchzucker | 35,0 mg |
| Maisstärke | 15,0 mg |
| Polyvinylpyrrolidon | 4,5 mg |
| Magnesiumstearat | 0,5 mg |
| | 80,0 mg |

Herstellung:

Der Wirkstoff wird mit Milchzucker und Stärke gemischt und danach mit der wäßrigen Polyvinylpyrrolidon-Lösung gleichmäßig befeuchtet.

| | |
|---|---|
| Feuchtsiebung: | 1,5 mm-Maschenweite |
| Trocknung: | Umlufttrockenschrank bei 45°C |
| Trockensiebung: | 1,0 mm-Maschenweite |

Das trockne Granulat wird nach Zumischung des Schmiermittels zu Tabletten verpreßt.

Tabletten: 6 mm Ø, beidseitige Facette, einseitige Teilkerbe, biplan.

Beispiel VI

Dragées zu 25 mg 5-Chlor-2-[4-(3-pentyl)-piperidino]benzoesäure

Hergestellung des preßfertigen Mischung analog Beispiel IV.

Verpressung zu bikonvexen Dragéekernen von 80,0 mg Gewicht, 5 mm Ø und Wölbungsradius 5 mm.

Die Kerne werden mit einer praxisüblichen Zuckerdragiersuspension auf ein Gewicht von 110 mg im Dragierkessel dragiert und anschließend mit einer Poliersuspension poliert.

Patentansprüche

1. Neue Carbonsäure-Derivate der allgemeinen Formel

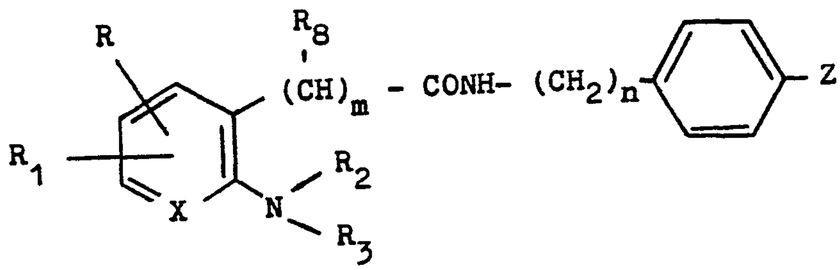

,(I)

in der

m die Zahl 0 oder 1,

n die Zahl 1 oder 2,

R ein Wasserstoffatom oder eine Alkylgruppe mit 1 bis 3 Kohlenstoffatomen,

$R_1$ ein Wasserstoff-, Fluor-, Chlor-, Brom- oder Jodatom, eine Alkyl- oder Alkoxygruppe mit jeweils 1 bis 4 Kohlenstoffatomen, eine Cyano-, Hydroxy- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_2$ und $R_3$ zusammen mit dem dazwischen liegenden Stickstoffatom eine Piperidino-, 3,5-Dimethyl-piperidino-, Octahydro-1H-azonino-, Decahydro-azecino-, 1,3-Dihydro-isoindolo-, Hexahydro-isoindolo- oder Octahydro-isoindologruppe, eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, der durch eine oder mehrere Alkylgruppen mit jeweils 1 bis 3 Kohlenstoffatomen substituiert sein kann, oder eine durch

ein Halogenatom, eine Alkoxygruppe mit 1 bis 3 Kohlenstoffatomen oder eine Aminogruppe substituierte 1,3-Dihydro-isoindolgruppe oder auch, wenn m die Zahl 1 darstellt, die Pyrrolidino-, Hexamethylenimino- oder Heptamethyleniminogruppe,

X eine CH-Gruppe oder ein Stickstoffatom,

$R_8$ ein Wasserstoffatom, eine Alkylgruppe mit 1 bis 3 Kohlenstoffatomen oder eine Arylgruppe und

Z eine gegebenenfalls veresterte Carboxygruppe bedeuten, deren optisch aktive Antipoden, sofern sie ein asymmetrisches Kohlenstoffatom enthalten, und deren Salze, insbesondere deren physiologisch verträgliche Säureadditionssalze mit anorganischen oder organischen Säuren oder Basen.

2. Neue Carbonsäure-Derivate der allgemeinen Formel I gemäß Anspruch 1, in der

m die Zahl 0,

n die Zahl 2,

R ein Wasserstoffatom oder die Methylgruppe,

$R_1$ ein Fluor-, Chlor-, Brom- oder Jodatom, eine Methoxy-, Trifluormethyl-, Cyan-, Methylphenyl- oder Chlorphenylgruppe oder eine Alkylgruppe mit 1 bis 4 Kohlenstoffatomen,

$R_2$ und $R_3$ zusammen mit dem dazwischenliegenden Stickstoffatom eine Piperidino-, 3,5-Dimethyl-piperidino-, Octahydro-1H-azonino-, Decahydro-azecino- oder Octahydroisoindologruppe, eine in 4-Stellung durch eine Alkylgruppe mit 5 bis 9 Kohlenstoffatomen substituierte Piperidinogruppe oder eine Azabicycloalkylgruppe mit 7 bis 12 Kohlenstoffatomen, die durch eine oder zwei Methylgruppen substituiert sein kann,

X eine CH-Gruppe oder ein Stickstoffatom und

Z eine Carboxy- oder Alkoxycarbonylgruppe, in der die Alkoxygruppe 1 bis 3 Kohlenstoffatome enthalten kann, bedeuten, und deren Additionssalze, insbesondere deren physiologisch verträgliche Salze mit anorganischen oder organischen Säuren und auch Basen, wenn Z eine Carboxygruppe darstellt.

3. Neue Carbonsäure-Derivate der allgemeinen Formel I gemäß Anspruch 1, in der
m die Zahl 0 oder 1,
n die Zahl 1 oder 2,
X die CH-Gruppe oder ein Stickstoffatom,
R ein Wasserstoffatom oder die Methylgruppe,
$R_1$ ein Wasserstoff-, Fluor-, Chlor-, Brom- oder Jodatom, eine Cyano-, Trifluormethyl-, Hydroxy-, Methoxy-, Methyl-, Methylphenyl-, Chlorphenyl- oder Bromphenylgruppe,
$R_2$ und $R_3$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch eine Methoxy- oder Aminogruppe, ein Chlor- oder Bromatom substituierte 1,3-Dihydro-isoindologruppe, eine Hexahydro-isoindolo- oder 2-Aza-bicyclo-nonan-2-ylgruppe oder

eine Octahydro-1H-azoninogruppe, wenn
m die Zahl 1 oder
m die Zahl 0,
$R_1$ ein Wasserstoff-, Fluor- oder Jodatom oder eine Methylgruppe und
X eine CH-Gruppe oder
m die Zahl 0,
X ein Stickstoffatom und
$R_1$ kein Wasserstoffatom bedeuten, oder

eine Decahydro-1H-azecinogruppe, wenn
m die Zahl 1 oder
m die Zahl 0,
$R_1$ eine Methylgruppe, ein Wasserstoff-, Fluor-, Brom- oder Jodatom und

X die CH-Gruppe oder
m die Zahl O,
X ein Stickstoffatom und
$R_1$ kein Wasserstoffatom bedeuten, oder

eine Piperidinogruppe, wenn
m die Zahl 1 oder
m die Zahl O,
X ein Stickstoffatom und
$R_1$ kein Wasserstoffatom oder
m die Zahl O,
X eine CH-Gruppe und
$R_1$ ein Wasserstoffatom bedeuten, oder

eine Octahydro-isoindologruppe, wenn
m die Zahl 1 oder
m die Zahl O,
$R_1$ ein Wasserstoff-, Fluor-, Brom- oder Jodatom, eine Methyl-, Hydroxy-, Methoxy- oder Cyanogruppe und
X die CH-Gruppe oder
m die Zahl O und
X ein Stickstoffatom bedeuten, oder

eine 3,5-Dimethyl-piperidinogruppe, wenn
m die Zahl 1 oder
m die Zahl O,
$R_1$ ein Wasserstoff-, Chlor- oder Bromatom,
R die Methylgruppe und
X ein Stickstoffatom oder
m die Zahl O,
$R_1$ eine Methyl-, Hydroxy- oder Cyangruppe, ein Fluor- oder Jodatom und
X eine CH-Gruppe bedeuten, oder
auch eine Pyrrolidino-, Hexamethylenimino- oder Heptamethyleniminogruppe, wenn
m die Zahl 1 darstellt,

$R_8$ ein Wasserstoffatom, eine Methyl- oder Phenylgruppe und

Z eine Carboxy- oder Alkoxycarbonylgruppe mit insgesamt 2 bis 4 Kohlenstoffatomen bedeuten, deren optisch aktive Antipoden, sofern sie ein asymmetrisches Kohlenstoffatom enthalten, und deren Salze, insbesondere deren physiologisch verträgliche Säureadditionssalze mit anorganischen oder organischen Säuren oder Basen.

4. Neue Carbonsäure-Derivate der allgemeinen Formel I gemäß Anspruch 1, in der

m die Zahl 0 oder 1,

n die Zahl 2,

X eine CH-Gruppe,

Z eine Carboxy- oder Alkoxycarbonylgruppe mit insgesamt 2 bis 4 Kohlenstoffatomen,

R ein Wasserstoffatom,

$R_1$ in 5-Stellung ein Fluor-, Chlor-, Brom- oder Jodatom oder eine Methylgruppe oder auch ein Wasserstoffatom, wenn $R_2$ und $R_3$ zusammen mit dem dazwischenliegenden Stickstoffatom die Octahydro-1H-azoninogruppe darstellt,

$R_2$ und $R_3$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch ein Chlor- oder Bromatom substituierte 1,3-Dihydro-isoindologruppe, eine 2-Aza-bicyclo-nonan-3-al- oder Hexahydro-isoindologruppe oder

eine Octahydro-1H-azoninogruppe, wenn

m die Zahl 1 oder

m die Zahl 0 und

$R_1$ eine Methylgruppe, ein Wasserstoff-, Fluor- oder Jodatom darstellen, oder

eine Pyrrolidino-, Piperidino- oder Hexamethyleniminogruppe, wenn

m die Zahl 1 darstellt, und

$R_8$ ein Wasserstoffatom, eine Methyl- oder Phenylgruppe bedeuten, deren optisch aktive Antipoden, sofern sie ein asymmetrisches Kohlenstoffatom enthalten, und deren Salze, insbesondere deren physiologisch verträgliche Säureadditionssalze mit anorganischen oder organischen Säuren oder Basen.

5. Neue Carbonsäurederivate der allgemeinen Formel I gemäß Anspruch 3, in der
R, $R_8$, m, n, X und Z wie im Anspruch 4 definiert sind,
$R_1$ in 5-Position eine Methylgruppe, ein Fluor- oder Chloratom,
$R_2$ und $R_3$ zusammen mit dem dazwischenliegenden Stickstoffatom eine 2-Aza-bicyclo/3.3.1/nonan-2-yl- oder Octahydro-1H-azoninogruppe darstellt, deren optisch aktive Antipoden, sofern sie ein asymmetrisches Kohlenstoffatom enthalten, und deren physiologisch verträgliche Additionssalze mit anorganischen oder organischen Säuren und auch Basen, wenn Z die Carboxygruppe darstellt.

6. 4-/2-/2-(2-Azabicyclo/3.3.1/nonan-2-yl)-5-chlor-benzoylamino/äthyl/benzoesäure, deren Alkylester mit 1 bis 3 Kohlenstoffatom und deren Additionssalze.

7. 4-/2-/5-Fluor-2-(octahydro-1H-azonino)-benzoylamino/äthyl/-benzoesäure, deren Alkylester mit 1 bis 3 Kohlenstoffatomen und deren Additionssalze.

8. 4-/2-/5-Methyl-2-(octahydro-1H-azonino)-benzoylamino/äthyl/-benzoesäure, deren Alkylester mit 1 bis 3 Kohlenstoffatomen und deren Additionssalze.

9. Arzneimittel, enthaltend eine Verbindung gemäß den Ansprüchen 1 bis 8 neben einem oder mehreren inerten Trägerstoffen und/oder Verdünnungsmitteln.

10. Verfahren zur Herstellung von neuen Carbonsäureamiden der allgemeinen Formel

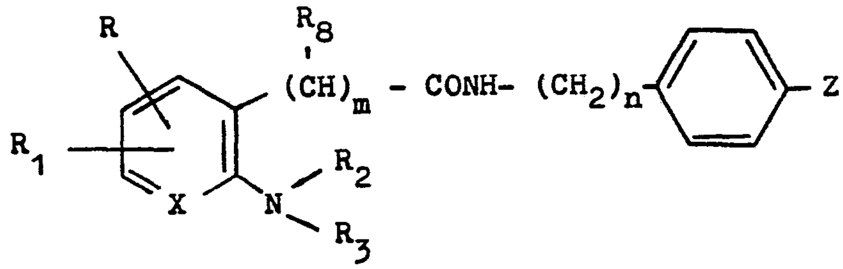

,(I)

in der

m die Zahl 0 oder 1,

n die Zahl 1 oder 2,

R ein Wasserstoffatom oder eine Alkylgruppe mit 1 bis 3 Kohlenstoffatomen,

$R_1$ ein Wasserstoff-, Fluor-, Chlor-, Brom- oder Jodatom, eine Alkyl- oder Alkoxygruppe mit jeweils 1 bis 4 Kohlenstoffatomen, eine Cyano-, Hydroxy- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_2$ und $R_3$ zusammen mit dem dazwischen liegenden Stickstoffatom eine Piperidino-, 3,5-Dimethyl-piperidino-, Octahydro-1H-azonino-, Decahydro-azecino-, 1,3-Dihydro-isoindolo-, Hexahydro-isoindolo- oder Octahydro-isoindologruppe, eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, der durch eine oder mehrere Alkylgruppen mit jeweils 1 bis 3 Kohlenstoffatomen substituiert sein kann, oder eine gegebenenfalls durch ein Halogenatom, eine Alkoxygruppe mit 1 bis 3 Kohlenstoffatomen oder eine Aminogruppe substituierte 1,3-Dihydro-isoindologruppe oder auch, wenn m die Zahl 1 darstellt, die Pyrrolidino-, Hexamethylenimino- oder Heptamethyleniminogruppe,

X eine CH-Gruppe oder ein Stickstoffatom,

$R_8$ ein Wasserstoffatom, eine Alkylgruppe mit 1 bis 3 Kohlenstoffatomen oder eine Arylgruppe und

Z eine gegebenenfalls veresterte Carboxygruppe bedeuten

sowie von deren optisch aktiven Antipoden, sofern sie ein asymmetrisches Kohlenstoffatom enthalten, und von deren Additionssalzen, insbesondere von deren physiologisch verträglichen Salzen mit anorganischen oder organischen Säuren und auch Basen, wenn Z eine Carboxygruppe darstellt, dadurch gekennzeichnet, daß

a) eine Carbonsäure der allgemeinen Formel

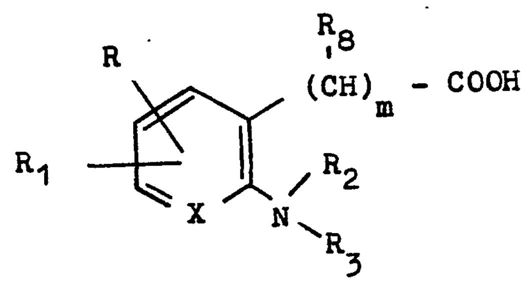

,(XI)

in der
R, $R_1$, $R_2$, $R_3$, $R_8$, X und m wie eingangs definiert sind, oder deren gegbenenfalls im Reaktionsgemisch hergestelltes reaktionsfähiges Derivat mit einem Amin der allgemeinen Formel

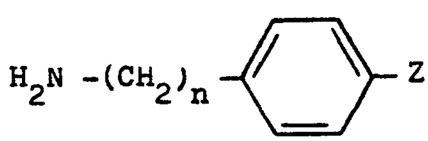

,(XII)

in der
Z und n wie eingangs definiert sind, oder mit einem gegebenenfalls im Reaktionsgemisch gebildeten N-aktivierten Amin der allgemeinen Formel XII, wenn eine Carbonsäure der allgemeinen Formel XI eingesetzt wird und wenn Z in einem N-aktivierten Amin der allgemeinen Formel XII keine Carboxygruppe darstellt, umgesetzt wird oder

b) zur Herstellung von Verbindungen der allgemeinen Formel I, in der
X eine CH-Gruppe und m die Zahl 0 darstellt, eine Verbindung der allgemeinen Formel

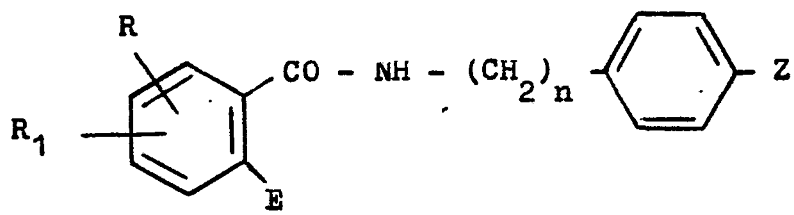 ,(XIII)

in der
R, $R_1$, Z und n wie eingangs definiert sind und
E einen mustauschbaren Rest wie ein Halogenatom darstellt, mit einem Amin der allgemeinen Formel

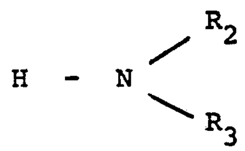 ,(XIV)

in der
$R_2$ und $R_3$ wie eingangs definiert sind, umgesetzt wird oder

c) zur Herstellung von Verbindungen der allgemeinen Formel I, in der Z eine Carboxygruppe darstellt, eine Verbindung der allgemeinen Formel

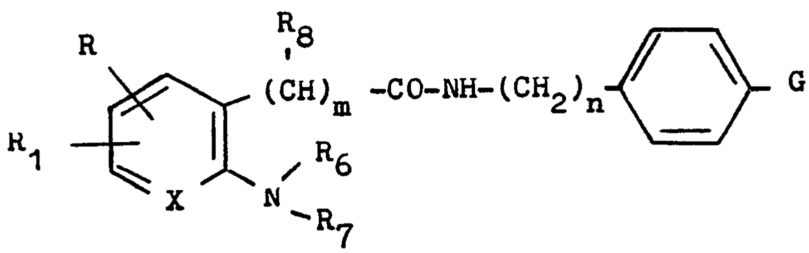

,(XV)

in der
R, $R_1$ bis $R_3$, $R_8$, X, m und n wie eingangs definiert sind und G eine durch Oxidation in eine Carboxygruppe überführbare Gruppe bedeutet, oxydiert wird oder

d) zur Herstellung von Verbindungen der allgemeinen Formel I, in der Z eine Carboxygruppe darstellt, eine Verbindung der allgemeinen Formel

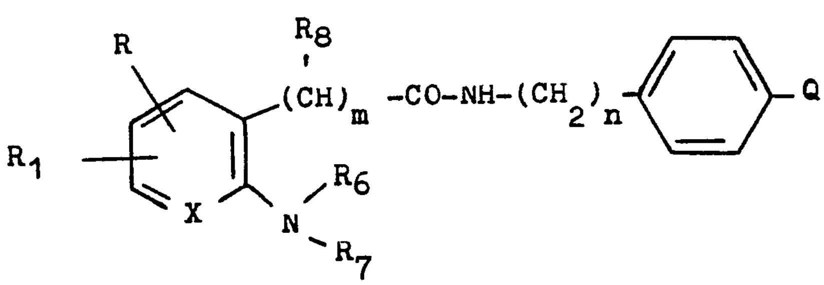

,(XVI)

in der
R, $R_1$ bis $R_3$, $R_8$, X, m und n wie eingangs definiert sind und Q eine durch Hydrolyse in eine Carboxygruppe überführbare Gruppe darstellt, hydrolysiert wird oder

e) zur Herstellung von Verbindungen der allgemeinen Formel I, in der X eine CH-Gruppe und m die Zahl 0 darstellt, ein Amid der allgemeinen Formel

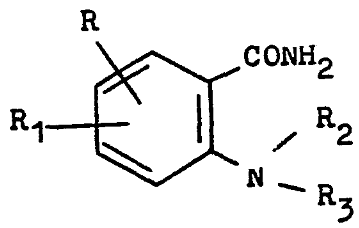

,(XVII)

in der
R und $R_1$ bis $R_3$ wie eingangs definiert sind, oder dessen Alkalisalz mit einer Verbindung der allgemeinen Formel

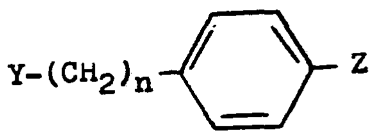

,(XVIII)

in der
Z und n wie eingangs definiert sind und
Y eine nukleophile Austrittsgruppe wie ein Halogenatom oder eine Sulfonyloxygruppe darstellt, umgesetzt wird oder

f) zur Herstellung von Verbindungen der allgemeinen Formel I, in der
X eine CH-Gruppe und
Z eine Carboxygruppe bedeuten, eine Verbindung der allgemeinen Formel

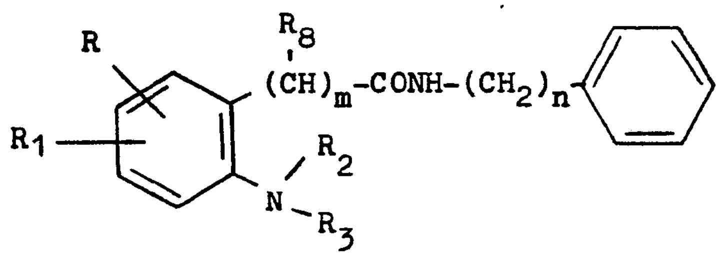

,(XIX)

in der
R, $R_1$ bis $R_3$, $R_8$, m und n wie eingangs definiert sind, mit einem Oxalylhalogenid oder Phosgen in Gegenwart einer Lewis-Säure acyliert wird oder

g) zur Herstellung von Verbindungen der allgemeinen Formel I, in der
X eine CH-Gruppe und
Z eine Carboxylgruppe bedeuten, eine Verbindung der allgemeinen Formel

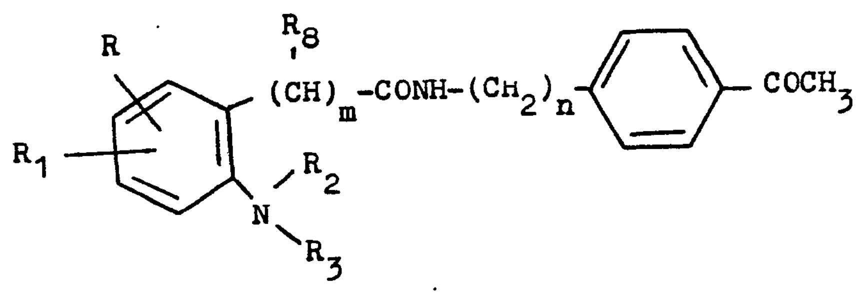

,(XX)

in der
R, $R_1$ bis $R_3$, $R_8$, m und n wie eingangs definiert sind, mit einem gegebenenfalls im Reaktionsgemisch hergestellten Hypohalogenit umgesetzt wird

und gewünschtenfalls anschließend eine so erhaltene Verbindung der allgemeinen Formel I, in der Z eine Carboxygruppe darstellt, mittels Veresterung in eine entsprechende Verbindung der allgemeinen Formel I, in der Z eine veresterte Carboxygruppe darstellt, übergeführt wird und/oder

eine erhaltene Verbindung der allgemeinen Formel I in ihre Additionssalze, insbesondere in ihre physiologisch verträglichen Salze mit anorganischen oder organischen Säuren und auch Basen, wenn Z eine Carboxygruppe darstellt, übergeführt wird.

11. Neue 2-Amino-carbonsäure-Derivate der allgemeinen Formel

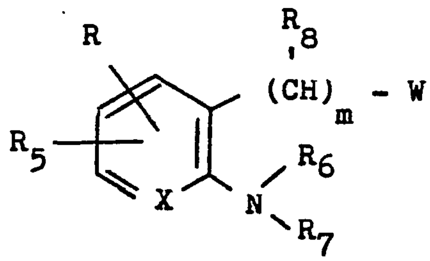

,(Ia)

in der

m die Zahl 0 oder 1,

W eine gegebenenfalls veresterte Carboxygruppe,

X ein Stickstoffatom oder die CH-Gruppe,

R ein Wasserstoffatom oder eine Alkylgruppe mit 1 bis 3 Kohlenstoffatomen,

$R_5$ ein Wasserstoff- oder Halogenatom, eine Amino-, Cyano-, Hydroxy-, Carboxy- oder Acetaminogruppe, eine Alkyl- oder Alkoxygruppe mit jeweils 1 bis 4 Kohlenstoffatomen,

$R_6$ und $R_7$ zusammen mit dem dazwischenliegenden Stickstoffatom eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, welche durch eine oder mehrere Alkylgruppen mit 1 bis 3 Kohlenstoffatomen substituiert sein kann, eine Octahydro-1H-azonino-, 1,3-Dihydro-isoindolo-, Hexahydro-isoindolo- oder Octahydro-isoindologruppe oder eine durch ein Halogenatom , eine Alkoxygruppe mit 1 bis 3 Kohlenstoffatomen oder Aminogruppe substituierte 1,3-Dihydro-isoindologruppe und

$R_8$ ein Wasserstoffatom, eine Alkylgruppe mit 1 bis 3 Kohlenstoffatomen oder eine Arylgruppe bedeuten, deren optisch aktive Antipoden, sofern sie ein asymmetrisches Kohlenstoffatom, enthalten, und deren Salze, insbesondere deren physiologisch verträgliche Additionssalze mit anorganischen oder organischen Säuren und Basen.

12. Neue 2-Amino-carbonsäurederivate der allgemeinen Formel Ia gemäß Anspruch 11, in der
m die Zahl 0,
R ein Wasserstoffatom,
X eine CH-Gruppe,
$R_5$ eine Aminogruppe, ein Fluor-, Chlor- oder Bromatom,
$R_6$ und $R_7$ zusammen mit dem dazwischenliegenden Stickstoffatom eine Octahydro-isoindologruppe, eine in 4-Stellung durch eine Alkylgruppe mit 5 bis 9 Kohlenstoffatomen substituierte Piperidinogruppe oder eine Azabicycloalkylgruppe mit 7 bis 12 Kohlenstoffatomen im Bicycloalkanring, die durch eine oder zwei Methylgruppen substituiert sein kann, und
W eine Carboxy- oder Alkoxycarbonylgruppe, in der die Alkoxygruppe 1 bis 3 Kohlenstoffatomen enthalten kann, bedeuten, und deren Additionssalze mit anorganischen oder organischen Säuren und auch Basen, wenn W die Carboxygruppe darstellt.

13. Neue 2-Amino-carbonsäurederivate der allgemeinen Formel Ia gemäß Anspruch 11, in der
m und X wie im Anspruch 11 definiert sind,
R ein Wasserstoffatom oder eine Methylgruppe,
$R_5$ ein Wasserstoff-, Fluor-, Chlor-, Brom- oder Jodatom, eine Alkylgruppe mit 1 bis 4 Kohlenstoffatomen, eine Hydroxy-, Methoxy-, Amino-, Cyano-, Carboxy- oder Acetaminogruppe,
$R_6$ und $R_7$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch ein Chlor- oder Bromatom, eine Methoxy- oder eine Aminogruppe substituierte 1,3-Dihydroisoindologruppe, eine Hexahydro-isoindolo- oder 2-Azabicyclononan-2-ylgruppe oder auch eine Octahydro-isoindologruppe, wenn m die Zahl 1 oder

X ein Stickstoffatom oder
m die Zahl O,
$R_5$ ein Wasserstoff-, Fluor-, Brom- oder Jodatom, eine Alkylgruppe mit 1 bis 4 Kohlenstoffatomen, eine Hydroxy-, Methoxy-, Cyano-, Carboxy- oder Acetaminogruppe und
X eine CH-Gruppe darstellen,
$R_8$ ein Wasserstoffatom, eine Methyl- oder Phenylgruppe und
W eine Carboxy- oder eine Alkoxycarbonylgruppe mit insgesamt 2 bis 4 Kohlenstoffatomen bedeuten, deren optisch aktive Antipoden, sofern sie ein asymmetrisches Kohlenstoffatom enthalten, und deren Salze, insbesondere deren physiologisch verträgliche Säureadditionssalze mit anorganischen oder organischen Säuren oder Basen.

14. Neue 2-Amino-carbonsäurederivate der allgemeinen Formel Ia gemäß Anspruch 11, in der
m die Zahl O oder 1,
X eine CH-Gruppe,
R ein Wasserstoffatom,
$R_5$ in 5-Position ein Wasserstoff-, Chlor- oder Bromatom,
$R_6$ und $R_7$ zusammen mit dem dazwischenliegenden Stickstoffatom eine in 4-Stellung durch eine Alkylgruppe mit 5 bis 9 Kohlenstoffatomen substituierte Piperidinogruppe, eine gegebenenfalls durch ein Chlor- oder Bromatom substituierte 1,3-Dihydro-isoindologruppe, eine 2-Aza-bicyclononan-3-yl- oder Hexahydro-isoindologruppe oder

eine Octahydro-isoindologruppe, wenn
m die Zahl 1 oder
m die Zahl O und
$R_5$ ein Bromatom darstellen,
$R_8$ ein Wasserstoffatom, eine Methyl- oder Phenylgruppe und

W die Carboxygruppe bedeuten, deren optisch aktive Antipoden, sofern sie ein asymmetrisches Kohlenstoffatom enthalten, und deren Salze, insbesondere deren physiologisch verträgliche Additionssalze mit anorganischen oder organischen Säuren und Basen.

15. 5-Chlor-2-[4-(3-pentyl)-piperidino]-benzoesäure und deren Additionssalze.

16. 5-Chlor-2-(octahydro-isoindol-2-yl)-nicotinsäure und deren Additionssalze.

17. 5-Brom-2-(octahydro-isoindolo-2-yl)-benzoesäure und deren Additionssalze.

18. 5-Chlor-2-(2,3,3a,4,7,7a-hexahydro-1H-isoindolo-2-yl)-benzoesäure und deren Additionssalze.

19. Arzneimittel, enthaltend eine Verbindung gemäß den Ansprüchen 11 bis 18 neben einem oder mehreren inerten Trägerstoffen und/oder Verdünnungsmitteln.

20. Verwendung einer Verbindung der allgemeinen Formel I gemäß den Ansprüchen 1 bis 8 bzw. der allgemeinen Formel Ia gemäß den Ansprüchen 11 bis 18 zur Herstellugn eines Arzneimittels auf nichtchemischem Wege zur Bekämpfung von Intermediärstoffwechselerkrankungen.

21. Verfahren zur Herstellung von neuen 2-Amino-carbonsäure-Derivaten der allgemeinen Formel

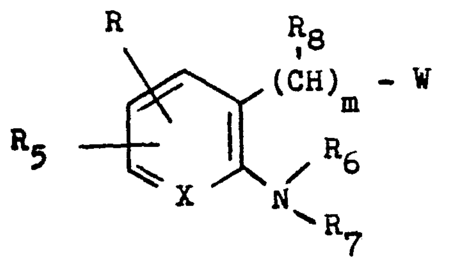

,(Ia)

in der

m die Zahl 0 oder 1,

W eine gegebenenfalls veresterte Carboxygruppe,

X ein Stickstoffatom oder die CH-Gruppe,

R ein Wasserstoffatom oder eine Alkylgruppe mit 1 bis 3 Kohlenstoffatomen,

$R_5$ ein Wasserstoff- oder Halogenatom, eine Amino-, Cyano-, Hydroxy-, Carboxy- oder Acetaminogruppe, eine Alkyl- oder Alkoxygruppe mit jeweils 1 bis 4 Kohlenstoffatomen,

$R_6$ und $R_7$ zusammen mit dem dazwischenliegenden Stickstoffatom eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, welche durch eine oder mehrere Alkylgruppen mit 1 bis 3 Kohlenstoffatomen substituiert sein kann, eine Octahydro-1H-azonino-, 1,3-Dihydro-isoindolo-, Hexahydro-isoindolo- oder Octahydro-isoindologruppe oder eine durch ein Halogenatom , eine Alkoxygruppe mit 1 bis 3 Kohlenstoffatomen oder Aminogruppe substituierte 1,3-Dihydro-isoindologruppe und

$R_8$ ein Wasserstoffatom, eine Alkylgruppe mit 1 bis 3 Kohlenstoffatomen oder eine Arylgruppe bedeuten, sowie von deren optisch aktiven Antipoden, sofern sie ein asymmetrisches Kohlenstoffatom enthalten, und von deren Additionssalzen mit anorganischen oder organischen Säuren oder auch Basen, wenn W die Carboxygruppe darstellt, dadurch gekennzeichnet, daß

a) zur Herstellung einer Verbindung der allgemeinen Formel Ia, in der W die Carboxygruppe darstellt, eine Verbindung der allgemeinen Formel

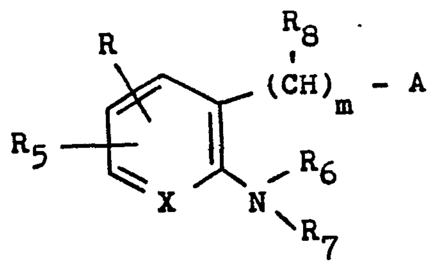

,(II)

in der
R, $R_5$ bis $R_8$, m und X wie eingangs definiert sind und
A eine durch Hydrolyse in eine Carboxygruppe überführbare Gruppe darstellt, hydrolysiert wird oder

b) zur Herstellung einer Verbindung der allgemeinen Formel Ia, in der $R_5$ eine Aminogruppe darstellt, eine Nitroverbindung der allgemeinen Formel

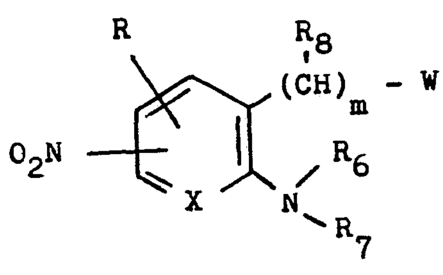

,(III)

in der
R, $R_6$ bis $R_8$, m, W und X wie eingangs definiert sind, reduziert wird oder

c) zur Herstellung von Verbindungen der allgemeinen Formel Ia, in der $R_5$ eine Hydroxygruppe, eine Cyangruppe, ein Wasserstoff-, Fluor-, Chlor- oder Bromatom darstellt, eine Verbindung der allgemeinen Formel

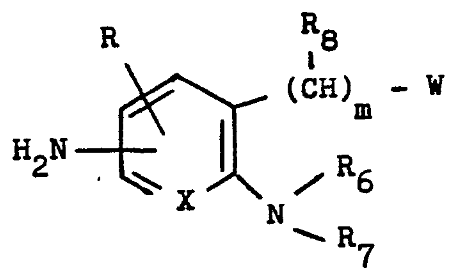

,(IV)

in der
R, $R_6$ bis $R_8$, m, W und X wie eingangs definiert sind, mit einem Nitrit in ein Diazoniumsalz übergeführt wird und das so erhaltene Diazoniumsalz anschließend in Gegenwart von Schwefelsäure, in Gegenwart von Kupfer oder in Gegenwart eines entsprechenden Kupfer-(I)-Salzes durch Erwärmen in die entsprechende Verbindung übergeführt wird oder

d) zur Herstellung einer Verbindung der allgemeinen Formel Ia, in der $R_5$ ein Wasserstoffatom darstellt, eine Verbindung der allgemeinen Formel

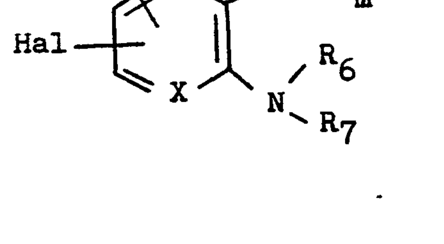

,(V)

in der
R, $R_6$ bis $R_8$, m, X und W wie eingangs definiert sind und
Hal ein Halogenatom darstellt, enthalogeniert wird oder

e) zur Herstellung einer Verbindung der allgemeinen Formel Ia, in der W die Carboxygruppe darstellt, eine Verbindung der allgemeinen Formel

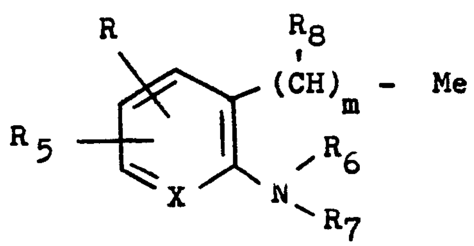

,(VI)

in der
R, $R_5$ bis $R_8$, m und X wie eingangs definiert sind und
Me ein Alkaliatom oder einen Erdalkaliatomhalogenidrest bedeutet, mit Kohlendioxid umgesetzt wird oder

f) zur Herstellung einer Verbindung der allgemeinen Formel Ia, in der X ein Stickstoffatom darstellt, eine Verbindung der allgemeinen Formel

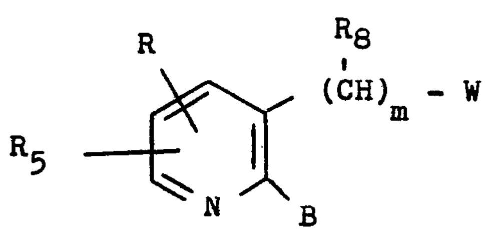

(VII)

in der
R, $R_5$ bis $R_8$, W und m wie eingangs definiert sind
und
B einen austauschbaren Rest darstellt, mit einem Amin der allgemeinen Formel

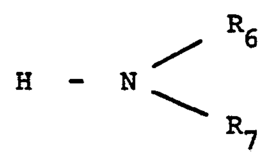
,(VIII)

in der
$R_6$ und $R_7$ wie eingangs definiert sind, umgesetzt wird oder

g) zur Herstellung einer Verbindung der allgemeinen Formel Ia, in der $R_5$ eine Alkoxygruppe mit 1 bis 4 Kohlenstoffatomen darstellt, eine Verbindung der allgemeinen Formel

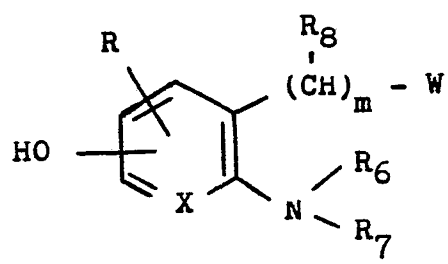
,(IX)

in der
R, $R_6$ bis $R_8$, X, W und m wie eingangs definiert sind, mit einer Verbindung der allgemeinen Formel

$$D - R_5' \quad ,(X)$$

in der

$R_5$' eine Alkylgruppe mit 1 bis 4 Kohlenstoffatomen

und

D eine nukleophile Austrittsgruppe oder zusammen mit dem benachbarten H-Atom des Restes $R_5$' eine Diazogruppe bedeuten, umgesetzt und erforderlichenfalls anschließend hydrolysiert wird

und gewünschtenfalls anschließend eine so erhaltene Verbindung der allgemeinen Formel Ia, in der W eine veresterte Carboxygruppe darstellt, mittels Hydrolyse in eine entsprechende Verbindung der allgemeinen Formel Ia, in der W die Carboxygruppe darstellt, übergeführt wird

und/oder eine erhaltene Verbindung der allgemeinen Formel Ia in ihre Additionssalze mit anorganischen oder organischen Säuren und auch Basen, wenn W die Carboxygruppe darstellt, übergeführt wird.

0056144

Patentansprüche (Benennungsland: Österreich)

1. Verfahren zur Herstellung von neuen Carbonsäureamiden der allgemeinen Formel

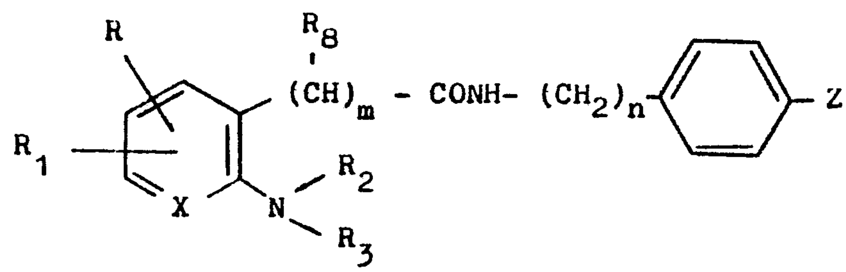

,(I)

in der

m die Zahl 0 oder 1,

n die Zahl 1 oder 2,

R ein Wasserstoffatom oder eine Alkylgruppe mit 1 bis 3 Kohlenstoffatomen,

$R_1$ ein Wasserstoff-, Fluor-, Chlor-, Brom- oder Jodatom, eine Alkyl- oder Alkoxygruppe mit jeweils 1 bis 4 Kohlenstoffatomen, eine Cyano-, Hydroxy- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_2$ und $R_3$ zusammen mit dem dazwischenliegenden Stickstoffatom eine Piperidino-, 3,5-Dimethyl-piperidino-, Octahydro-1H-azonino-, Decahydro-azecino-, 1,3-Dihydro-isoindolo-, Hexahydro-isoindolo- oder Octahydro-isoindologruppe, eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, der durch eine oder mehrere Alkylgruppen mit jeweils 1 bis 3 Kohlenstoffatomen substituiert sein

kann, oder eine gegebenenfalls durch ein Halogenatom, eine Alkoxygruppe mit 1 bis 3 Kohlenstoffatomen oder eine Aminogruppe substituierte 1,3-Dihydro-isoindologruppe oder auch, wenn m die Zahl 1 darstellt, die Pyrrolidino-, Hexamethylenimino- oder Heptamethyleniminogruppe,

X eine CH-Gruppe oder ein Stickstoffatom,

$R_8$ ein Wasserstoffatom, eine Alkylgruppe mit 1 bis 3 Kohlenstoffatomen oder eine Arylgruppe und

Z eine gegebenenfalls veresterte Carboxygruppe bedeuten

sowie von deren optisch aktiven Antipoden, sofern sie ein asymmetrisches Kohlenstoffatom enthalten, und von deren Additionssalzen, insbesondere von deren physiologisch verträglichen Salzen mit anorganischen oder organischen Säuren und auch Basen, wenn Z eine Carboxygruppe darstellt, dadurch gekennzeichnet, daß

a) eine Carbonsäure der allgemeinen Formel

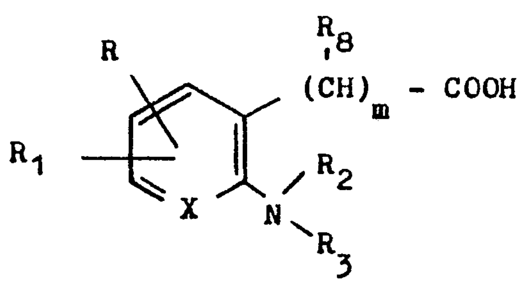

,(XI)

in der
R, $R_1$, $R_2$, $R_3$, $R_8$, X und m wie eingangs definiert sind, oder deren gegbenenfalls im Reaktionsgemisch hergestelltes reaktionsfähiges Derivat mit einem Amin der allgemeinen Formel

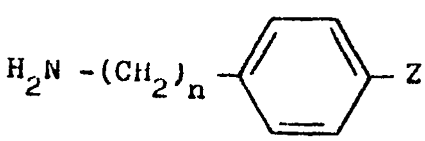

,(XII)

in der
Z und n wie eingangs definiert sind, oder mit einem gegebenenfalls im Reaktionsgemisch gebildeten N-aktivierten Amin der allgemeinen Formel XII, wenn eine Carbonsäure der allgemeinen Formel XI eingesetzt wird und wenn Z in einem N-aktivierten Amin der allgemeinen Formel XII keine Carboxygruppe darstellt, umgesetzt wird oder

b) zur Herstellung von Verbindungen der allgemeinen Formel I, in der
X eine CH-Gruppe und m die Zahl O darstellt, eine Verbindung der allgemeinen Formel

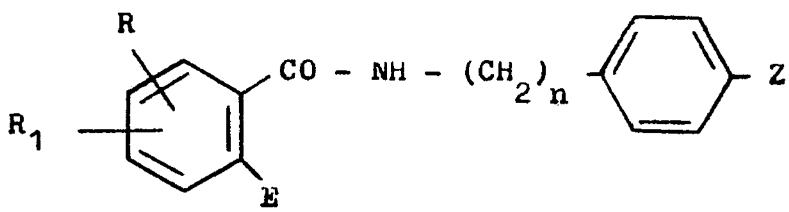 ,(XIII)

in der
R, $R_1$, Z und n wie eingangs definiert sind und
E einen austauschbaren Rest wie ein Halogenatom darstellt, mit einem Amin der allgemeinen Formel

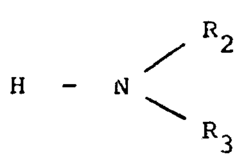 ,(XIV)

in der
$R_2$ und $R_3$ wie eingangs definiert sind, umgesetzt wird oder

c) zur Herstellung von Verbindungen der allgemeinen Formel I, in der Z eine Carboxygruppe darstellt, eine Verbindung der allgemeinen Formel

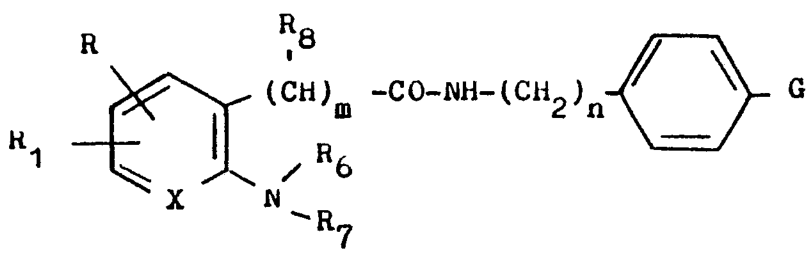

,(XV)

in der

R, $R_1$ bis $R_3$, $R_8$, X, m und n wie eingangs definiert sind und G eine durch Oxidation in eine Carboxygruppe überführbare Gruppe bedeutet, oxydiert wird oder

d) zur Herstellung von Verbindungen der allgemeinen Formel I, in der Z eine Carboxygruppe darstellt, eine Verbindung der allgemeinen Formel

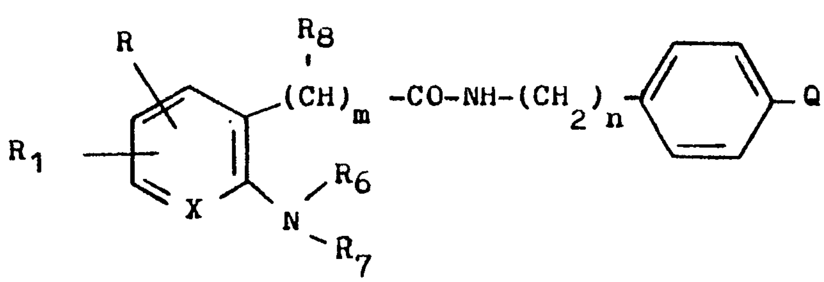

,(XVI)

in der

R, $R_1$ bis $R_3$, $R_8$, X, m und n wie eingangs definiert sind und Q eine durch Hydrolyse in eine Carboxygruppe überführbare Gruppe darstellt, hydrolysiert wird

oder

e) zur Herstellung von Verbindungen der allgemeinen Formel I, in der X eine CH-Gruppe und m die Zahl 0 darstellt, ein Amid der allgemeinen Formel

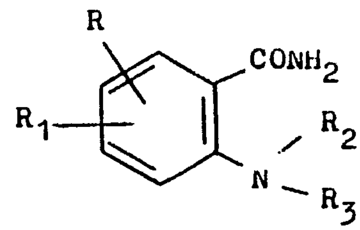
,(XVII)

in der
R und $R_1$ bis $R_3$ wie eingangs definiert sind, oder dessen Alkalisalz mit einer Verbindung der allgemeinen Formel

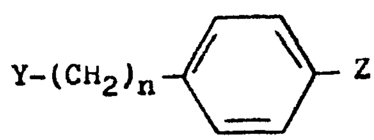
,(XVIII)

in der
Z und n wie eingangs definiert sind und
Y eine nukleophile Austrittsgruppe wie ein Halogenatom oder eine Sulfonyloxygruppe darstellt, umgesetzt wird oder

f) zur Herstellung von Verbindungen der allgemeinen Formel I, in der
X eine CH-Gruppe und
Z eine Carboxygruppe bedeuten, eine Verbindung der allgemeinen Formel

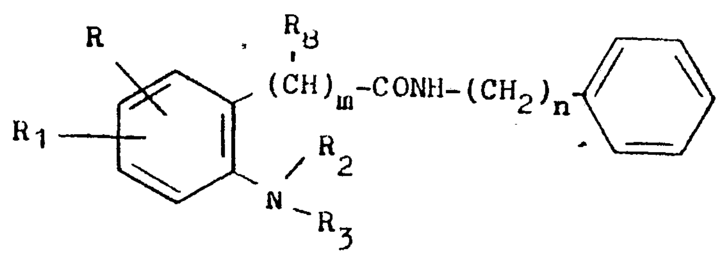
,(XIX)

in der
R, $R_1$ bis $R_3$, $R_8$, m und n wie eingangs definiert sind, mit einem Oxalylhalogenid oder Phosgen in Gegenwart einer Lewis-Säure acyliert wird oder

g) zur Herstellung von Verbindungen der allgemeinen Formel I, in der
X eine CH-Gruppe und
Z eine Carboxylgruppe bedeuten, eine Verbindung der allgemeinen Formel

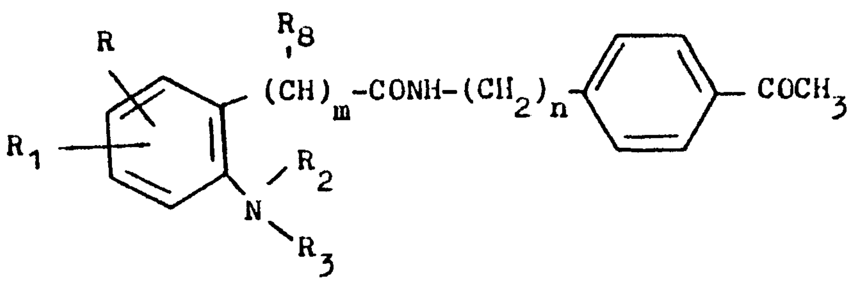

,(XX)

in der
R, $R_1$ bis $R_3$, $R_8$, m und n wie eingangs definiert sind, mit einem gegebenenfalls im Reaktionsgemisch hergestellten Hypohalogenit umgesetzt wird

und gewünschtenfalls anschließend eine so erhaltene Verbindung der allgemeinen Formel I, in der Z eine Carboxygruppe darstellt, mittels Veresterung in eine entsprechende Verbindung der allgemeinen Formel I, in der Z eine veresterte Carboxygruppe darstellt, übergeführt wird und/oder

eine erhaltene Verbindung der allgemeinen Formel I in ihre Additionssalze, insbesondere in ihre physiologisch verträglichen Salze mit anorganischen oder organischen Säuren und auch Basen, wenn Z eine Carboxygruppe darstellt, übergeführt wird.

2. Verfahren nach Anspruch 1 zur Herstellung von neuen Carbonsäureamiden der allgemeinen Formel

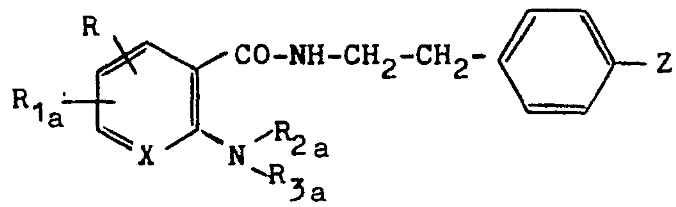

,(I)

in der

R, X und Z wie im Anspruch 1 definiert sind,

$R_{1a}$ ein Fluor-, Chlor-, Brom- oder Jodatom, eine Alkyl- oder Alkoxygruppe mit jeweils 1 bis 4 Kohlenstoffatomen, eine Cyano- oder Trifluormethylgruppe oder eine gegebenenfalls durch eine Alkylgruppe mit 1 bis 3 Kohlenstoffatomen, ein Fluor-, Chlor- oder Bromatom substituierte Phenylgruppe,

$R_{2a}$ und $R_{3a}$ zusammen mit dem dazwischenliegenden Stickstoffatom eine Piperidino-, 3,5-Dimethyl-piperidino-, Octahydro-1H-azonino- oder Decahydro-azecinogruppe, eine durch eine Alkylgruppe mit 5 bis 10 Kohlenstoffatomen substituierte Piperidinogruppe oder eine Azabicycloalkylgruppe mit 7 bis 12 Kohlenstoffatomen im Bicycloalkanring, welche durch eine oder mehrere Alkylgruppen mit jeweils 1 bis 3 Kohlenstoffatomen substituiert sein kann, oder eine Octahydro-isoindologruppe bedeuten, sowie von deren Additionssalze, insbesondere von deren physiologisch verträglichen Salze mit anorganischen oder organischen Säuren und auch Basen, wenn Z eine Carboxygruppe darstellt, dadurch gekennzeichnet, daß

a) eine Carbonsäure der allgemeinen Formel

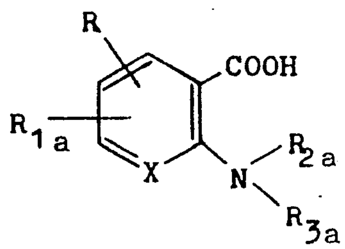

,(XIa)

in der
R und X wie im Anspruch 1 und
$R_{1a}$, $R_{2a}$ und $R_{3a}$ wie eingangs definiert sind, oder deren gegebenenfalls im Reaktionsgemisch hergestelltes reaktionsfähiges Derivat mit einem Amin der allgemeinen Formel

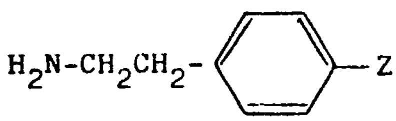

,(XIIa)

in der
Z wie im Anspruch 1 definiert ist, oder mit einem gegebenenfalls im Reaktionsgemisch gebildeten N-aktivierten Amin der allgemeinen Formel XIIa, wenn eine Carbonsäure der allgemeinen Formel XIa eingesetzt wird und wenn Z in einem N-aktivierten Amin der allgemeinen Formel XIIa keine Carboxygruppe darstellt, umgesetzt wird oder

b) zur Herstellung von Verbindungen der allgemeinen Formel I, in der
X eine CH-Gruppe darstellt, eine Verbindung der allgemeinen Formel

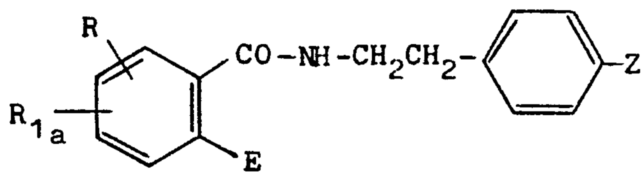

, (XIIIa)

in der
R, E und Z wie im Anspruch 1 und $R_{1a}$ wie eingangs definiert ist, mit einem Amin der allgemeinen Formel

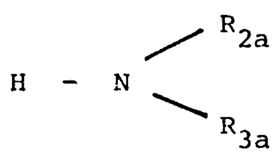

, (XIVa)

in der
$R_{2a}$ und $R_{3a}$ wie eingangs definiert sind, umgesetzt wird oder

c) zur Herstellung von Verbindungen der allgemeinen Formel I, in der Z eine Carboxygruppe darstellt, eine Verbindung der allgemeinen Formel

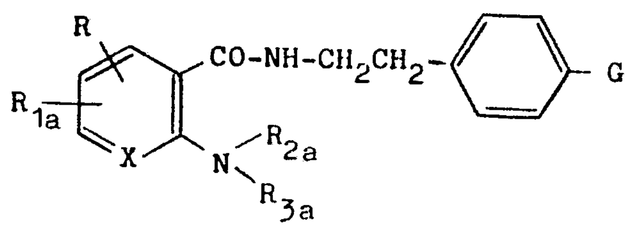

, (XVa)

in der
R, G und X wie im Anspruch 1 und $R_{1a}$ bis $R_{3a}$ wie eingangs definiert sind, oxydiert wird oder

d) zur Herstellung von Verbindungen der allgemeinen Formel I, in der Z eine Carboxygruppe darstellt, eine Verbindung der allgemeinen Formel

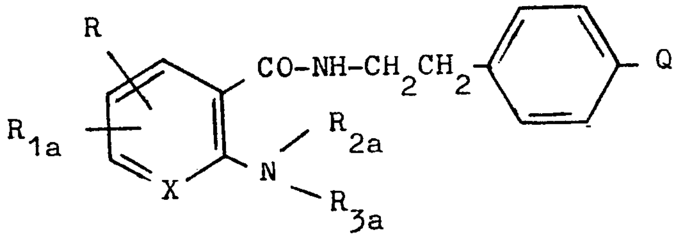

,(XVIa)

in der
R, Q und X wie im Anspruch 1 und $R_{1a}$ bis $R_{3a}$ wie eingangs definiert sind, hydrolysiert wird oder

e) zur Herstellung von Verbindungen der allgemeinen Formel I, in der X eine CH-Gruppe darstellt, ein Amid der allgemeinen Formel

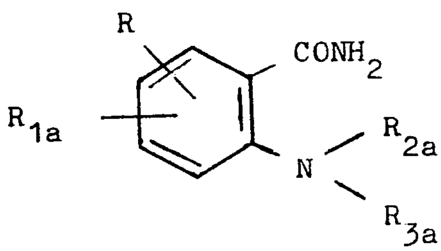

,(XVIIa)

in der
R wie im Anspruch 1 und $R_{1a}$ bis $R_{3a}$ wie eingangs definiert sind, oder dessen Alkalisalz mit einer Verbindung der allgemeinen Formel

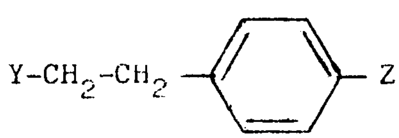

,(XVIIIa)

in der
Z und Y wie im Anspruch 1 definiert sind, umgesetzt wird oder

f) zur Herstellung von Verbindungen der allgemeinen Formel I, in der
X eine CH-Gruppe und
Z eine Carboxygruppe bedeuten, eine Verbindung der allgemeinen Formel

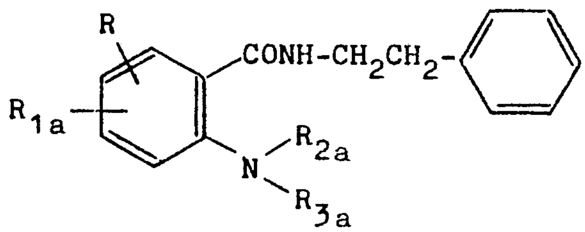

,(XIXa)

in der
R wie im Anspruch 1 und $R_{1a}$ bis $R_{3a}$ wie eingangs definiert sind, mit einem Oxalylhalogenid oder Phosgen in Gegenwart einer Lewis-Säure acyliert wird oder

g) zur Herstellung von Verbindungen der allgemeinen Formel I, in der
X eine CH-Gruppe und
Z eine Carboxygruppe bedeuten, eine Verbindung der allgemeinen Formel

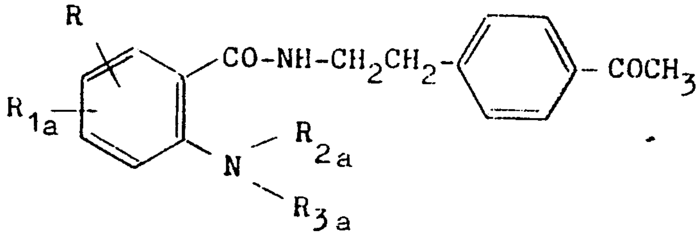

,(XXa)

in der
R wie im Anspruch 1 definiert ist und $R_{1a}$ bis $R_{3a}$ wie eingangs definiert sind, mit einem gegebenenfalls im Reaktionsgemisch hergestellten Hypohalogenit umgesetzt wird

und gewünschtenfalls anschließend eine so erhaltene Verbindung der allgemeinen Formel I, in der Z eine Carboxygruppe darstellt, mittels Veresterung in eine entsprechende Verbindung der allgemeinen Formel I, in der Z eine veresterte Carboxygruppe darstellt, übergeführt wird und/oder

eine erhaltene Verbindung der allgemeinen Formel I in ihre Additionssalze, insbesondere in ihre physiologisch verträglichen Salze mit anorganischen oder organischen Säuren und auch Basen, wenn Z eine Carboxygruppe darstellt, übergeführt wird.

Priorität: 10. Januar 1981 (P 31 00 535.7)

3. Verfahren zur Herstellung von neuen 2-Amino-carbonsäure-Derivaten der allgemeinen Formel

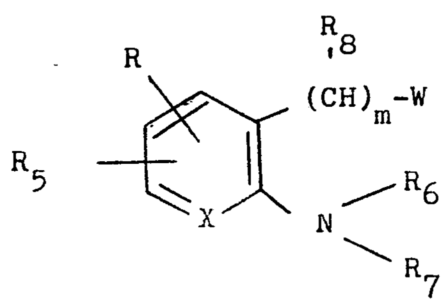
,(Ia)

in der
m die Zahl 0 oder 1,
W eine gegebenenfalls veresterte Carboxygruppe,
X ein Stickstoffatom oder die CH-Gruppe,
R ein Wasserstoffatom oder eine Alkylgruppe mit 1 bis 3 Kohlenstoffatomen,

$R_5$ ein Wasserstoff- oder Halogenatom, eine Amino-, Cyano-, Hydroxy-, Carboxy- oder Acetaminogruppe, eine Alkyl- oder Alkoxygruppe mit jeweils 1 bis 4 Kohlenstoffatomen,

$R_6$ und $R_7$ zusammen mit dem dazwischenliegenden Stickstoffatom eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, welche durch eine oder mehrere Alkylgruppen mit 1 bis 3 Kohlenstoffatomen substituiert sein kann, eine Octahydro-1H-azonino-, 1,3-Dihydro-isoindolo-, Hexahydro-isoindolo- oder Octahydro-isoindologruppe oder eine durch ein Halogenatom , eine Alkoxygruppe mit 1 bis 3 Kohlenstoffatomen oder Aminogruppe substituierte 1,3-Dihydro-isoindologruppe und

$R_8$ ein Wasserstoffatom, eine Alkylgruppe mit 1 bis 3 Kohlenstoffatomen oder eine Arylgruppe bedeuten, sowie von deren optisch aktiven Antipoden, sofern sie ein asymmetrisches Kohlenstoffatom enthalten, und von deren Additionssalzen mit anorganischen oder organischen Säuren oder auch Basen, wenn W die Carboxygruppe darstellt, dadurch gekennzeichnet, daß

a) zur Herstellung einer Verbindung der allgemeinen Formel Ia, in der W die Carboxygruppe darstellt, eine Verbindung der allgemeinen Formel

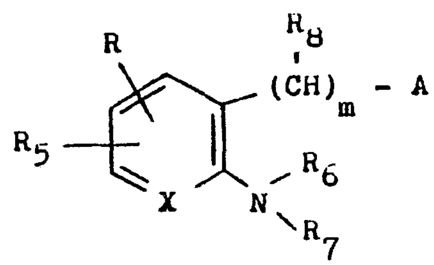

,(II)

in der

R, $R_5$ bis $R_8$, m und X wie eingangs definiert sind und

A eine durch Hydrolyse in eine Carboxygruppe überführbare Gruppe darstellt, hydrolysiert wird oder

b) zur Herstellung einer Verbindung der allgemeinen Formel Ia, in der $R_5$ eine Aminogruppe darstellt, eine Nitroverbindung der allgemeinen Formel

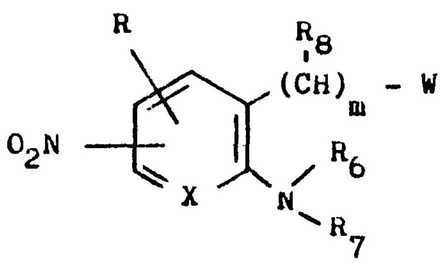

,(III)

in der
R, $R_6$ bis $R_8$, m, W und X wie eingangs definiert sind, reduziert wird oder

c) zur Herstellung von Verbindungen der allgemeinen Formel Ia, in der $R_5$ eine Hydroxygruppe, eine Cyangruppe, ein Wasserstoff-, Fluor-, Chlor- oder Bromatom darstellt, eine Verbindung der allgemeinen Formel

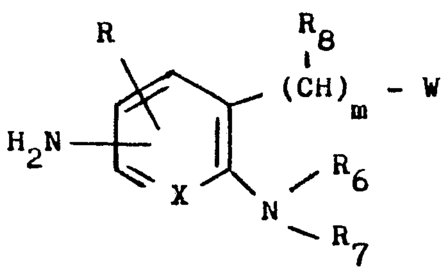

,(IV)

in der
R, $R_6$ bis $R_8$, m, W und X wie eingangs definiert sind, mit einem Nitrit in ein Diazoniumsalz übergeführt wird und das so erhaltene Diazoniumsalz anschließend in Gegenwart von Schwefelsäure, in Gegenwart von

Kupfer oder in Gegenwart eines entsprechenden Kupfer-(I)-Salzes durch Erwärmen in die entsprechende Verbindung übergeführt wird oder

d) zur Herstellung einer Verbindung der allgemeinen Formel Ia, in der $R_5$ ein Wasserstoffatom darstellt, eine Verbindung der allgemeinen Formel

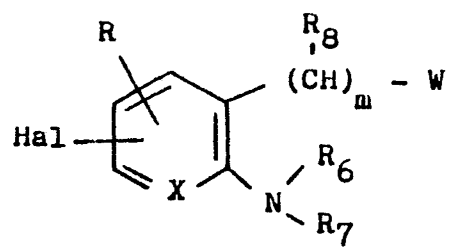

,(V)

in der
R, $R_6$ bis $R_8$, m, X und W wie eingangs definiert sind und
Hal ein Halogenatom darstellt, enthalogeniert wird oder

e) zur Herstellung einer Verbindung der allgemeinen Formel Ia, in der W die Carboxygruppe darstellt, eine Verbindung der allgemeinen Formel

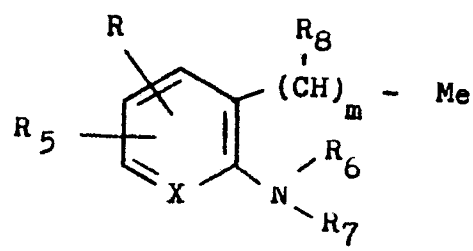

,(VI)

in der
R, $R_5$ bis $R_8$, m und X wie eingangs definiert sind und
Me ein Alkaliatom oder einen Erdalkaliatomhalogenidrest bedeutet, mit Kohlendioxid umgesetzt wird oder

f) zur Herstellung einer Verbindung der allgemeinen Formel Ia, in der X ein Stickstoffatom darstellt, eine Verbindung der allgemeinen Formel

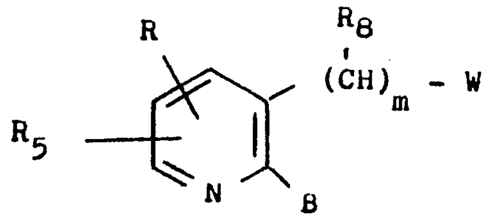

(VII)

in der
R, $R_5$ bis $R_8$, W und m wie eingangs definiert sind
und
B einen austauschbaren Rest darstellt, mit einem Amin der allgemeinen Formel

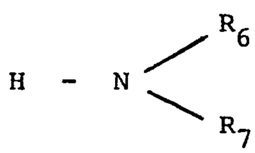

,(VIII)

in der
$R_6$ und $R_7$ wie eingangs definiert sind, umgesetzt wird oder

g) zur Herstellung einer Verbindung der allgemeinen Formel Ia, in der $R_5$ eine Alkoxygruppe mit 1 bis 4 Kohlenstoffatomen darstellt, eine Verbindung der allgemeinen Formel

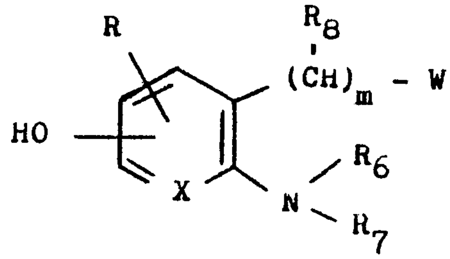

,(IX)

in der
R, $R_6$ bis $R_8$, X, W und m wie eingangs definiert sind, mit einer Verbindung der allgemeinen Formel

$$D - R_5' \quad ,(X)$$

in der
$R_5$' eine Alkylgruppe mit 1 bis 4 Kohlenstoffatomen und
D eine nukleophile Austrittsgruppe oder zusammen mit dem benachbarten H-Atom des Restes $R_5$' eine Diazogruppe bedeuten, umgesetzt und erforderlichenfalls anschließend hydrolysiert wird

und gewünschtenfalls anschließend eine so erhaltene Verbindung der allgemeinen Formel Ia, in der W eine veresterte Carboxygruppe darstellt, mittels Hydrolyse in eine entsprechende Verbindung der allgemeinen Formel Ia, in der W die Carboxygruppe darstellt, übergeführt wird
und/oder eine erhaltene Verbindung der allgemeinen Formel Ia in ihre Additionssalze mit anorganischen oder organischen Säuren und auch Basen, wenn W die Carboxygruppe darstellt, übergeführt wird.

4. Verfahren nach Anspruch 3 zur Herstellung von neuen 2-Aminobenzoesäure-Derivaten der allgemeinen Formel

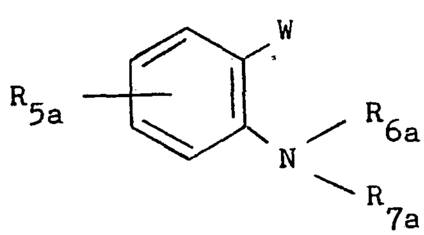

,(Ia)

in der
W wie im Anspruch 3 definiert ist,
$R_{5a}$ eine Aminogruppe, ein Fluor-, Chlor- oder Bromatom,
$R_{6a}$ und $R_{7a}$ zusammen mit dem dazwischenliegenden Stickstoffatom eine durch eine Alkylgruppe mit 5 bis 10 Kohlenstoffatomen substituierte Piperidinogruppe, eine Azabicycloalkylgruppe mit 7 bis 12 Kohlenstoffatomen im Bicycloalkanring, welche durch eine oder mehrere Alkylgruppen mit jeweils 1 bis 3 Kohlenstoffatomen substituiert sein kann, oder eine Octahydro-isoindologruppe bedeuten, sowie von deren Additionssalzen mit anorganischen oder organischen Säuren oder auch Basen, wenn W die Carboxygruppe darstellt, dadurch gekennzeichnet, daß

b) zur Herstellung von Verbindungen der allgemeinen Formel Ia, in der $R_{5a}$ eine Aminogruppe darstellt, eine Nitroverbindung der allgemeinen Formel

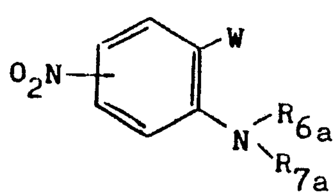

,(IIIa)

in der
W wie im Anspruch 3 und
$R_{6a}$ und $R_{7a}$ wie eingangs definiert sind, reduziert wird oder

c) zur Herstellung von Verbindungen der allgemeinen Formel Ia, in der $R_{5a}$ ein Fluor-, Chlor- oder Bromatom darstellt, eine Verbindung der allgemeinen Formel .

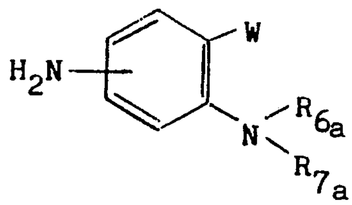

, (IVa)

in der

W wie im Anspruch 3 und

$R_{6a}$ und $R_{7a}$ wie eingangs definiert sind, mit einem Nitrit in ein Diazoniumsalz übergeführt wird und das so erhaltene Diazoniumsalz anschließend in Gegenwart von Schwefelsäure, in Gegenwart von Kupfer oder in Gegenwart eines entsprechenden Kupfer-(I)-Salzes durch Erwärmen in die entsprechende Verbindung übergeführt wird

und gewünschtenfalls anschließend eine so erhaltene Verbindung der allgemeinen Formel Ia, in der W eine veresterte Carboxygruppe darstellt, mittels Hydrolyse in eine entsprechende Verbindung der allgemeinen Formel Ia, in der W die Carboxygruppe darstelt, übergeführt wird

und/oder eine erhaltene Verbindung der allgemeinen Formel Ia in ihre Additionssalze mit anorganischen oder organischen Säuren und auch Basen, wenn W die Carboxygruppe darstellt, übergeführt wird.

Priorität: 10. Januar 1981 (P 31 00 535.7)

5. Verfahren gemäß den Ansprüchen 1 bis 4, dadurch gekennzeichnet, daß die Umsetzung in einem Lösungsmittel durchgeführt wird.

6. Verfahren gemäß den Ansprüchen 1a, 2a und 5, dadurch gekennzeichnet, daß die Umsetzung bei Temperaturen zwischen -25 und 250°C, vorzugsweise jedoch bei Temperaturen zwischen -10°C und der Siedetemperatur des verwendeten Lösungsmittels, durchgeführt wird.

7. Verfahren gemäß den Ansprüchen 1b, 2b und 5, dadurch gekennzeichnet, daß die Umsetzung bei Temperaturen zwischen 20 und 150°C, vorzugsweise jedoch bei der Siedetemperatur des Reaktionsgemisches, z.B. bei 100°C, durchgeführt wird.

8. Verfahren gemäß den Ansprüchen 1c, 2c und 5, dadurch gekennzeichnet, daß die Umsetzung bei Temperaturen zwischen 0 und 100°C, vorzugsweise jedoch bei Temperaturen zwischen 20 und 50°C, durchgeführt wird.

9. Verfahren gemäß den Ansprüchen 1d, 2d und 5, dadurch gekennzeichnet, daß die Umsetzung bei der Siedetemperatur des Reaktionsgemisches durchgeführt wird.

10. Verfahren gemäß den Ansprüchen 1e, 2e und 5, dadurch gekennzeichnet, daß die Umsetzung bei Temperaturen zwischen 20 und 180°C, vorzugsweise jedoch bei Temperaturen zwischen 50 und 150°C, durchgeführt wird.

11. Verfahren gemäß den Ansprüchen 1f, 2f und 5, dadurch gekennzeichnet, daß die Umsetzung bei Temperaturen zwischen 0 und 80°C, vorzugsweise jedoch bei Temperaturen zwischen 20 und 60°C, durchgeführt wird.

12. Verfahren gemäß den Ansprüchen 1g, 2g und 5, dadurch gekennzeichnet, daß die Umsetzung bei Temperaturen zwischen 0 und 80°C, vorzugsweise jedoch bei Temperaturen zwischen 25 und 50°C, durchgeführt wird.

13. Verfahren gemäß den Ansprüchen 3a und 5, dadurch gekennzeichnet, daß die Umsetzung bei Temperaturen zwischen -10 und 120°C, vorzugsweise bei Temperaturen zwischen Raumtemperatur und der Siedetemperatur des Reaktionsgemisches, durchgeführt wird.

14. Verfahren gemäß den Ansprüchen 3b, 4b und 5, dadurch gekennzeichnet, daß die Reduktion bei Temperaturen zwischen 0 und 50°C durchgeführt wird.

15. Verfahren gemäß den Ansprüchen 3c, 4c und 5, dadurch gekennzeichnet, daß das Diazoniumsalz bei niederen Temperaturen, z.B. bei Temperaturen zwischen -10 und 5°C, hergestellt wird.

16. Verfahren gemäß den Ansprüchen 3c, 4c und 5, dadurch gekennzeichnet, daß das Diazoniumsalz auf Temperaturen zwischen 15 und 90°C erhitzt wird.

17. Verfahren gemäß den Ansprüchen 3d und 5, dadurch gekennzeichnet, daß die Umsetzung bei Temperaturen zwischen 0 und 75°C, vorzugsweise bei Raumtemperatur, und bei einem Wasserstoffdruck von 1 bis 5 bar durchgeführt wird.

18. Verfahren gemäß den Ansprüchen 3f und 5, dadurch gekennzeichnet, daß die Umsetzung bei Temperaturen zwischen 20 und 150°C, zweckmäßigerweise bei Temperaturen zwischen 80 und 100°C, durchgeführt wird.

19. Verfahren gemäß den Ansprüchen 3g und 5, dadurch gekennzeichnet, daß die Umsetzung bei Temperaturen zwischen 0 und 100°C, vorzugsweise jedoch bei Temperaturen zwischen 15 und 70°C, durchgeführt wird.

0056144

Europäisches Patentamt

# EUROPÄISCHER RECHERCHENBERICHT

Nummer der Anmeldung
EP 81 11 0731.7

| Kategorie | EINSCHLÄGIGE DOKUMENTE: Kennzeichnung des Dokuments mit Angabe, soweit erforderlich, der maßgeblichen Teile | betrifft Anspruch | KLASSIFIKATION DER ANMELDUNG (Int. Cl.) |
|---|---|---|---|
| P,X | EP - A1 - 0 023 569 (K. THOMAE GMBH)<br>* Ansprüche, Beispiele 22 to 27, 36 to 38, 69, 70, 79, 80, 91 bis 97, 99 bis 112, 132 bis 137, 164, 173 bis 192, 215 bis 218, 220 bis 223, 225, 226, 229 bis 232, 271 bis 289 *<br>& DE - A1 - 2 928 352<br>& DE- A1 - 2 949 259<br>& DE - A1 - 3 016 651<br>& DE - A1 - 3 016 650<br>-- | 1-5, 9-14, 19-21 | C 07 D 295/14<br>C 07 D 211/14<br>C 07 D 209/44<br>C 07 D 221/22<br>C 07 D 213/56<br>C 07 D 401/04<br>C 07 D 213/55<br>A 61 K 31/455<br>A 61 K 31/55<br>A 61 K 31/40<br>A 61 K 31/395<br>A 61 K 31/445<br>//(C 07 D 401/04, 211/14, 213/56) |
| A | US - A - 4 163 788 (CIBA-GEIGY CORP.)<br>* Zusammenfassung *<br>-- | 11 | RECHERCHIERTE SACHGEBIETE (Int Cl.³) |
| A | DE - A1 - 2 655 144 (BASF AG)<br>* Anspruch 1 *<br>-- | | A 61 K 31/455<br>C 07 D 209/44<br>C 07 D 211/14<br>C 07 D 213/55<br>C 07 D 213/56<br>C 07 D 213/82<br>C 07 D 221/22<br>C 07 D 295/14<br>C 07 D 401/04 |
| A | DE - A1 - 2 500 157 (HOECHST AG)<br>* Anspruch 1 *<br>---- | | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 01-04-1982 | BREW |

EPA form 1503.1 06.78